(19) **Europäisches Patentamt**

**European Patent Office**

**Office européen des brevets**

(11) **EP 1 083 226 A1**

(12) **EUROPEAN PATENT APPLICATION**
published in accordance with Art. 158(3) EPC

(43) Date of publication:
14.03.2001 Bulletin 2001/11

(21) Application number: 99919666.0

(22) Date of filing: 24.05.1999

(51) Int. Cl.7: **C12N 15/62**, C12N 15/13,
C07K 16/28, C07K 16/46,
C12P 21/08, C12N 5/12,
C12M 1/00, G01N 33/53,
A61K 39/395

(86) International application number:
PCT/JP99/02711

(87) International publication number:
WO 99/61629 (02.12.1999 Gazette 1999/48)

(84) Designated Contracting States:
DE FR GB IT

(30) Priority: 25.05.1998 JP 15995798
26.05.1998 JP 16302398

(71) Applicants:
• Asahi Kasei Kogyo Kabushiki Kaisha
Osaka-shi, Osaka 530-8205 (JP)
• ASAHI MEDICAL Co., Ltd.
Tokyo 101-8482 (JP)

(72) Inventors:
• ONO, Mitsuharu
Fuji-shi, Shizuoka 416-0933 (JP)

• SOKA, Takayuki
Fuji-shi, Shizuoka 416-0939 (JP)
• MORIMOTO, Ikuo
Setagaya-ku, Tokyo 156-0043 (JP)
• MIYAMURA, Koichi
Nagoya-shi, Aichi 462-0823 (JP)

(74) Representative:
Forstmeyer, Dietmar, Dr. rer. nat., Dipl.-Chem. et
al
Boeters & Bauer,
Bereiteranger 15
81541 München (DE)

(54) **CELL SEPARATION DEVICE AND SEPARATION METHOD**

(57) A device for separating CD (cluster differentiation)-positive cells of human hematopoietic undifferentiated cells and a method for separating or detecting the same.

In the above device and method, CD-positive cells are separated by using CD molecule-recognizing recombinant antibodies obtained by isolating anti-CD4 or anti-CD34 antibody gene and expressing the same.

The device and method are efficaciously employed in the field of medicine for collecting hematopoietic undifferentiated cells, eliminating lymphocytes from cells to be used in bone marrow transplantation, detecting leukemic cells, etc. It is also possible to prepare medicinal compositions for autoimmune diseases by using the above antibodies.

EP 1 083 226 A1

## Description

TECHNICAL FIELD

The present invention relates to a device for separating cells using a novel recombinant antibody and a method for separating or detecting cells utilizing thereof. As more specific examples, the present invention relates to a method for separating or detecting human CD4- or CD34-positive cells using a novel recombinant antibody to human CD (Cluster differentiation) 4 antigen or CD34 antigen and to a device for separating such cells.

Furthermore, the present invention relates to an antibody having particularly high affinity for and high specificity to human CD4 antigen and to a medicinal composition containing the antibody, which are useful for the determination of amount or detection of antigens as well as for the application to pharmaceutical preparations and to medical equipments.

BACKGROUND ART

By using an antibody to a surface antigen of human blood cell, only a particular cell can be separated, eliminated, recovered, or detected from among a group of blood cells. For example, elimination of CD4-positive cells improves the symptom of patients suffering autoimmune disease while recovery of CD34-positive cells can concentrate hematopoietic precursor cells which can be utilized for transplantation.

Human CD4 is a glycoprotein having a molecular weight of 55 kD, which is present as a monomer on the cell membrane and expressed mainly in T cells. T cells expressing CD4 (about 65% of peripheral T cells) have properties of helper T cells and recognize an antigen presented by class II MHC molecule. Also, CD4 is known to bind to HIV (human immunodeficiency virus: AIDS virus) and serves as a receptor upon infection. In immune systems, the helper T cells are indispensable for proper response to antigens and essential for the most of antibody responses of B cells. The helper T cells are activated when they recognize nonself antigen bound to the surface of mainly antigen presenting cells and secrete interleukin 2 to stimulate the propagation of T cells, which in turn assists the activation of B cells and further promotes production of antibodies. There are those helper T cells that not only assist lymphocytes but also activate macrophages by secretion of $\gamma$- interferons.

In healthy persons, to prevent self disorders, various mechanisms such as clonal deletion of lymphocytes having a self reactive antigen receptor, clonal anergy thereof, and suppression of such functions are provided (immune tolerance). Although the mechanism of occurrence of autoimmune diseases is not fully understood, disorders in such immune tolerance mechanisms are considered to cause autoimmune diseases. Lymphocyte clone having a self reactive antigen receptor is not limited to those helper T cells which express CD4. However, it is recognized that the helper T cells are important in autoimmune diseases.

As a therapeutic method for certain autoimmune diseases, administration of adrenal cortex hormone preparations, extraction of spleen, administration of immunosuppressive agents, and the like are applied depending on the degree of the symptom. However, in spite of drug therapy or splenectomy, there are many cases which show no efficacious recovery. In actuality, no perfect therapeutic method for such patients has been established so that it is hastened to take a countermeasure therefor.

As another therapeutic method for autoimmune diseases, elimination by adsorption of self antibodies by extracorporeal circulation of blood using an immunoadsorbent column has been tried in various manners. For example, application of a column with a carrier to which protein A having bindability to immunoglobulin is immobilized has been investigated. However, protein A is expensive and is not so excellent in adsorption specificity. Furthermore, cases in which side effects occurred have been reported. Therefore, various problems remain to be solved before it can be utilized in medical hardware.

Recently, not only elimination of liquid factors from blood but also blood cell separation techniques have been reported. However, there are problems that the bindability to antigens is poor depending on ligands used, no sufficient elimination of cells can be expected due to low reaction specificity with the antigen or some other reasons, or unintended cells are adsorbed nonspecifically.

JP-A-3-32680 discloses a separation material to which apeptide or lipid having affinity for T lymphocytes is immobilized. JP-A-6-154316, JP-A-6-154317, JP-A-6-218051, and JP-A-6-269663 disclose CD4-positive cell collecting materials comprised by nonwoven fabric to which peptides having affinity for CD4-positive cells are immobilized. Each of them has the problem that the affinity for and specificity to antigen of the peptide used as a ligand are insufficient so that no satisfactory cell adsorptive power can be expected. In particular, the latter one uses complementarity determining regions "CDR" closely relating to the formation of antigen binding sites by anti-CD4 antibodies and a portion of peptide of a framework region "framework" intervenient therebetween. Use of only a portion thereof does not provide effective antigen binding. It is known that CDR, for each of H chain and L chain, includes three regions called "CDR-1," "CDR-2," and "CDR-3" from the N-terminal side. It is essential for expecting effective antigen binding that the steric configurations of "CDR-1," "CDR-2," and "CDR-3" of at least one of H chain and L chain are maintained substantially the same as that of the antibody.

Conventionally, human CD34 has been reported as the cell surface antigen of hematopoietic undifferentiated cells and anti-MY10 antibody producing hybridomas have been known as cells producing antibodies recognizing the human CD34 molecules (U.S. Patent No. 4,965,204 and J.Immunology, 133, 157 (1984)). Thereafter, many reports have been made on anti-human CD34 antibodies and attempts have been made to apply them to separation of hematopoietic undifferentiated cells and the like in medical fields such as bone marrow transplantation. In order to make effective use of such application as directed to medical treatment, there is an increasing need for producing anti-human CD34 antibodies efficiently and developing medical equipment utilizing them.

CD34 molecules are known as undifferentiated cell antigen markers expressed on undifferentiated cells of blood and their application to separation of undifferentiated cells has been reported (S. Saeland, et al., Blood, 72, 1580 (1988), etc.). Furthermore, development is being made of medical equipment for separating undifferentiated cells from a group of differentiated cells and cancer cells, utilizing combination of anti-CD34 antibody and biotin, avidin, or magnetic beads (Dreger, et al., Exp. Hematol., 23, 147 (1995)).

Antibodies generally have strong bindability to antigens and are high in specificity so that application to medical treatment is expected. However, upon fabrication of medical equipment, the cost on the production of antibodies is concerned about. It is necessary to efficiently produce antibodies and apply them to medical equipment. To this end, as a method for efficiently producing antibodies, it is desired to establish a technology for preparing recombinant antibodies and apply them to medical equipment.

DISCLOSURE OF THE INVENTION

The present invention practices modifications such as human-mouse-chimera formation and single chain antibody formation by a gene engineering technique while maintaining high affinity for and high specificity to human CD4 antigen being had by the antibodies and provides recombinant antibodies which enable a decrease in antigenicity, an increase in the amount of production of antibodies, and a decrease in production costs.

Ordinary antibodies present in organisms require both of an antigen-specific variable region nucleotide sequence and a constant region gene. There is known a small number of classes for the constant region gene. In contrast, the variable region nucleotide sequences of H chain and L chain are very high in diversity and hence much effort is required for procuring target variable region nucleotide sequences. However, the present inventors have wielded binding experiments by gene amplification (PCR), enzyme immunoassay (EIA) using antigens, or a flow cytometer to thereby procure genes encoding target human CD4 antibody and human CD34 antigen from among plural antibody genes in the hybridoma and have determined their sequence and functions as follows. Furthermore, the present inventors have recombined the genes to enable a mass production of human-mouse-chimera antibodies and single chain antibodies.

Utilization of the recombinant antibodies makes it possible to produce antibodies, which have heretofore been obtained from mouse ascites or by cell culture, by bacteria and the like. This gave rise to the possibility that medical equipment will be supplied at low costs.

In general, preparation of monoclonal antibodies is started by separating B lymphocytes from an animal immunized with a target antigen and fusing them with myeloma cells to make hybridomas. Then, screening using a target antigen is performed to procure antibody-producing hybridomas. On this occasion, to procure hyboridomas producing antibodies having high bindability to the antigen and high specificity, many cells must be screened and much effort is required.

As a result of intensive investigation by the present inventors, it has now been found that antibodies whose CDR-1, CDR-2, and CDR-3 of H chain variable region extracted from anti-CD4 antibody producing hybridoma 4H5 are the amino acid sequences described under Sequence ID Nos. 1, 2, and 3, respectively, in the Sequence Listing and whose CDR-1, CDR-2, and CDR-3 of L chain variable region extracted from anti-CD4 antibody producing hybridoma 4H5 are the amino acid sequences described under Sequence ID Nos. 4, 5, and 6, respectively, in the Sequence Listing have high affinity for and high specificity to human CD4 antigen.

Taking an example thereof, the binding affinity of OKT4A antibody, known as an anti-human CD4 antibody, for human CD4 antigen has been reported to be such that $KA=4\times10^8$ $M^{-1}$ (Virginia L. Pulito, et al., The Journal of Immunology, 156: 2840-2850 (1996)). Therefore, the dissociation constant (KD: the lower this value is, the higher the binding affinity) is such that $KD=2.5\times10^{-9}$ M (from 1/KA=KD). On the other hand, as described in the examples hereinbelow, the dissociation constant of 4H5 antibody to human CD4 antigen is such that $KD=8.08\times10^{-10}$ M (Example 5). This shows that 4H5 antibody can bind human CD4 antigen more strongly than OKT4A antibody can.

4H5 Antibody producing hybridoma, Mouse-Mouse hybridoma 4H5, has been deposited at National Institute of Bioscience and Human-Technology, Agency of industrial Science and Technology, Ministry of International Trade and Industry, Japan under Accession Number: FERM BP-6729.

EP 1 083 226 A1

Ordinary antibodies present in organisms require both of an antigen-specific variable region nucleotide sequence and a constant region gene. There is known a small number of classes for the constant region gene. In contrast, the variable region nucleotide sequences of H chain and L chain are very high in diversity and hence much effort is required for procuring target variable region nucleotide sequences. However, the present inventors have wielded binding experiments by gene amplification (PCR) or enzyme immunoassay (EIA) using antigens to thereby procure target antibody genes to human CD4 antigen from among plural antibody genes in the hybridoma and have recombined the genes to enable a mass production of human-mouse-chimera antibodies and single strand antibodies.

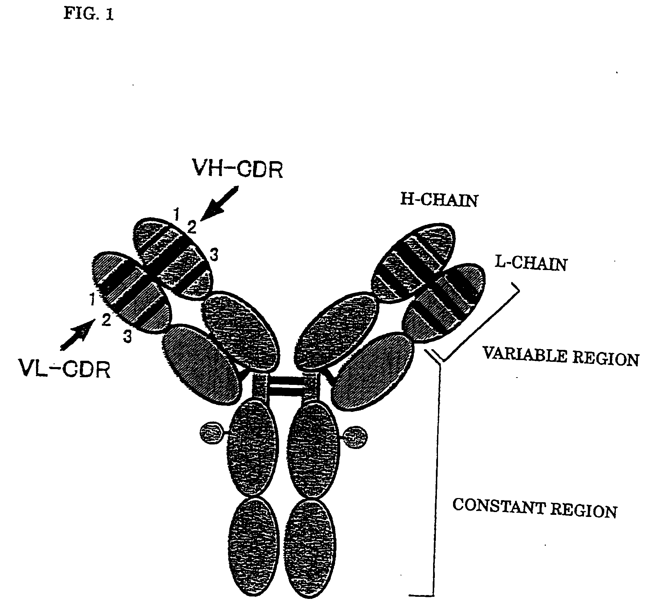

Ordinary antibodies comprise each two kinds of polypeptide, larger and smaller ones. The subunit of the larger one is called "H chain" and the subunit of the smaller one is called "L chain." The respective peptides consist of a "variable region" (or "V region") which exists on the N-terminal side and forms an antigen binding site and a "constant region" (or "Fc") which is constant by class of antibody. The variable region is further divided into complementarity determining regions "CDR" which are closely related to the formation of antigen binding sites and framework regions "framework" intervenient therebetween. It is known that CDR, for each of H chain and L chain, includes three regions called "CDR-1," "CDR-2," and "CDR-3" from the N-terminal side. Fig. 1 is a schematic diagram illustrating the structure of IgG.

The number of amino acids constituting the variable region of an antibody may often vary from antibody to antibody. The amino acid sequence of the variable part of H chain of 4H5 antibody is indicated by Sequence ID No. 35 in the Sequence Listing. In Sequence ID No. 35 in the Sequence Listing, amino acids 1 to 30 indicates framework 1, amino acids 31 to 35 indicates CDR-1, amino acids 36 to 49 indicates framework 2, amino acids 50 to 66 indicates CDR-2, amino acids 67 to 98 indicates framework 3, amino acids 99 to 107 indicates CDR-3, and amino acids 108 to 118 indicates framework 4. The amino acid sequence of the variable part of L chain of 4H5 antibody is indicated by Sequence ID No. 36 in the Sequence Listing. In Sequence ID No. 36 in the Sequence Listing, amino acids 1 to 23 indicates framework 1, amino acids 24 to 38 indicates CDR-1, amino acids 39 to 53 indicates framework 2, amino acids 54 to 60 indicates CDR-2, amino acids 61 to 92 indicates framework 3, amino acids 93 to 101 indicates CDR-3, and amino acids 102 to 111 indicates framework 4. The boundaries between the frameworks and CDR have been determined with reference to the gene sequence of variable region of mouse antibody described in Sequences of Proteins of Immunological Interest, 5th edition, 1991 (published by USA NIH).

Sequence ID No. 7 in the Sequence Listing shows nucleotide sequence corresponding to the amino acids 9 to 118 in the Sequence ID No. 35 in the Sequence Listing. Sequence ID No. 8 in the Sequence Listing shows nucleic acid nucleotide sequence corresponding to the amino acids 9 to 111 in the Sequence ID No. 36 in the Sequence Listing.

Sequence ID No. 39 in the Sequence Listing shows gene sequence and amino acid sequence encoded thereby of the variable part of H chain of anti-MY10 antibody, which is an antibody that binds to anti-CD34 antigen. In Sequence ID No. 39 in the Sequence Listing, amino acids 1 to 30 indicates framework 1, amino acids 31 to 35 indicates CDR-1, amino acids 36 to 49 indicates framework 2, amino acids 50 to 65 indicates CDR-2, amino acids 66 to 97 indicates framework 3, amino acids 98 to 106 indicates CDR-3, and amino acids 107 to 117 indicates framework 4.

Sequence ID No. 40 in the Sequence Listing shows gene sequence and amino acid sequence encoded thereby of the variable part of L chain of MY10 antibody. In Sequence ID No. 40 in the Sequence Listing, amino acids 1 to 23 indicates framework 1, amino acids 24 to 39 indicates CDR-1, amino acids 40 to 54 indicates framework 2, amino acids 55 to 61 indicates CDR-2, amino acids 62 to 93 indicates framework 3, amino acids 94 to 102 indicates CDR-3, and amino acids 103 to 113 indicates framework 4.

Sequence ID No. 41 in the Sequence Listing shows gene sequence and amino acid sequence encoded thereby of the single chain antibody. In Sequence ID No. 41 in the Sequence Listing, amino acids 1 to 39 indicates a sequence containing a signal for secretion from Escherichia coli, amino acids 40 to 156 indicates a variable region of H chain, amino acids 157 to 171 indicates a linker, amino acids 172 to 284 indicates a variable region of L chain, and amino acids 285 to 302 indicates a sequence containing an E-tag.

pCANTAB5E Plasmid (Pharmacia) is provided with a sequence containing a part of H chain and L chain, i.e., amino acids 140 to 169 as a linker. This is used in the present invention.

The linker bindable to H chain and L chain, which is included by the plasmid in the present invention, is not limited to the above sequence and can be used. The method for isolating single strand antibodies from hybridomas may use Recombinant Phage Antibody System kit (Pharmacia) and the like. However, where no human CD34 antigen necessary for screening is present as in the present invention, kits cannot be used effectively so that great ingenuity is required.

The present invention relates to the following device for separating cells, method for separating or detecting cells using the same.

(1) A device for separating CD4-positive cells using an antibody comprising a chimera antibody or a single chain antibody which binds to CD4 molecules or combinations thereof.

4

(2) A device for separating CD4-positive cells using a chimera antibody comprising an H chain variable region of which CDR-1 is an amino acid sequence of Asp Tyr Val Ile Asn (Sequence ID No. 1 in the Sequence Listing), CDR-2 is an amino acid sequence of Glu Ile Tyr Pro Gly Ser Gly Ser Ala Tyr Tyr Asn Glu Met Phe Lys Gly (Sequence ID No. 2 in the Sequence Listing), and CDR-3 is an amino acid sequence of Arg Gly Thr Gly Thr Gly Phe Ala Tyr (Sequence ID No. 3 in the Sequence Listing), an L chain variable region of which CDR-1 is an amino acid sequence of Lys Ala Ser Gln Ser Val Asp Tyr Asp Gly Asp Ser Tyr Met Asn (Sequence ID No. 4 in the Sequence Listing), CDR-2 is an amino acid sequence of Ala Ala Ser Asn Leu Glu Ser (Sequence ID No. 5 in the Sequence Listing), and CDR-3 is an amino acid sequence of Gln Gln Ser Ser Glu Asp Pro Pro Thr (Sequence ID No. 6 in the Sequence Listing), and an Fc region of a human type.

(3) A device for separating CD4-positive cells using a single chain antibody comprising an H chain variable region of which CDR-1 is an amino acid sequence of Asp Tyr Val Ile Asn (Sequence ID No. 1 in the Sequence Listing), CDR-2 is an amino acid sequence of Glu Ile Tyr Pro Gly Ser Gly Ser Ala Tyr Tyr Asn Glu Met Phe Lys Gly (Sequence ID No. 2 in the Sequence Listing), and CDR-3 is an amino acid sequence of Arg Gly Thr Gly Thr Gly Phe Ala Tyr (Sequence ID No. 3 in the Sequence Listing), and an L chain variable region of which CDR-1 is an amino acid sequence of Lys Ala Ser Gln Ser Val Asp Tyr Asp Gly Asp Ser Tyr Met Asn (Sequence ID No. 4 in the Sequence Listing), CDR-2 is an amino acid sequence of Ala Ala Ser Asn Leu Glu Ser (Sequence ID No. 5 in the Sequence Listing), and CDR-3 is an amino acid sequence of Gln Gln Ser Ser Glu Asp Pro Pro Thr (Sequence ID No. 6 in the Sequence Listing).

(4) A method for separating or detecting human CD4-positive cells using an antibody comprising a chimera antibody or a single chain antibody which binds to CD4 molecules or combinations thereof.

(5) A method for separating or detecting human CD4-positive cells using a chimera antibody comprising an H chain variable region of which CDR-1 is an amino acid sequence of Asp Tyr Val Ile Asn (Sequence ID No. 1 in the Sequence Listing), CDR-2 is an amino acid sequence of Glu Ile Tyr Pro Gly Ser Gly Ser Ala Tyr Tyr Asn Glu Met Phe Lys Gly (Sequence ID No. 2 in the Sequence Listing), and CDR-3 is an amino acid sequence of Arg Gly Thr Gly Thr Gly Phe Ala Tyr (Sequence ID No. 3 in the Sequence Listing), an L chain variable region of which CDR-1 is an amino acid sequence of Lys Ala Ser Gln Ser Val Asp Tyr Asp Gly Asp Ser Tyr Met Asn (Sequence ID No. 4 in the Sequence Listing), CDR-2 is an amino acid sequence of Ala Ala Ser Asn Leu Glu Ser (Sequence ID No. 5 in the Sequence Listing), and CDR-3 is an amino acid sequence of Gln Gln Ser Ser Glu Asp Pro Pro Thr (Sequence ID No. 6 in the Sequence Listing), and an Fc region of a human type.

(6) A method for separating or detecting human CD4-positive cells using a single chain antibody comprising an H chain variable region of which CDR-1 is an amino acid sequence of Asp Tyr Val Ile Asn (Sequence ID No. 1 in the Sequence Listing), CDR-2 is an amino acid sequence of Glu Ile Tyr Pro Gly Ser Gly Ser Ala Tyr Tyr Asn Glu Met Phe Lys Gly (Sequence ID No. 2 in the Sequence Listing), and CDR-3 is an amino acid sequence of Arg Gly Thr Gly Thr Gly Phe Ala Tyr (Sequence ID No. 3 in the Sequence Listing), an L chain variable region of which CDR-1 is an amino acid sequence of Lys Ala Ser Gln Ser Val Asp Tyr Asp Gly Asp Ser Tyr Met Asn (Sequence ID No. 4 in the Sequence Listing), CDR-2 is an amino acid sequence of Ala Ala Ser Asn Leu Glu Ser (Sequence ID No. 5 in the Sequence Listing), and CDR-3 is an amino acid sequence of Gln Gln Ser Ser Glu Asp Pro Pro Thr (Sequence ID No. 6 in the Sequence Listing).

Also, the present invention relates to the following device for separating cells, method for separating or detecting cells using the same.

(1) A device for separating CD34-positive cells using an antibody comprising a chimera antibody or a single chain antibody which binds to CD34 molecules or combinations thereof.

(2) A device for separating CD34-positive cells using an antibody comprising an H chain variable region of which CDR-1 is an amino acid sequence of Ser-His-Gly-Val-His (Sequence ID No. 43 in the Sequence Listing), CDR-2 is an amino acid sequence of Val-Ile-Trp-Gly-Ala-Gly-Arg-Thr-Asp-Tyr-Asn-Ala-Ala-Phe-Ile-Se r (Sequence ID No. 44 in the Sequence Listing), and CDR-3 is an amino acid sequence of Asn-Arg-Tyr-Glu-Ser-Tyr-Phe-Asp-Tyr (Sequence ID No. 45 in the Sequence Listing), an L chain variable region of which CDR-1 is an amino acid sequence of Arg-Ser-Ser-Gln-Asn-Leu-Val-His-Ser-Asn-Gly-Asn-Thr-Tyr-Leu-Hi s (Sequence ID No. 46 in the Sequence Listing), CDR-2 is an amino acid sequence of Lys-Val-Ser-Asn-Arg-Phe-Ser-Gly-Val-Pro-Asp-Arg-Phe (Sequence ID No. 47 in the Sequence Listing), and CDR-3 is an amino acid sequence of Ser-Gln-Ser-Thr-His-Val-Pro-Leu-Thr (Sequence ID No. 48 in the Sequence Listing), and an Fc region of a human type.

(3) A device for separating CD34-positive cells using a single chain antibody comprising an H chain variable region of which CDR-1 is an amino acid sequence of Ser-His-Gly-Val-His (Sequence ID No. 43 in the Sequence Listing), CDR-2 is an amino acid sequence of Val-Ile-Trp-Gly-Ala-Gly-Arg-Thr-Asp-Tyr-Asn-Ala-Ala-Phe-Ile-Se r (Sequence ID No. 44 in the Sequence Listing), and CDR-3 is an amino acid sequence of Asn-Arg-Tyr-Glu-Ser-Tyr-Phe-Asp-Tyr (Sequence ID No. 45 in the Sequence Listing), an L chain variable region of which CDR-1 is an amino acid sequence of Arg-Ser-Ser-Gln-Asn-Leu-Val-His-Ser-Asn-Gly-Asn-Thr-Tyr-Leu-Hi s (Sequence ID No. 46 in the Sequence Listing), CDR-2 is an amino acid sequence of Lys-Val-Ser-Asn-Arg-Phe-Ser-Gly-Val-Pro-Asp-Arg-Phe (Sequence ID No. 47 in the Sequence Listing), and CDR-3 is an amino acid sequence of Ser-Gln-Ser-Thr-His-Val-Pro-Leu-Thr (Sequence ID No. 48 in the Sequence Listing).

(4) The device for separating CD34-positive cells as described in any one of (1) to (3) above, using an antibody added with an amino acid sequence containing a basic amino acid or acidic amino acid on the C-terminal or N-terminal or both of the amino acid sequence of the antibody.

(5) A method for separating or detecting human CD34-positive cells using an antibody comprising a chimera antibody or a single chain antibody which binds to CD34 molecules, or combinations thereof.

(6) A method for separating or detecting human CD34-positive cells using an antibody comprising an H chain variable region of which CDR-1 is an amino acid sequence of Ser-His-Gly-Val-His (Sequence ID No. 43 in the Sequence Listing), CDR-2 is an amino acid sequence of Val-Ile-Trp-Gly-Ala-Gly-Arg-Thr-Asp-Tyr-Asn-Ala-Ala-Phe-Ile-Se r (Sequence ID No. 44 in the Sequence Listing), and CDR-3 is an amino acid sequence of Asn-Arg-Tyr-Glu-Ser-Tyr-Phe-Asp-Tyr (Sequence ID No. 45 in the Sequence Listing), an L chain variable region of which CDR-1 is an amino acid sequence of Arg-Ser-Ser-Gln-Asn-Leu-Val-His-Ser-Asn-Gly-Asn-Thr-Tyr-Leu-Hi s (Sequence ID No. 46 in the Sequence Listing), CDR-2 is an amino acid sequence of Lys-Val-Ser-Asn-Arg-Phe-Ser-Gly-Val-Pro-Asp-Arg-Phe (Sequence ID No. 47 in the Sequence Listing), and CDR-3 is an amino acid sequence of Ser-Gln-Ser-Thr-His-Val-Pro-Leu-Thr (Sequence ID No. 48 in the Sequence Listing), and an Fc region of a human type.

(7) A method for separating or detecting human CD34-positive cells using a single chain antibody comprising an H chain variable region of which CDR-1 is an amino acid sequence of Ser-His-Gly-Val-His (Sequence ID No. 43 in the Sequence Listing), CDR-2 is an amino acid sequence of Val-Ile-Trp-Gly-Ala-Gly-Arg-Thr-Asp-Tyr-Asn-Ala-Ala-Phe-Ile-Se r (Sequence ID No. 44 in the Sequence Listing), and CDR-3 is an amino acid sequence of Asn-Arg-Tyr-Glu-Ser-Tyr-Phe-Asp-Tyr (Sequence ID No. 45 in the Sequence Listing), an L chain variable region of which CDR-1 is an amino acid sequence of Arg-Ser-Ser-Gln-Asn-Leu-Val-His-Ser-Asn-Gly-Asn-Thr-Tyr-Leu-Hi s (Sequence ID No. 46 in the Sequence Listing), CDR-2 is an amino acid sequence of Lys-Val-Ser-Asn-Arg-Phe-Ser-Gly-Val-Pro-Asp-Arg-Phe (Sequence ID No. 47 in the Sequence Listing), and CDR-3 is an amino acid sequence of Ser-Gln-Ser-Thr-His-Val-Pro-Leu-Thr (Sequence ID No. 48 in the Sequence Listing).

(8) The method for separating or detecting human CD34-positive cells as described in any one of (5) to (7) above, using an antibody added with an amino acid sequence containing a basic amino acid or acidic amino acid on the C-terminal or N-terminal or both of the amino acid sequence of the antibody.

Furthermore, the present invention relates to the following antibody, nucleic acid encoding the antibody, method for producing antibodies using the nucleic acid, and medicinal composition comprising the antibody.

(1) An antibody comprising an H chain variable region of which CDR-1, CDR-2, and CDR-3 are amino acid sequences described in Sequence ID Nos. 1, 2, and 3, respectively, in the Sequence Listing and having affinity for CD4 antigen.

(2) An antibody comprising an L chain variable region of which CDR-1,CDR-2, and CDR-3 are amino acid sequences described in Sequence ID Nos. 4, 5, and 6, respectively, in the Sequence Listing and having affinity for CD4 antigen

(3) A monoclonal antibody to CD4 antigen, produced by hybridoma 4H5 having an accession number FERM BP-6729.

(4) A nucleic acid encoding the antibody as described in any one of (1) to (3) above.

(5) The nucleic acid as described in (4) above, containing the nucleotide sequence described in Sequence ID Nos. 7 and 8.

(6) A method for producing antibodies using the nucleic acid as described in (4) or (5) above.

(7) A recombinant antibody which can be obtained by the method described in (6) above and having affinity for CD4 antigen.

(8) The recombinant antibody as described in (7) above, wherein Fc region of the antibody is of a human type.

(9) The recombinant antibody as described in (7) above, wherein the antibody is a single chain antibody.

(10) A medicinal composition comprising the antibody as described in any one of (1) to (3) above and a pharmaceutically acceptable carrier.

(11) A medicinal composition comprising the recombinant antibody as described in any one of (7) to (9) above and a pharmaceutically acceptable carrier.

The "antibody" as used herein includes besides antibodies in the form of those usually present in organisms, molecules containing at least one antigen binding site formed by variable regions of H chain or L chain of an antibody or combinations thereof. For example, it includes a peptide which consists of the variable region of H chain alone, Fab which consists of a set of an H chain fragment and an L chain fragment, (Fab')$_2$ which consists of two sets of an H chain fragment and an L chain fragment, a single chain antibody "ScFv" comprising an H chain fragment and an L chain fragment bound in series on the same peptide, and the like.

The "human CD4 antigen" as used herein has been reported by Parnes, J. R. (Adv. Immunology, 44, 265 (1989)) and known as an antigen marker for helper T cells, expressed on lymphocytes. In humans, the CD4 antigen is expressed on monocytes, macrophages, etc.

That the antigen specificity and strength of binding to antigen of an antibody is determined mainly by the amino acid sequence of CDR is shown by humanization of mouse antibodies (Gussow, D. and Seemann, G., Methods in Enzymology, 203:99-121 (1991); Glaser, S.M. et al., J. Immunology 149:2607-2614 (1992)).

For secretion and expression of an antibody by COS7 cells, various vectors can be used (Whittle, N. and Adair, J. et al. (1987), Protein Eng., 1 (6), 499-505; Sutter, K.D. and Feys, V. et al. (1992), Gene 113, 223-30). Utilizing human antibody expression plasmid (pG1), plasmid pG14H5 which can express human-mouse chimera anti-human CD4 antibody and plasmid pG1My10 which can express human CD34 antibody were prepared. By gene transfer of these into COS7 cells , human anti-CD4 antibody can be produced. Human-mouse chimera antibody as used herein refers to an antibody produced by a gene constituted by a variable region having a hybridoma-derived mouse gene sequence and a constant region having a human-derived gene sequence. pG1 is a plasmid obtained by removing the transmembrane domain (TM) added to the sequence of human Cγ1 in pSE plasmid described in International Publication WO95/15393 to thereby improve such that antibody can be secreted in organism as in the case of ordinary antibodies. Details of its preparation are shown as Reference Example 1. That is, it has a gene sequence encoding the human constant region and can express a human-mouse chimera antibody by linking the mouse variable region gene.

The "chimera antibody" as used herein refers to an antibody obtained by artificially combining the amino acid sequences of antibodies of different animal species. For example, it refers to an antibody containing a mouse hybridoma-derived CDR and a human antibody-derived amino acid sequence in the constant region. Furthermore, its framework may contain a human-derived amino acid sequence.

The "human CD34 antigen" as used herein has been reported by Civin et al. (U. S. Patent No. 4,965,204 and J. Immunology, 133, 157 (1984)) and is known as an antigen marker for hematopoietic undifferentiated cells, expressed on undifferentiated cells in blood.

The "substantially the same function" as used herein means that the epitope on an antigen molecule and binding power to the antigen are substantially the same. It is known that even if replacement of amino acids occurs in the framework in the variable region or in the constant region, frequently antibodies of essentially the same function are generated. That the antigen specificity and strength of binding to antigen of an antibody is determined mainly by the amino acid sequence of CDR is shown by humanization of mouse antibodies (Gussow, D. and Seemann, G., Methods in Enzymology, 203:99-121 (1991); Glaser, S.M., et al., J. Immunology 149:2607-2614 (1992)).

COS7 cells are cultured usually using 10% fetal bovine serum (FBS)-added Dulbecco's Modified Eagle's Medium (DMEM medium) in the presence of 5% $CO_2$ at 37°C. A gene transfer method into COS7 cells and a breeding production method of cells after the gene transfer are described in a textbook on experiments such as T. Yokota and K. Arai: Bio Manual Series 4, Gene Transfer and Expression Analysis Method, published by Yodosha (1994). The method of gene transfer into COS7 cells may be a DEAE dextran method (Bebbington, C. R. (1991); METHODS: A Companion to Methods in Enzymology, 2(2), 136-45) as well as an electroporation method.

In the present invention, the constant region gene incorporated into pG1 is Cγ1, so that each clone was expressed as IgG1. Upon production of antibodies, to avoid contamination of serum-derived bovine antibodies, it is desirable that cultivation be performed in a serum-free DMEM medium. The anti-CD4 antibody secreted in the culture supernatant can be readily purified by a purification method for general IgG antibodies using, for example, protein A or protein G.

As a host in the case of industrial production, CHO cell and myeloma Sp 2/0 cell are well known (Xiang, J. et a). (1990) Mol. Immun., 27, 809; Bebbington, C. R. et al. (1992) Bio/technology, 10, 169; Larrick, J. W. and Wallace, E. F. et al. (1992) Immunol. Rev. 130, 69-85; Deyev, S. M. and Lieber, A. et al. (1994) Appl. Biochem. Biotechnol. 47 (2-3), 143-54). For example, in the case of CHO cells, a method for selecting clones having high productivity with an agent such as MTX has been reported (Bebbington, C. R. (1991) METHODS: A Companion to Methods in Enzymology, 2(2), 136-45). If stable high productivity strain can be procured, the strain can be utilized for industrial production of recombinant anti-human CD4 antibody.

When utilized as a medical preparation, human-mouse chimera antibody is extremely low in antigenicity in the human body as compared with the mouse antibody and its safety is more improved. In the case where an ordinary antibody is converted to F(ab')$_2$ by decomposing it with a protease such as pepsin, the fragment which utilizes human Fc is high in safety as compared with the mouse-derived one. Upon utilizing it to medical equipment, there is the possibility that the antibody utilized as a ligand undergoes decomposition by a protease or the like to release in minute amounts. Concerning such decomposates, those humanized can be said to be higher in safety.

In the case of single chain antibodies, the effect of decreasing antigenicity can be expected by reducing the molecular weight. The antibody converted to Fab by treating the antibody with a protease such as papain still contains the mouse constant region. In contrast, the single chain antibody is free of them, so that the antigenicity can be decreased to a very low level. As a method for isolating the single chain antibodies from hybridomas, Recombinant Phage Antibody System kit (Pharmacia) and the like may be utilized. However, where the single chain antibody is toxic to Escherichia coli, a production host, and causes death of Escherichia coli or decomposition of the single chain antibody occurs, kits cannot be used effectively so that great ingenuity is required. The single chain antibody can be prepared by studying inductive vector such as pSE380 plasmid (Invitrogen) or pET24d(+) plasmid (Novagen) and bacterial species serving as a host. In production, besides the above-described method, an eukaryotic cell expression system, an insect cell expression system, and a yeast cell expression system can be utilized efficaciously. As the linker which links H chain and L chain, 15 amino acid residues made of three repetitions of (Gly-Gly-Gly-Gly-Ser) was used. However, linkers not limited to this sequence may be used.

Using the antibodies produced by the present invention, CD4-positive cells can be separated. By the present invention, it is possible to efficiently separate CD4-positive cells from a blood cell suspension outside the body or from blood by extracorporeal circulation. The separated cells or CD4-positive cell-eliminated cell group can be used in the treatment of various diseases. Preparation of cell mixed liquor or blood preparation from which CD4-positive cells are eliminated, or preparation of cell suspension comprising CD4-positive cells as a major component or CD4-positive cell preparation can be performed with ease. By eliminating CD4-positive cells, improvement in the symptom of chronic rheumatism, multiple sclerosis, systemic lupus erythematosus, etc. which are autoimmune diseases can be expected.

As the configuration of antibody, the produced antibody molecules can be utilized as they are. Also, Fab, $F(ab')_2$, Fv or Fd etc., which are fragments containing antigen binding sites obtained by treatment with various kinds of protease may also be applied. These fragments are explained in, for example, "Introduction to Antibody Engineering" (Chijinshokan) etc. Besides, where the present invention is applied to extracorporeal circulation of blood in a human body, it is useful to use chimera antibodies of which the portion other than the variable region is made of a human type antibody, taking into consideration side effects and antigenicity in case the antibody is released in blood. The method for preparing the antibodies is not limited particularly but antibodies purified to high purity must be used. The method for producing monoclonal antibodies may be a usually practiced method in which a hybridoma is propagated in mouse abdominal cavity and the produced antibody is recovered from the ascites, or a method for obtaining an antibody from a culture supernatant in a serum-free medium. In the case of fragmented peptides, they can be obtained by an enzyme treatment of antibody molecules. However, they can be produced in bacteria, yeast, etc. by genetic engineering techniques. Combination of the monoclonal antibodies, monoclonal antibody-derived antibody fragments, peptides, etc. obtained by the methods results in stronger bindability.

Currently, as a method for separating cells, a method for separating only target cells specifically utilizing antibodies is being developed. For example, mention may be made of a method for separating fluorescent antibody-labeled cells, a method in which a ligand having affinity for the target cells is immobilized on a water-insoluble carrier and the target cells are directly or indirectly bound thereto, a separation method by immune adsorptive column and a separation method using immunomagnetic beads.

The method for separating fluorescent antibody-labeled cells is a method in which first a cell mixed liquor is incubated together with a fluorescent-labeled monoclonal antibody which recognizes membrane antigen expressed on target cells and then treated cells are irradiated with laser beam by a cell sorter or the like taking advantage of generation of fluorescence only by the antibody-bound cells to separate the fluorescent antibody-bound cell.

International Publication WO87-04628 describes a method in which a monoclonal antibody to an antigen present on the surface of membrane of a target cell is used after immobilizing it directly on the surface of a device for separation and a method in which first a cell mixed liquor is incubated with a monoclonal antibody which binds to the membrane antigen on a target cell and then treated in a device for separating cells to which is immobilized a ligand such as an anti-immunoglobulin antibody that binds to the antibody on the cell surface. Separation of cells is a method for separating cells in which antigen-positive cells are directly or indirectly bound to a monoclonal antibody immobilized to the carrier or device. In these methods, separation occurs on a plastic dish having immobilized thereto an antibody, the cell mixed liquor is poured first onto the plastic dish to which antibody is immobilized and the dish is incubated in order to bind the antibody to the membrane antigen on the target cell. After the incubation, the plastic dish is washed to eliminate the cells not bound thereto and separate them. An immunoadsorbent column method is a method in which a ligand such as an antibody to the membrane antigen on the target cell is immobilized to the surface of beads, the beads are packed in a column, and separation of cells is performed therein.

International Publication WO91-16116 describes a method in which biotin-labeled antibody to the membrane antigen on a target cell is added to a cell mixed liquor, the mixture is incubated to form a cell-antibody-biotin association and then passed through a column packed with beads composed of porous acrylamide gel to which avidin is immobilized, and the target cell is separated by binding in the column taking advantage of strong binding of biotin-avidin.

An immunomagnetic bead method is a method in which first a cell mixed liquor is incubated with magnetic beads to which an antibody is bound to label the target cell with magnetic bead. After the labeling, a magnetic device is used to separate the labeled cells from unlabeled cells.

However, to practice separation of cells by these methods, a large amount of antibody molecules is necessary and its cost is concerned about upon its application in particular to medical sites. The recombinant antibodies produced by the present invention can reduce the cost with ease.

As one of carriers to which two kinds or more antibodies that recognize the same cell are directly immobilized, water-insoluble carrier can be utilized. It is also possible to combine CD4 molecules and other molecules expressed on CD4-positive cells, for example, Fusin (CXCR-4) (Feng, Y et al., Science, 272 (5263), 872-877 (1996)) acting as a second receptor for HIV infection, which is a G protein-coupled receptor, and CCR-5 (Deng, H et al., Nature, 381 (6584), 661-666 (1996)) also serving as a second receptor for HIV etc. These molecules are expressed less frequently and the efficiency of separation is not so high. However, by combining the antibody recognizing CD4 molecules and the antibody recognizing these molecules simultaneously, these expressed cells can be isolated.

Similarly, carriers to which two kinds or more antibodies recognizing different antigen molecules on the same cell are directly immobilized can be utilized. It is possible to combine CD34 molecule with other molecules expressed on undifferentiated cell, for example c-kit (Yarden et al., EMBOJ.,6, 3341 (1987)), which is a SCF receptor, receptor FLK-2 (Mathews et al., Cell, 65, 1143 (1991)), α-chain of interleukin-3 receptor (Kitamura et al., Cell, 66, 1165 (1991)), etc. These molecules are expressed in low frequencies and are separated at low efficiencies. However, combination with CD34 molecule and antibody simultaneously enables the isolation of expressed cells thereof.

For example, biotin or magnetic bead is bound in advance to an antibody to be immobilized to a cell and then a substance which readily binds to biotin or magnetic bead, for example, a water-insoluble carrier which binds to or contains avidin or magnetism is reacted to readily recover the water-insoluble carrier to which the cell is bound. Also, by reacting target hematopoietic undifferentiated cells with CD34 antibody in advance, and then reacting the cells with a water-insoluble carrier to which anti-immunoglobulin antibody is bound, and washing or recovering the water-insoluble carrier, only the target cells can be separated selectively. Utilization of antibodies immobilized to magnetic bead, which is a water-insoluble carrier, can perform the separation more efficiently by use of a magnet, etc. The container used in the present invention may be in the form of, for example, a column packed with a water-insoluble carrier, a flask or flask-like case packed with a water-insoluble carrier. Devices for separating hematopoietic undifferentiated cells can be fabricated by a combination of such a shape of container and the water-insoluble carrier to which the antibody is directly or indirectly immobilized. The devices can also be made one having high applicability as a system for separating cells by combining them with a pump for flowing a cell suspension, physiological saline or the like.

The container used in the present invention may be in the form of, for example, a column packed with a water-insoluble carrier, a flask or flask-like case packed with a water-insoluble carrier. Devices for separating CD4-positive cells can be fabricated by a combination of such a shape of container and the water-insoluble carrier to which the antibody is directly or indirectly immobilized. The devices can be made one having high applicability as a system for separating cells by combining them with a pump for flowing a cell suspension, physiological saline or the like.

Upon immobilizing an antibody to the water-insoluble carrier, it is useful to bind the antibody and water-insoluble carrier via a spacer intervening therebetween. Furthermore, the water-insoluble carrier and the compound may be bound to each other via a molecule (spacer) having an optional length, as needed. For the details of the spacer, reference may be made to, for example, "Affinity Chromatography" (K. Kasai, et al., Tokyo Kagaku Dojin Publishing (1991) p.105-108). Examples of the spacer include polymethylene chain and polyethylene glycol chain and so on. The length of the spacer is preferably 500 Angstroms or less and more preferably 200 Angstroms or less. As a method for binding a compound to the water-insoluble carrier via a spacer, mention may be made of, for example, a method in which where agarose is used as the water-insoluble carrier, the hydroxyl groups of agarose are reacted with and bound to an isocyanate group on one side of the hexamethylene diisocyanate used as the spacer and then the remaining isocyanate group and an amino group, a hydroxyl group, a carboxyl group, or the like of the antibody are reacted and bonded, and so on.

The water-insoluble carrier as used herein means one which is in a solid state in an aqueous solution at room temperature and may be in any form. Examples of form include sphere, cube, plate, chip, fiber, flat membrane, sponge, hollow fiber, etc. Among these, spherical, granular and fibrous ones are desirable from the viewpoints of ease of closest packing, easy retention of antibodies on the surface relatively uniformly, securement of relatively large effective area, and flowability of cell suspension.

The material of water-insoluble carrier may be a either inorganic or organic compound as far as it can hold antibodies on the surface. However, organic polymer compounds are desirable since they generate less eluates upon contact with the cell suspension and since their shape can be regulated more readily. Examples of such include polymers and copolymers of vinyl based compounds or derivatives thereof, such as polypropylene, polystyrene, polymethacrylate esters, polyacrylate esters, polyacrylic acid, and polyvinyl alcohol, polyamides based compounds such as nylon-6 or -66, polyester based compounds such as polyethylene terephthalate, plant-derived polysaccharide based compounds such as cellulose, and so on. They may be combined with a magnet to make it easy to recover such water-insoluble carriers.

Modification of water-insoluble carriers and imparting hydrophilicity on the surface are used advantageously in the present invention. Immobilization of organism-derived proteins such as albumin and globulin, introduction of synthetic functional groups can impart hydrophilicity to the surface of water-insoluble carrier. Examples of synthetic functional groups for imparting hydrophilicity include polyethylene glycol chain having 2 to 1,500 repeating units, a hydroxyl group, an amide group, an ether group, and an ester group. However, the polyethylene glycol chain and hydroxyl group may be bound to each other or present separately. Examples of monomers having a polyethylene glycol chain for imparting hydrophilicity include terminal methoxymethacrylates such as methoxydiehtylene glycol methacrylate, and methoxytriethylene glycol methacrylate, terminalmethoxy acrylates such as methoxydiethylene glycol acrylate and methoxytriethylene glycol acrylate, and methacrylates and acrylates having a terminal hydroxyl group in which a hydrogen atom is bonded instead of a methyl group, and monomers which have in general a polyethylene glycol chain containing 2 to 1,500 repeating units having at least one polymerizable functional group at the terminal thereof. Furthermore, when bound directly to a carrier, the synthetic functional group includes polyethylene glycol derivatives having 2 to 1,500 repeating units. Examples of monomer having other substituent group imparting hydrophilicity include vinylpyrrolidone, etc. but not limited thereto. Moreover, one or more polymerizable functional groups may be present. The polymerizable functional group refers to functional groups which can polymerize by a single or two or more functional groups. Examples thereof include carbon-to-carbon multiple bond compounds such as a vinyl group, an acetylene group, and a diene group, cyclic structures such as an epoxy group and an oxetane group, and so on but not limited thereto.

Further, non-ionic functional groups may be present together with the above functional groups. Examples thereof include nonionic functional groups particularly those intended to improve hydrophilicity, for example, amide groups such as a dimethylamide group, a diethylamide group, and a diisopropyl amide group, polyester chains, e.g., aromatic polyester chains and aliphatic polyester chains, such as a polyethylene terephthalate chain, and a polybutylene terephthalate chain, polyether chains such as a methylene glycol chain and a propylene glycol chain, polycarbonate chains, and the like nonionic hydrophilic functional groups, or nonionic functional groups in general intended to impart hydrophobicity, for example, alkyl chains, fluorinated alkyl chains, allyl chain, etc.. Any of the above functional groups may coexist. Preferably, those having nonionic hydrophilic functional group or groups provide good results.

As a method for modifying the water-insoluble carrier and impart hydrophilicity on its surface, any known method such as a grafting method with a covalent bond, an ionic bond, radiation or plasma, physical adsorption, embedding or insolubilization of precipitates on the surface of the substrate can be used. Therefore, a method for practicing surface modification by a known method such as graft polymerization of a polymer compound or monomers thereof with radiation or plasma or covalent bond formation (JP-A-1-249063 and JP-A-3-502094) can be advantageously used in the present invention.

The method in which an antibody is immobilized to the surface of water-insoluble carrier includes a method for covalently bonding the antibody to the surface of water-insoluble carrier by a grafting method using chemical means, or radiation or electron beam or a method for covalently bonding the antibody to the surface of water-insoluble carrier via a functional group on the surface of water-insoluble carrier by a chemical means, or a like method. Of these, the covalently bonding method via a functional group is preferred since there is no danger of causing the elution of antibody upon use. Where the water-insoluble carrier has a cover layer thereon, antibodies may be insolubilized on the surface of the cover layer.

As an example of obtaining an activated group for immobilizing an antibody on the surface of water-insoluble carrier or of a cover layer thereon, there are cited a cyanogen halide method, an epichlorohydrin method, a bisepoxide method, a bromoacetyl bromide method, an acetamide halide method, etc. More specifically, mention may be made of an amino group, a carboxyl group, a hydroxyl group, a thiol group, an acid anhydride, a succinylimide group, a substituting halogen group, an aldehyde group, an epoxy group, a tosyl group, etc. A bromocyan method, an N-hydrosuccinimde group method, and an acetamide halide method are particularly desirable in view of ease of immobilization of antibodies.

As the haloacetaminomethylating agent used for activated groups, N-hydroxymethylchloroacetamide, N-hydroxymethylfluoroacetamide, N-hydroxytnethylbromoacetamide, N-hydroxymethyliodoacetamide, etc. are used advantageously. Of these, preferably, N-hydroxymethylbromoacetamide, N-hydroxymethyliodoacetamide, etc. are used advantageously from the viewpoint of economy and stability. The fluoro group or chloro group introduced into the water-insoluble carrier can be readily converted into an iodine group or a bromine group by treating with a solution of potassium iodide or potassium bromide after the introduction of the activated groups. As an acid catalyst for use in the production of the water-insoluble carrier having introduced the activated groups, any one may be used as far as it is a strong acid, particularly a protic acid. Non-limitative examples include sulfonic acid derivatives of trifluoromethane, methane, benzene, toluene, etc. and Friedel-Crafts catalysts such as sulfuric acid, zinc chloride, aluminum chloride, and tin tetrachloride may be used advantageously.

Antibodies produced by the genes of the present invention are bound to a toxin and can be Administrated to patients suffering chronic rheumatism, multiple sclerosis, systemic lupus erythematosus, etc. to decrease CD4-positive lymphocyte clones having self reactive antigen receptors. Such modified antibodies can be utilized in peripheral blood from patients recovered by apheresis and pheripheral blood leukocyte suspensions. By combining the gene of the variable region derived from other antibody, production of bi-specific antibodies and multi-specific antibodies is also possible.

Furthermore, the present invention provides a medicinal composition comprising the monoclonal antibody of the present invention as described above and a pharmaceutically acceptable carrier.

The anti-CD4 antibody gene of the present invention thus obtained enables efficient production of anti-CD4 antibody and provides various forms of therapeutic preparations. The thus produced recombinant antibodies may be contained in the medicinal compositions together with substances used for retaining the activity of the antibody upon administration to human body, such as carriers and stabilizers having pharmaceutically acceptable compositions. As the carrier and stabilizers, human serum albumin, gelatin, etc. can be cited. The term pharmaceutically acceptable means that there occurs no undesirable side effect accompanied by administration such as nausea, vertigo, and vomiturition, immune response to preparations frequent administration thereof. Alternatively, they may be in the form of liquid medicinal compositions together with suitable solvents, diluents, and stabilizers which are pharmaceutically acceptable. Furthermore, in addition to the above medicinal compositions, they may be in the form of slow-releasing transplants such as microspheres, liposomes, etc. intended for regulating the concentration in organism.

In the present invention, where parenteral administration (injection) is practiced, a suitable amount of chimera antibody and single chain antibody is on the order of 10 μg to 1 mg/kg body weight. Where cells are treated therewith outside the body, a suitable amount thereof is on the order of 0.1 μg to 1 mg/ml.

By combining genes of the variable region derived from other antibodies, production of bi-specific antibodies and multi-specific antibodies is possible. The term multi-specific antibody means an antibody which have at least 2 kinds of antigen binding sites recognizing different antigens or epitopes. Among them, bi-specific antibody means an antibody which have two or more kinds of antigen binding sites recognizing different antigens or epitopes. For example, a bi-specific converted antigen of which one of the antigen binding sites of an ordinary antigen recognizes CD4 antigen or CD34 antigen and the other antigen binding site thereof recognizes T lymphocyte antigen CD3, etc. can strongly recognize CD4-positive T lymphocyte or lymphocytes in CD34-positive leukemia cells so that it can induce the effect of eliminating leukemia cells, etc. Besides CD3, general lymphocyte markers such as CD2, CD5, CD6, and CD7 can be utilized. By combining these and allowing them to express as IgM, bi-specific or multi-specific antibodies can be prepared. Besides bi-specific conversion upon production, bi-specific or multi-specific conversion can be achieved by producing and purifying monoclonal antibodies and then binding the antibodies to each other.

In the case of single strand antibodies, bi-specific or multi-specific conversion upon production is possible and bi-specific or multi-specific antibodies can be produced by repeatedly arranging H chain and L chain of a combination of plural antigen recognizing sites in one peptide. Binding can be effected after production and purification.

It is possible to combine them not only for different antigens but also on different epitopes in the same molecule. For example, combination of different anti-CD4 antibodies is possible. Several anti-CD4 antibodies have been reported. For example, Leu-3a antibody, Leu-3b antibody (Becton Dickinson), OKT4 antibody, OKT4A antibody (Ortho), T4 antibody (Coulter), MT310 antibody (Dacco), F101-69 antibody, 16P25 antibody (Biosys), Edu-2 antibody, MEM-115 antibody (Synbas Biotechnology), 7E14 antibody (Exulfia Corporation), BL4 antibody, 13B8.2 antibody (Immunotech), KT69-7 antibody (Lab Vision Corporation), B-F51 antibody (Progen Biotechnique), BL-TH4 antibody (Sun Vio BV), 94B1 antibody, L197 antibody, L80 antibody, L93 antibody, L201 antibody, L34 antibody, L202 antibody, L200 antibody, L252 antibody, 73F11 antibody, 72G4 antibody, NUTH1 antibody, L71 antibody, RFT4 antibody, MT151 antibody, MT321 antibody (D. G. Healey et al., Eur. J. Immunol., $\underline{21}$, 1491 (1991)), OKT4B antibody, OKT4C antibody, OKT4D antibody, OKT4E antibody, OKT4F antibody, Q6D3 antibody, L77 antibody, L104 antibody, L83 antibody, L190 antibody, L122 antibody, L120 antibody (Matthias Merkenschlager et al., The J. Immunol., $\underline{145}$, 2839, 1990), and so on are known and these antibody genes can be isolated and used for production.

Similarly, it is possible to combine them not only for different antigens but also on different epitopes in the same molecule. For example, combination of different anti-CD34 antibodies is possible. Several anti-CD34 antibodies have been reported. For example, anti-HPCA-2 antibody (Becton Dickinson), anti-HPCA-1 antibody (Becton Dickinson), 4A1 (Nichirei), B1.3C5 (Katz et al., Leuk. Res., $\underline{9}$, 191 (1985)), 12.8, 115.2 (Andrews et al., Blood, $\underline{67}$, 842 (1986)), ICH3 (Watt et al., Leukaemia, $\underline{1}$, 417 (1987)) , Tuk3 (Unchanska-Ziegler et al., Tissue Antigens, $\underline{33}$, 230 (1989)), QBEND10 (Fina et al., Blood, $\underline{75}$, 2417 (1990)), CD34 (Ab-1) (Oncogene Sciences), Immu-133 (Barrande et al., Hybridoma, $\underline{12}$, 203 (1993), etc. are known and these antibody genes can be isolated and used for production.

Not only by combinations of H chain and L chain but also peptide of H chain only or peptide of L chain only can be utilized. Such antibody species may be selected depending on applications in which they are utilized. Selection can be made by strength of binding, antigenicity, etc.

The amino acid sequences of single chain antibodies are indicated by Sequence ID Nos. 9 and 10 in the Sequence Listing. Also, examples of nucleic acid sequence encoding them are shown together with the amino acid sequences. In the Sequence ID No. 9 in the Sequence Listing, the amino acids 1 to 22 indicates a sequence containing a signal for secretion from Escherichia coli, amino acids 23 to 133 indicates a variable region of L chain, amino acids 134 to 148 indicates a linker, amino acids 149 to 266 indicates a variable region of H chain, and amino acids 267 to 305 indicates a sequence containing FLAG-tag (amino acids 270 to 277), c-myc-tag (amino acids 281 to 290), and His×6-tag (amino acids 296 to 301)). In the Sequence ID No. 10 in the Sequence Listing, the amino acids 1 to 22 indicates a sequence containing a signal for secretion from Escherichia coli, amino acids 23 to 140 indicates a variable region of H chain, amino acids 141 to 155 indicates a linker, amino acids 156 to 266 indicates a variable region of L chain, and amino acids 267 to 305 indicates a sequence containing FLAG-tag (amino acids 270 to 277), c-myc-tag (amino acids 281 to 290), and His×6-tag (amino acids 296 to 301)).

A single chain antibody can be expressed in Escherichia coli cells by deleting the amino acids 1 to 20 of the amino acid sequences described under Sequence ID Nos. 9 and 10 in the Sequence Listing. The amino acids 267 to 305 of the amino acid sequences described under Sequence ID Nos. 9 and 10 in the Sequence Listing is a sequence for detecting and purifying single chain antibodies and it can be deleted or replaced by any sequence. Preparation of a vector containing a signal for secretion from Escherichia coli and a sequence for detection and purification is shown as Reference Example 2. The linker in the amino acid sequence 134 to 148 described under Sequence ID No. 9 in the Sequence Listing and the linker in the amino acid sequence 141 to 155 described under Sequence ID No. 10 in the Sequence Listing may be replaced by any sequence as far as the steric configuration of the variable regions of L chain and H chain are retained substantially the same as the 4H5 antibody. The Lys residues at the 305 positions of the amino acid sequences under Sequence ID Nos. 9 and 10 in the Sequence Listing functions effectively so as to align the orientation of single chain antibody molecules where the antibody is immobilized to the water-insoluble carrier. In order to increase the efficiency of immobilization, plural Lys residues may be introduced. It is also effective to introduce Cys residues. The above-described modifications can be practiced with ease by genetic engineering techniques.

In the practice of the present invention, the constant region gene incorporated into expression plasmid pG1 is C$\gamma$1, so that each clone was expressed as IgG1. Upon production of antibodies, to avoid contamination of serum-derived bovine antibodies, it is desirable that cultivation be performed in a serum-free DMEM medium. The anti-CD34 antibody secreted in the culture supernatant in this way can be readily purified by a purification method for general IgG antibodies using, for example, protein A or protein G. As a host in the case of industrial production, CHO cell and myeloma Sp 2/0 cell are well known (Xiang, J. et al. (1990) Mol. Immun., 27, 809; Bebbington, C. R. et al. (1992) Bio/technology, 10, 169; Larrick, J. W. and Wallace, E. F. et al. (1992) Immunol. Rev. 130, 69-85; Deyev, S. M. and Lieber, A. et al. (1994) Appl. Biochem. Biotechnol. 47 (2-3), 143-54).

For example, in the case of CHO cells, a method for selecting clones having high productivity with an agent such as MTX has been reported (Bebbington, C. R. (1991) METHODS: A companion to Methods in Enzymology, 2(2), 136-45). If stable high productivity strain can be procured, the strain can be utilized for industrial production of recombinant anti-human CD34 antibody.

As the configuration of antibody, the produced antibody molecules can be utilized as they are. Also, Fab, F(ab')$_2$, Fv or Fd, which are fragments containing antigen binding sites obtained by treatment with various kinds of protease may also be applied. These fragments are explained in, for example, "Introduction to Antibody Engineering" (Kanemitsu, p20-22, Chijinshokan). Besides, where the present invention is applied to extracorporeal circulation of blood in a human body, it is useful to use chimera antibodies of which the portion other than the variable region is made of a human type antibody, taking into consideration side effects and antigenicity in case the antibody is released in blood. The method for preparing the antibodies is not limited particularly but antibodies purified to high purity must be used. The method for producing monoclonal antibodies may be a usually practiced method in which a hybridoma is propagated in mouse abdominal cavity and the produced antibody is recovered from the ascites, or a method for obtaining an antibody from a culture supernatant cultured in a serum-free medium. In the case of fragmented peptides, they can be obtained by an enzyme treatment of antibody molecules. However, they can be produced in bacteria, yeast, etc. by genetic engineering techniques. Combination of the monoclonal antibodies, monoclonal antibody-derived antibody fragments, peptides, etc. obtained by the methods results in stronger bindability.

CD34 molecules have been reported by Civin et al. (U.S. Patent No. 4,965,204 and J. Immunology, 133, 157 (1984)) and are known as undifferentiated cell antigen markers expressed on undifferentiated cells of blood specifically and their application to separation of undifferentiated cells has been reported (S. Sealed, et al., Blood, 72, 1580 (1988), etc.). Furthermore, development is being made of medical equipment for separating undifferentiated cells from a group of differentiated cells and cancer cells, utilizing biotin, avidin, or combinations with magnetic beads (Dragger, et al., Exp. Hematol., 23, 147 (1995)).

The hematopoietic undifferentiated cells (CD34-positive cells) separated by the present invention means those cells which can propagate by specified stimulation and which can differentiate adult cells such as blood erythrocytes, monocytes, granulocytes, lymphocytes, and macrophages into at lest one of them. Particularly, they include undifferentiated blood cells which form colonies when cultivated in a semi-solid medium in the presence of a suitable propagation factor and hematopoietic stem cells having a differentiation potency into all series and self-propagation potency. The cells are contained abundantly in bone marrow and cord blood. The cells are also contained in peripheral blood but they can be induced in peripheral blood by administration of cytokines such as granulocyte colony stimulating factor (G-CSF). Techniques for propagating hematopoietic undifferentiated cells outside the body by cultivation of these cells have been increasingly reported. Cell suspensions containing those hematopoietic undifferentiated cells may be used as a material for separating hematopoietic undifferentiated cells by the present invention.

The device for separating cells of the present invention can be utilized as medical equipment to carry out separation of hematopoietic undifferentiated cells. Hematopoietic undifferentiated cells can be separated efficiently from blood cell suspension outside the body or from blood by extracorporeal circulation and the separated cells can be used in the treatment of various diseases.

It is an effective means for incrasing medical effects to isolate or concentrate required hematopoietic undifferentiated cells or eliminate unrequired cell from a solution containing various cell species as in blood. Bone marrow transplantation is a method for transplanting normal hematopoietic undifferentiated cells to patients suffering cancers, such as patients suffering leukemia with bone marrow destruction and patients suffering congenital immune diseases such as aplastic anemia, and is being established as an effective treatment for such patients. Recently, methods for obtaining hematopoietic undifferentiated cells not only from bone marrow but also from peripheral blood and cord blood are used. Allogeneic bone marrow transplantation is a method in which bone marrow liquid recovered from healthy person is infused to a patient whose bone marrow has been destructed by anticancer agent or radiation. In this case, the healthy person has substantially the same HLA type, which is a leukocyte type, as that of the patient. This method has the problem that it causes GVHD (graft versus host disease), which is a rejection. Therefore, in recent years, there has been an increasing demand for separating hematopoietic undifferentiated cells in order to prevent graft versus host disease (GVHD) as a result of bone marrow autotransplantation or allogeneic bone marrow transplantation carried out in the treatment of malignant tumors or cancers in the hematopoietic system.

One feature of the present invention is to utilize recombinant antibodies. Utilization of recombinant antibodies enables modification of antibodies to those which can be readily immobilized to insoluble carriers. By addition of an amino acid sequence to the C-terminal or N-terminal of the peptide constituting the antibody, the antibody can be efficiently immobilized to the substrate such as insoluble carrier.

In the case of human-mouse chimera antibodies, an amino acid sequence containing basic or acidic amino acids is added as a sequence which the natural antibodies do not have in respect of the C-terminal or N-terminal of H chain and L chain or combinations thereof. Where the sequence is genetically modified in the midway of the Fc portion to convert the amino acid concerned to a basic or acidic amino acid, the C-terminal side containing the modified portion can be deemed as an added amino acid sequence. It is suitable for making a device for separation to make the Fc portion capable of being readily bound to insoluble carrier, which is not limited to the case of human-mouse chimera antibodies.

In single chain antibodies, it is desirable to add a sequence for immobilizing a peptide to a substrate to the C-terminal or N-terminal of the peptide.

Sequences containing basic amino acids or acidic amino acids are desirable as amino acids contained in the sequence to be added. However, any amino acid is included in the present invention as far as the amino acid is intended to immobilize antibodies to the substrate.

As the basic amino acid, lysine, hydroxylysine, arginine, histidine, etc., can be cited. As the acidic amino acids, aspartic acid, glutamic acid, etc. can be cited. In particular, it is more efficient to use lysine, glutamic acid, and aspartic acid.

The length of the sequence is not particularly limited. Addition of mono amino acid such as lysine, aspartic acid, glutamic acid, or the like is also effective. However, addition of a sequence of a plurality of such amino acids is more effective. Where addition is made to an antibody having a low molecular weight as in the case of single chain antibodies, addition of a relatively long sequence reduces steric hindrance when the antibody after immobilization to the substrate is bound to the antigen so that more effectiveness of a device for separation can be expected.

The antibody added to the sequence of the present invention makes the pigment for detection to readily bind thereto so that detection of the antibody bound to the antigen enables detection and assay of the antigen.

## BRIEF DESCRIPTION OF THE DRAWINGS

Fig. 1 is a schematic diagram illustrating the structure of IgG.
Fig. 2 is a schematic diagram illustrating plasmid pG1.
Fig. 3 is a schematic diagram illustrating plasmid pSE380ScFv.
Fig. 4 is a schematic diagram illustrating plasmid pET24ScFv.
Fig. 5 is a diagram illustrating the antigen binding affinity of 4H5 antibody and MT310 antibody, in correlation between the antibody concentration and determined value (ABS) in ELISA.

Fig. 6 illustrates bindability of chimera antibody to KG 1a cells. (1) stands for a positive control in which mouse CD34 antibody was reacted and (2) illustrates that the recombinant antibody can stain KG 1a cells which are CD34-positive cells, where black peaks indicate staining as compared with white peaks which are negative controls.

BEST MODE FOR CARRYING OUT THE INVENTION

Hereinafter, the present invention will be described by examples, which further explain the present invention by concrete examples. However, the present invention should not be construed as being limited thereto.

[Reference Example 1]

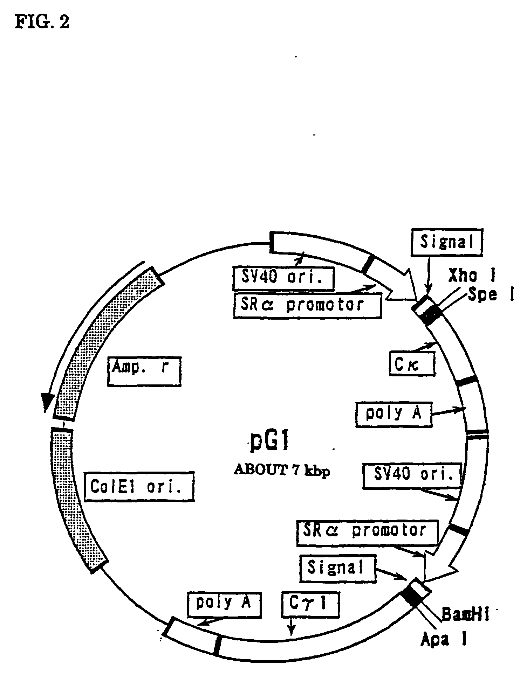

pG1 plasmid was prepared. The transmembrane (TM) region of expression vector pSE (International Publication WO95/15393) of a membrane-bound type human antibody was eliminated to prepare a vector capable of secreting and expressing a human antibody. First an expression vector pSE was digested with a restriction enzyme SalI to make the cleaved end blunt end. This reaction was operated by using DNA Blunting Kit (TaKaRa Shuzo) according to the protocols attached to the kits. The expression vector pSE after the above-described treatment was digested with a restriction enzyme ApaI and electrophoresed in a 0.7% agarose gel to extract and purify pSE vector DNA deleted of a gene region containing cγ1 gene and a transmembrane region (TM). The reaction was operated using GeneClean II Kit (Bio 101) according to the protocols attached to the kits. The end of the extracted pSE vector cleaved with the restriction enzyme was treated with bovine alkaline phosphatase (TaKaRa Shuzo) so that no self ligation could occur. Next, plasmid DNA pUCCG1 in which the whole-length human Cγ1 gene was incorporated was digested with a restriction enzyme KpnI (TaKaRa Shuzo) and the cleaved end was made blunt end. Thereafter, this was digested with a restriction enzyme ApaI. The reaction product was electrophoresed in 0.7% agarose gel to extract and purified a DNA fragment containing the whole-length of the Cγ1 gene. The DNA fragment and the previously treated pSE vecor were ligated with a ligation kit Ver. 2 (TaKaRa Shuzo), the ligated product was introduced into Escherichia coli DH5 strain. From colonies which emerged several colonies were selected and cultured and plasmid DNA was extracted and purified by conventional methods. The cleaving patterns of the plasmids were examined using suitable restriction enzymes present in pSE vector and one having the pattern coincided with the expected pattern was selected. The plasmid obtained by the above operations was named pG1. Fig. 2 is a diagram illustrating pG1 plasmid.

[Reference Example 2]

As the vector producing single chain antibody (ScFv antibody), a plasmid containing a secretion signal from Escherichia coli and a sequence encoding the tag for purification and detection was prepared prior to the introduction of anti-CD4 antibody gene. The secretion signal (signal sequence of PelB protein) from Escherichia coli was procured by mixing two kinds of Sequence ID No. 21 in the Sequence Listing

(5'TCATGAAATACCTGCTGCCGACCGCTGCTGCTGGTCTGCTGCTCCTCGCGGCCCAG3')

and Sequence ID No. 22 in the Sequence Listing

(5'TGCGGCCGCAGCCATGGTGTTTGCGGCCATCGCCGGCTGGGCCGCGAGGAGCAGCA3')

in equivalent amounts. The mixture was thermally denatured at 94°C for 5 minutes and then annealed at 65°C for 5 minutes. Then, elongation reaction was performed at 72°C for 10 minutes using GeneAmp PCR Reagent Kit with AmpliTaq DNA Polymerase (TaKaRa Shuzo) with a polymerase. The fragment was cloned using TA cloning kit (Invitrogen). Thus, cDNA encoding the secretion signal from Escherichia coli was procured. The sequence encoding the tag for purification and detection was procured by mixing two kinds of Sequence ID No. 23 in the Sequence Listing

(5'TGCGGCCGCAGACTACAAGGATGACGATGACAAAGGCTCGAGCGAGCAGAAGCTGA3')

and Sequence ID No. 24 in the Sequence Listing

(5'GGTGGGTCGACCTCGAGCCCAGATCCTCTTCGCTGATCAGCTTCTGCTCGCTCGAGC3'

)

in equivalent amounts. The mixture was thermally denatured at 94°C for 5 minutes and then annealed at 60°C for 5 minutes. Then, elongation reaction was performed at 72°C for 10 minutes using GeneAmp PCR Reagent Kit with Ampli Taq DNA Polymerase (TaKaRa Shuzo) with a polymerase. Using the sample after the reaction as a template, PCR reaction was further conducted using the primer set forth below. The amplified fragments were cloned using TA cloning kit (Invitrogen). Thus, cDNA encoding the tag for purification and detection was procured. As the primers were used Sequence ID No. 25 in the Sequence Listing (5'TGCGGCCGCAGACTACAAGGATG3') and Sequence ID No. 26 in the Sequence Listing

(5'TAAGCTTATCATTTGGTCGA

CCCGTGGTGATGATGATGGTGGGTCGACCTCGAGCC3').

The PCR conditions were such that using GeneAmp PCR Reagent Kit with AmpliTaq DNA Polymerase (TaKaRa Shuzo), a cycle of 94°C for 1 minute, 62°C for 1 minute, and 72°C for 1 minute was repeated 18 times. The PCR apparatus used was Model name: DNA Thermal Cycler 480 (Perkin Elmer). Of the cloned genes, the cDNA encoding the secretion signal from Escherichia coli was digested with restriction enzymes BspHI (New England Biolabs) and NotI (TaKaRa Shuzo) while the cDNA encoding the tag for purification and detection was digested with restriction enzymes HindIII (TaKaRa Shuzo) and NotI (TaKaRa Shuzo), followed by electrophoresis in 3.5% agarose gel to cleave each DNA fragment and extracted. The extraction of DNA fragments from agarose gel was performed using PCRprep (Promega).

pET24d(+) plasmid (Novagen) was eliminated of the portion of the multi-cloning site of from the restriction enzyme NotI site to the restriction enzyme Bpu1102I site in advance. pET24d(+) plasmid was digested with restriction enzyme NotI (TaKaRa Shuzo) and restriction enzyme Bpu1102I (TaKaRa Shuzo) and blunt ended with Klenow fragment (New England Biolabs) and dNTP mixture (TaKaRa Shuzo) according to the protocols attached to the kits. The plasmid DNA fragments were electrophoresed in 1% agarose gel and cut out and extracted. This was allowed to self ligate to procure pET24d(+) plasmid from which the restriction enzyme sites were eliminated. For the self ligation, Ligation ver. 2 Kit (TaKaRa Shuzo) was used. For the extraction of DNA fragment from agarose gel, GeneCleanII Kit (Bio 101) was used.

The pSE380 plasmid (Invitrogen) or the pET24d(+) plasmid from which the above restriction enzyme sites were eliminated was digested with restriction enzyme NcoI (TaKaRa Shuzo) and HindIII (TaKaRa Shuzo) and electrophoresed in 0.8% agarose gel. Thus, a vector DNA fragment was extracted and purified. The cDNA encoding the secretion signal from Escherichia coli digested with the above restriction enzymes and the cDNA encoding the tag for purification and detection were ligated to the vector fragment. For each ligation, Ligation ver. 2 Kit (TaKaRa Shuzo) was used. Also, the extraction of the DNA fragment from the agarose gel was carried out using GeneCleanII Kit (Bio 101) was used.

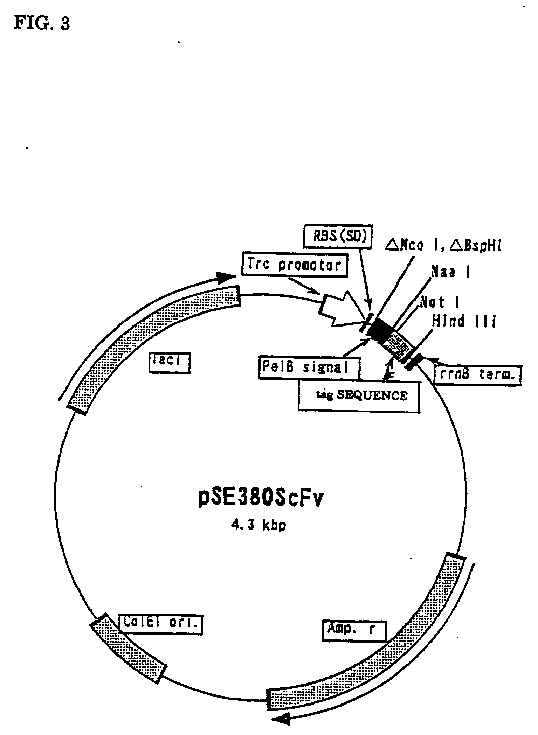

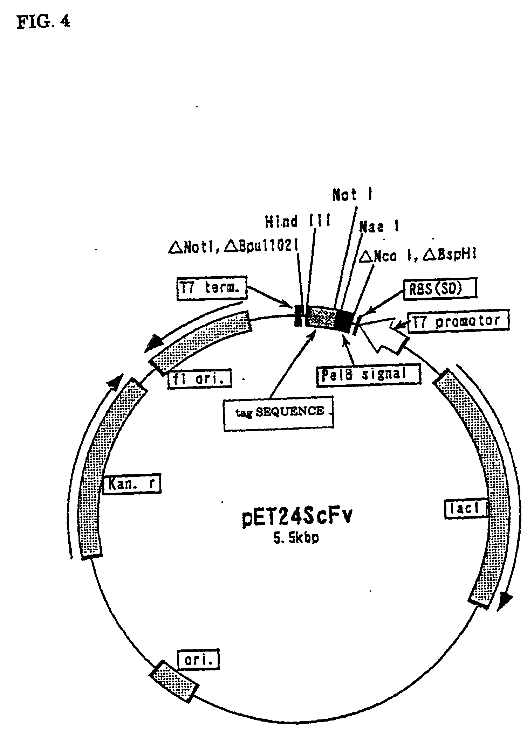

The plasmid vectors for producing ScFv antibody prepared by the above operations were named pSE380ScFv and pET24ScFv, respectively. Figs. 3 and 4 show schematic diagrams therefor.

[Example 1]

To evaluate binding affinity between various antibodies and human CD4, human soluble CD4 was produced and purified. 15 ml of peripheral blood was collected from healthy persons and made 30 ml with Hanks' balanced physiological saline (Gibco) and 13 ml aliquots were slowly overlayed in two centrifugation tubes each of which had been dispensed with 10ml each of Ficoll-Paque (Pharmacia) and separation was performed under the conditions of 800 rpm for 20 minutes. The cells in the mononuclear cell layer were recovered. The recovered cells were washed with Hanks' solution. mRNA was isolated from the obtained $1\times10^7$ cells using QuickPrep mRNA Purification Kit (Pharmacia) according to the protocols attached thereto. Using the obtained mRNA as a template, 1st strand cDNA was synthesized. This was performed using cDNA Synthesis Kit (Pharmacia) according to the attached protocols. Thereafter, the PCR method was practiced to amplify the target gene from the initiation codon to before the transmembrane region (TM), add a HindIII site at the 5'-terminal side, and a FLAG-tag sequence, a stop codon, and a NotI site at the 3'-terminal side. The primers used were Sequence ID No. 11 in the Sequence Listing (5'AAGCTTATGAACCGGGGAGTCCCTTTTA3') and Sequence ID No. 12 in the Sequence Listing

(5'GCGGCCGCTCACTTGTCATCGTCGTCCTTGTAGTCTGGCTGCACCGGGGTGGACCA3')

.

The PCR conditions were such that using GeneAmp PCR Reagent Kit with AmpliTaq DNA Polymerase (TaKaRa Shuzo), a cycle of 94°c for 1 minute, 57°C for 1 minute, and 72°C for 1 minute was repeated 30 times. The PCR apparatus used was Model name: DNA Thermal Cycler 480 (Perkin Elmer). The amplified gene fragments were cloned using TA cloning kit (Invitrogen). The nucleotide sequence was determined using DNA Sequencer ver. 1.2.0, ModelX373A (Applied Biosystems) according to the manufacturers protocol. The labeling reaction was performed using Universal M13 Reverse primer (Invitrogen) or Universal M13 Forward primer (Invitrogen) as a primer and using PRISM Ready Reaction Dye Deoxy Terminator Cycle Sequencing Kit (Applied Biosystems). The method was in accordance with the protocol attached to the kit. The labeling was performed using a labeling kit of ABI. After confirming the obtained gene sequence, eukaryotic cell expression vector, pcDNA3 plasmid (Invitrogen) and the cloned plasmid having a human soluble CD4cDNA were digested with restriction enzyme HindIII (TaKaRa Shuzo) and restriction enzyme NotI (TaKaRa Shuzo), electrophoresed in agarose gel by a conventional method, and then the vector and human soluble CD4cDNA were extracted and purified. This reaction was operated using GeneCleanII Kit (Bio 101) according to the protocol attached thereto. The two DNAs were ligated using Ligation Kit Ver. 2 (TaKaRa Shuzo) and the ligated product was introduced into Escherichia coli DH5 strain. From the colonies which emerged, several colonies were selected and cultivated and then plasmid DNA was extracted and purified by a conventional method. The digestion patterns of the plasmids were examined using suitable restriction enzymes p102 sent in pcDNA3 and one having the pattern coincided with the expected pattern was selected.

Next, the obtained human soluble CD4 expression plasmid was introduced into COS7 cells by the DEAE dextran method (Bebbington, C. R. (1991); METHODS: A companion to Methods in Enzymology, $\underline{2}$(2), 136-45). Four (4) days before the incorporation of the gene, COS7 cells were re-inoculated in 10% fetal bovine serum (FBS)-added Dulbecco's Modified Eagle's Medium (DMEM) to about $6.1\times10^5$ cells/10 ml per 100mm dish and cultured. After 4 days, first the supernatant was removed, the cells were gently washed with PBS(-), and 4 ml of 10% FBS-added DMEM was added thereto. Then, DEAE dextran/human soluble CD4 expression plasmid mixture was sprayed uniformly on the cells and the cells were incubated at 37°C for 4 hours. The mixture used was prepared by mixing an aqueous 20 mg/ml DEAE dextran (Pharmacia) solution with a solution of 0.17 µg/µl of human soluble CD4 expression plasmid in TBS(-) (20 mM Tris-HCl (pH 7.4), 0.15 M NaCl) in a ratio of 2:1 (v/v). This was added in an amount of 180 µl/dish. After the incubation, the supernatant was discarded and 5 ml of 10% dimethyl sulfoxide (DMSO)-added PBS(-) was added and left to stand for 1 minute. Then, the supernatant was discarded and after the residue was washed with PBS(-), 7 ml of 2% FBS-added DMEM containing 100 µM Chloroquine (Sigma) was added, and the mixture was incubated at 37°C for 3 hours. Thereafter, the supernatant was discarded, the residue was washed with PBS (-), 10 ml of 10% FBS-added DMEM was added and the cells were cultivated. The day next, the FBS-added DMEM was removed sufficiently with PBS(-) and DMEM and 10ml of serum-free DMEM was added and the production was started.

Three days after the initiation of the production, the culture supernatant was recovered and thereafter, the production was continued for about 2 weeks. The obtained culture supernatant was collected and purified by anti-FLAG-M2 gel (Eastman Chemical) column chromatography to obtain a purified preparation of human soluble CD4.

[Example 2]

Anti-human CD4 antibody producing hybridoma 4H5 (Accession No. FERM BP-6729) was cultivated in 10% FBS (ICN)-added DMEM medium (GIBCO). The hybridoma was subjected in advance to a limiting dilution method to separate clones and the culture supernatant was treated by a human soluble CD4 (Repligen)-immobilized ELISA method to select clones having high bindability to human soluble CD4. mRNA was isolated from the obtained $1\times10^7$ cells using QuickPrep mRNA Purification Kit (Pharmacia) according to the protocols attached thereto. Using the obtained mRNA as a template, 1st strand cDNA was synthesized. This was performed using cDNA Synthesis Kit (Pharmacia) according to the protocols attached thereto. Thereafter, the PCR method was practiced to amplify the target gene using cDNA procured as above as a template.

Concerning the procurement of H chain, Mouse Ig-Prime Kit (Novagen) was used as the primer and the gene was amplified from the primers having the sequences of Sequence ID No. 13 in the Sequence Listing (5'GGGAATTCATGRAATGSASCTGGGTYWTYCTCTT3') and Sequence ID No. 14 in the Sequence Listing (5'CCCAAGCTTCCAGGGRCCARKGGATARACNGRTGG3'), respectively. The PCR conditions were such that using GeneAmp PCR Reagent Kit with AmpliTaq DNA Polymerase (TaKaRa Shuzo), a cycle of 94°C for 1 minute, 50°C for 1 minute, and 72°C for 2 minutes was repeated 30 times. The PCR apparatus used was Model name: DNA Thermal Cycler 480 (Perkin Elmer). The amplified gene fragments were cloned using TA cloning kit (Invitrogen).

Concerning the procurement of L chain, preliminarily the amino acid sequence of the N-terminal of L chain was determined for 15 residues from the N-terminal using Protein sequencer (Applied Biosystems, 492 Protein Sequencer) according to the protocol attached thereto. As sense primers, a plurality of nucleotide sequences that can be encoded based on the N-terminal amino acid sequence were synthesized. As the antisense primers, a plurality of candidate sequences that mouse antibody gene cDNA can synthesize were synthesized with reference to the gene sequence of mouse antibody variable region described in Sequences of Proteins of Immunological Interest, 5th edition, 1991 (published by USA NIH). A PCR method was performed using the sense primers and antisense primers in combination. From the primers having the sequences of Sequence ID No. 15 in the Sequence Listing (5'TGTGCCCTCGAGCTNACNCARAGYCCNGC3') and Sequence ID No. 16 in the Sequence Listing (5'ATGGATACTAGTGGTGCAGCATCAGCCC3'), respectively, an L chain variable region gene (partial length) was amplified. The PCR conditions were such that using GeneAmp PCR Reagent Kit with AmpliTaq DNA Polymerase (TaKaRa Shuzo), a cycle of 94°C for 1 minute, 55°C for 1 minute, and 72°C for 2 minutes was repeated 30 times. The PCR apparatus used was Model name: DNA Thermal Cycler 480 (Perkin Elmer). The amplified gene fragments were cloned using TA cloning kit (Invitrogen).

The obtained gene was determined on the nucleotide sequences of the H chain and L chain of the variable region (partial length) using DNA Sequencer ver. 1.2.0, Model 1373 (Applied Biosystems) according to the manufacturer's protocol. The labeling reaction was performed using Universal M13 Reverse primer (Invitrogen) or Universal M13 Forward primer (Invitrogen) as a primer and using PRISM Ready Reaction DyeDeoxy Terminator Cycle Sequencing Kit (Applied Biosystems). The method was in accordance with the protocol attached to the kit. The labeling was performed using a labeling kit of ABI.

Furthermore, concerning the procurement of L chain, the target gene was again amplified by a PCR method in order to obtain a not yet procured region in the N-terminal portion. As the primers, Mouse Ig-Prime Kit (Novagen) and a base chain sequence complimentary to the procured L chain "CDR-3" cDNA were used and genes were amplified from the primers having the sequences of Sequence ID No. 17 in the Sequence Listing (5'GGGAATTCATGGAGACAGACACACTCCTGCTAT3') and Sequence ID No. 18 in the Sequence Listing (5'CGTCGGAGGATCCTCACTACT3'), respectively. The PCR conditions were such that using GeneAmp PCR Reagent Kit with AmpliTaq DNA Polymerase (TaKaRa Shuzo), a cycle of 94°C for 1 minute, 50°C for 1 minute, and 72°C for 2 minutes was repeated 30 times. The PCR apparatus used was Model name: DNA Thermal Cycler 480 (Perkin Elmer). The amplified gene fragments were cloned using TA cloning kit (Invitrogen). Similarly, the nucleotide sequence of the variable region (N-terminal portion) of L chain was determined. Utilizing the restriction enzyme PflMI site and HindIII site in the variable region (partial length) of L chain cloned previously on pCR2.1 plasmid (attached to the TA cloning kit), the genes of the variable region (N-terminal portion) of L chain and the variable region (partial length) of L chain were recombined to construct the gene fragment of an L chain variable region (whole length). The restriction enzyme PflMI was available from New England Biolabs and the restriction enzyme HindIII was available from TaKaRa Shuzo. The extraction and purification after the electrophoresis in agarose gel were operated using Gene CleanII Kit (Bio 101) according to the protocol attached thereto. For the ligation of the two DNAs, Ligation Kit Ver.2 (TaKaRa Shuzo) was used.

The gene sequence of 4H5 antibody H chain variable region (whole length) determined by the above operations is shown by Sequence ID No. 37 together with its amino acid sequence and the gene sequence of L chain variable region (whole length) determined by the above operations is shown by Sequence ID No. 38 together with its amino acid sequence.

[Example 3]

Gene fragments containing the nucleotide sequences of H chain (amino acids 4 to 18) and L chain (amino acids 4 to 111) variable regions of the anti-human CD4 antibody (4H5 antibody) obtained in Example 2 were incorporated into pG1 plasmid to prepare a plasmid capable of expressing an anti-CD4 human-mouse chimera antibody. Using plasmid DNA of the clone containing the gene fragment of H chain variable region as a template and primers having the sequences of Sequence ID No. 19 in the Sequence Listing (5'CAGGATCCGCTGCAGCAGTCTGGACCT3') and Sequence ID No. 20 in the Sequence Listing (5'TGGGCCCGTCGTTTTGGCTGCAGAGAC3'), respectively, a fragment H chain variable region having newly introduced therein ApaI and BamHI restriction enzyme sites were prepared by a PCR method. The PCR conditions were such that using GeneAmp PCR Reagent Kit with AmpliTaq DNA Polymerase (TaKaRa Shuzo), a cycle of 94°C for 1 minute, 55°C for 1 minute, and 72°C for 2 minutes was repeated 30 times. The PCR apparatus used was Model name: DNA Thermal Cycler 480 (Perkin Elmer). The amplified gene fragments were cloned using TA cloning kit (Invitrogen). After digesting the DNA fragment containing the H chain (amino acids 4 to 118) variable region nucleotide sequence with restriction enzyme ApaI (TaKaRa Shuzo) and restriction enzyme BamHI (TaKaRa Shuzo), the product was spread on 2% agarose gel by electrophoresis and the gel was cut out and extracted. The extraction of the DNA fragment from the electrophoresed agarose gel was operated using GeneCleanII Kit (Bio 101) according to the protocol attached thereto.

Next, vector pG1 was digested with restriction enzymes ApaI and BamHI and then ligated to the one similarly cut out from 0.7% agarose gel electrophoresis and extracted. The ligation product was introduced into Escherichia coli JM109 strain. The ligation reaction was performed using Ligation Kit ver. 2 (TaKaRa Shuzo). The transformed Escherichia coli cells were inoculated on an ampicillin-containing LB plate, which was cultivated overnight. Several of the colonies which emerged were selected and plasmid DNAs were extracted and purified by a conventional method. These were digested with the restriction enzymes used upon the incorporation, i.e., restriction enzymes ApaI and BamHI, and one having inserted therein the fragment containing the H chain variable region nucleotide sequence was selected. Next, concerning L chain, plasmid DNA (gene fragment containing an L chain (amino acids 4 to 111) variable region nucleotide sequence) of the clone whose gene was amplified from the primers having the sequences of Sequence ID No. 13 in the Sequence Listing (5'TGTGCCCTCGAGCTNACNCARAGYCCNGC3') and Sequence ID No. 14 in the Sequence Listing (5'ATGGATACTAGTGGTGCAGCATCAGCCC3'), respectively, procured in Example 2 was digested with restriction enzymes XhoI (TaKaRa Shuzo) and SpeI (TaKaRa Shuzo). Thereafter, the digested product was separated by electrophoresis in 1% agarose gel and the gel containing DNA fragment containing an L chain variable region nucleotide sequence was cut out and extracted. The extraction of the DNA fragment from the electrophoresed agarose gel was operated using GeneCleanII Kit (Bio 101) according to the protocol attached thereto. Next, vector pG1 having inserted therein the DNA fragment containing the H chain variable region nucleotide sequence was digested with restriction enzymes XhoI and SpeI and then ligated to the one similarly cut out from 0.7% agarose gel electrophoresis and extracted. The ligation product was introduced into Escherichia coli JM109 strain. The ligation reaction was performed using Ligation Kit ver. 2 (TaKaRa Shuzo). The transformed Escherichia coli cells were inoculated on an ampicillin-containing LB plate, which was cultivated overnight. Several of the colonies which emerged were selected and plasmid DNAs were extracted and purified by a conventional method. These were digested with the restriction enzymes used upon the incorporation, i.e., restriction enzymes XhoI and ApaI, and one having inserted therein the fragment containing the H chain and L chain variable region nucleotide sequences was selected. The secretion type antibody expression plasmid obtained by the above method was named pG14H5.

[Example 4]

The secretion type antibody expression plasmid pG14H5 obtained in Example 3 was introduced into COS7 cells by the DEAE dextran method (Beb-bington, C. R. (1991); METHODS: A companion to Methods in Enzymology, 2(2), 136-45). Four (4) days before the incorporation of the gene, COS7 cells were re-inoculated in 10% fetal bovine serum (FBS)-added Dulbecco's Modified Eagle's Medium (DMEM) to about $6.1 \times 10^5$ cells/10 ml per 100mm dish and cultured. After 4 days, first the supernatant was removed, the cells were gently washed with PBS(-), and 4 ml of 10% FBS-added DMEM was added thereto. Then, DEAE dextran/secretion type antibody expression plasmid mixture was sprayed uniformly on the cells and the cells were incubated at 37°c for 4 hours. The mixture used was prepared by mixing an aqueous 20 mg/ml DEAE dextran (Pharmacia) solution with a solution of 0.17 μg/μl of secretion type antibody plasmid in TBS(-) (20 mM Tris-HCl (pH 7.4), 0.15 M NaCl) in a ratio of 2:1 (v/v). This was added in an amount of 180 μl/dish. After the incubation, the supernatant was discarded and 5 ml of 10% dimethyl sulfoxide (DMSO)-added PBS(-) was added and left to stand for 1 minute.

Then, the culture supernatant was discarded and after the residue was washed with PBS(-), 7 ml of 2% FBS-added DMEM containing 100 μM Chloroquine (Sigma) was added, and the mixture was incubated at 37°C for 3 hours. Thereafter, the supernatant was discarded, the residue was washed with PBS(-), 10 ml of 10% FBS-added DMEM was added and the cells were cultivated. The day next, the FBS-added DMEM was removed sufficiently with PBS(-) and DMEM and 10ml of serum-free DMEM was added and the production was started.

Three days after the initiation of the production, the supernatant was recovered and thereafter, the production was continued for about 2 weeks. The obtained culture supernatant was collected and purified by protein G Sepharose column chromatography to obtain a purified preparation of human-mouse chimera anti-CD4 antibody (hereinafter, referred to as chimera 4H5 antibody).

[Example 5]

The human soluble CD4 prepared in Example 1 was subjected to buffer replacement to 10 mM acetate buffer pH=5.0 using Superdex 200 gel filtration column (Pharmacia) and SMARTsystem (Pharmacia). The human soluble CD4 solution (250 µg/ml, 20 µl) was supplied to different lanes of Sensor Chip CM5 (Pharmacia) at a rate of 5 µl/min) so that it could be immobilized by an amine coupling method to obtain a human soluble CD4 immobilized sensor chip.

The 4H5 antibody or chimera 4H5 antibody (prepared in Example 4) was subjected to buffer replacement to HBSbuffer (Pharmacia) using Superdex 200 gel filtration column (Pharmacia) and SMARTsystem (Pharmacia). Using BIA-core 2000 (Pharmacia), the 4H5 antibody liquid or chimera 4H5 antibody liquid (50 µg/ml, 20 µl) was supplied as an analyte on the human soluble CD4 immobilized sensor chip fabricated in advance at a rate of 5 µl/min to each lane to obtain a sensor-gram upon association. Thereafter, HBSbuffer only was supplied to each lane of the sensor chip at a rate of 5 µl/min to obtain a sensor-gram upon dissociation. The analyses of the sensor-grams gave a dissociation constant of human soluble CD4 and anti-human CD4 antibody (KD: the lower this value is, the higher the binding affinity). As a result, the dissociation constant of 4H5 antibody to human CD4 antigen was such that KD=$8.08 \times 10^{-10}$ M (Kon=$6.60 \times 10^{3}$ $M^{-1}S^{-1}$, Koff=$5.33 \times 10^{-6}S^{-1}$). The dissociation constant of chimera 4H5 antibody was such that KD=$1.24 \times 10^{-9}$ M (Kon=$2.36 \times 10^{4}$ $M^{-1}S^{-1}$, Koff=$2.92 \times 10^{-5}S^{-1}$). This revealed that they can strongly bind to human CD4 antigen.

[Example 6]

A stable high productivity strain which can be used in industrial production of chimera 4H5 antibody was procured. As hosts for use in industrial production, CHO cells, myeloma Sp 2/0 cells are well known (Xiang, J. et al. (1990) Mol. Immun., 27, 809; Bebbington, C. R. et al. (1992) Bio/technology, 10, 169; Larrick, J. W. and Wallace, E. F. et al. (1992) Imunol. Rev. 130, 69-85; Deyev, S. M. and Lieber, A. et al. (1994) Appl. Biochem. Biotechnol. 47 (2-3), 143-54). For example, in the case of CHO cells, a method for selecting clones having high productivity with an agent such as MTX has been reported (Bebbington, C.R.(1991) METHODS: A companion to Methods in Enzymology, 2(2), 136-45) and a stable high productivity strain for producing chimera 4H5 antibody was procured with reference to the above method.

That is, CHOdhfr-strain (ATCCCRL-9096) was adjusted to $1.0 \times 10^{7}$ cells/ml in a buffer for electroporation (272 mM sucrose, 7mM phosphate buffer, 1mM $MgCl_2$, pH=7.4) and 500 µl of this was dispensed in an Electroporation Cuvette (Bio-rad) and ice-cooled. To this, 10 µg of pG14H5 plasmid prepared in Example 3 and 1 µg of pSV2dhfr plasmid (ATCC No. 37146) were mixed and the mixture was ice-cooled for 5 minutes. 3 µF, 0.55 kV electric pulse was applied to the mixture and the mixture was ice-cooled for 1 minute. Again, 3 µF, 0.55 kV electric pulse was applied to the mixture and the mixture was ice-cooled for 5 minutes. The load of electric pulse was achieved by using Gene Pulser of Bio-rad. The cells were transferred to 10 ml of a medium prepared by adding 10% FBS to F12 Medium (GIBCO) and cultivated in a 100 mm tissue culture dish (FALCON) at 37°C under 5% $CO_2$ overnight. The cells were detached from the dish using trypsin-EDTA solution (GIBCO), transferred to 120 ml of a medium prepared by adding 10% dialyzed FBS (GIBCO) to DMEM medium (GIBCO), and inoculated in four 150 mm tissue culture dishes. The medium was replaced by the same medium every 2 or 3 days. In about 2 weeks, colonies were formed to such an extent that can be observed by the naked eyes.

Several tens colonies were isolated and each was cultivated in DMEM medium + 10% dialyzed FBS (GIBCO) to which 20 nM of MTX (Amethopterin; SIGMA) was added. The medium was replaced by the same medium every 2 or 3 days and the cultivation was continued for 1 month. Each of the clone having acquired MTX drug resistance (clone capable of propagating in the same medium) was cultivated in the DMEM medium to which 100 nM of MTX was added + 10% dialyzed FBS medium. The medium was replaced by the same medium every 2 or 3 days and the cultivation was continued for 1 month. Finally, measurement was made of the concentration of chimera 4H5 antibody of the clone having acquired 100 nM MTX drug resistance (clone capable of propagating in the same medium) in the culture supernatant using human Immunoglobulin G (IgG) subclass EIA kit (The Binding site) for IgG1. Then stable high productivity strain producing chimera 4H5 antibody was selected. By the above technique, a stable expression strain which produces chimera 4H5 antibody at a rate of 5 µg/$10^6$ cells/day was created.

[Example 7]

To incorporate antibody genes into ScFv antibody producing vector, the genes described under Sequence ID Nos. 37 and 38 in the Sequence Listing obtained in Example 2 were amplified using primers containing a restriction enzyme sequence and a linker sequence by PCR method. As the primer for L chain, Sequence ID No. 27 in the Sequence Listing (5'AGCCGGCCATGGCCGACATTGTGCTGACCCAATCTCCA3') and Sequence ID No. 28 in the

Sequence Listing

(5'CTCCGGAGCCACCTCCGCCTGAACCGCCTCCACCTTTGATTTCCAGCTTGGTGCCTCC3

')

were used while as the primer for H chain, Sequence ID No. 29 in the Sequence Listing (5'CTCCGGAGGTGGCGGATCGCAGGTTCAGCTGCAGCAGTCT3') and Sequence ID No. 30 in the Sequence Listing (5'TGCGGCCGCTGCAGAGACAGTGACCAGAGTC3') were used. The PCR conditions were such that using GeneAmp PCR Reagent Kit with AmpliTaq DNA Polymerase (TaKaRa Shuzo), a cycle of 94°C for 45 seconds, 58°C for 45 seconds, and 72°C for 1 minute was repeated 18 times. The PCR apparatus used was Model name: DNA Thermal Cycler 480 (Perkin Elmer). The amplified fragments were each cloned using TA cloning kit (Invitrogen). The L chains of the genes were digested with restriction enzymes NaeI (TaKaRa Shuzo) and MroI (Toyobo) and the H chains of the genes were digested with restriction enzymes MroI (Toyobo) and NotI (TaKaRa Shuzo) and then electrophoresed in 2% agarose gel. The gel portion containing each DNA fragment was cut out and extracted. For the extraction of DNA fragment from the agarose gel, Gene CleanII Kit (Bio 101) was used.

After digesting pSE380ScFv or pET24ScFv vector prepared in Reference Example 2 with restriction enzymes NaeI (TaKaRa Shuzo) and NotI (TaKaRa Shuzo), the products were elctrophoresed in 1% agarose gel and the gel containing each DNA fragment was cut out and extracted. This was ligated with the L chain and H chain DNA fragments digested with the restriction enzymes as above. The ligation was performed using Ligation Ver. 2 Kit (TaKaRa Shuzo). For the extraction of DNA fragment from the agarose gel, GeneCleanII Kit (Bio 101) was used. The plasmids obtained by the above operations were introduced into Escherichia coli DH5 strain. The transformed Escherichia coli cells derived from pSE380ScFv vector were inoculated on an ampicillin-containing LB plate while those derived from pET24ScFv vector were inoculated on a kanamycin-containing LB plate, which were cultivated overnight. Several of the colonies which emerged were selected and plasmid DNAs were extracted and purified by a conventional method. The digestion patterns of the plasmids were examined using suitable restriction enzymes and those having the patterns coincided with the expected patterns were selected. The plasmids obtained by the above operations expressing 4H5ScFv (N-terminal VL-linker-VH, C-terminal type: hereinafter LH type) antibodies having the sequence of anti-CD4 antibody were named pSE380ScFv4H5LH or pET24ScFv4H5LH. Sequence ID No. 9 in the Sequence Listing shows the amino acid sequence of single chain antibody (ScFv4H5LH) together with an example of a nucleic acid nucleotide sequence encoding it.

[Example 8]

To incorporate antibody genes into ScFv antibody producing vector, the genes described under Sequence ID Nos. 37 and 38 in the Sequence Listing obtained in Example 2 were amplified using primers containing a restriction enzyme sequence and a linker sequence by PCR method. As the primer for H chain, Sequence ID No.31 in the Sequence Listing (5'AGCCGGCCATGGCCCAGGTTCAGCTGCAGCAGTCT3') and Sequence ID No. 32 in the Sequence Listing

(5'CTCCGGAGCCACCTCCGCCTGAACCGCCTCCACCTGCAGAGACAGTGACCAGAGTC3')

were used while as the primer for L chain, Sequence ID No. 33 in the Sequence Listing (5' CTCCGGAGGTGGCGGATCGGACATTGTGCTGACCCAATCTCCA3') and Sequence ID No. 34 in the Sequence Listing (5'TGCGGCCGCTTTGATTTCCAGCTTGGTGCCTCC3') were used. The PCR conditions were such that using GeneAmp PCR Reagent Kit with AmpliTaq DNA Polymerase (TaKaRa Shuzo), a cycle of 94°C for 45 seconds, 58°C for 45 seconds, and 72°C for 1 minute was repeated 18 times. The PCR apparatus used was Model name: DNA Thermal Cycler 480 (Perkin Elmer). The amplified fragments were each cloned using TA cloning kit (Invitrogen). The H chains of the genes were digested with restriction enzymes NaeI (TaKaRa Shuzo) and MroI (Toyobo) and the L chains of the genes were digested with restriction enzymes MroI (Toyobo) and NotI (TaKaRa Shuzo) and then electrophoresed in 2% agarose gel. The gel portion containing each DNA fragment was cut out and extracted. For the extraction of DNA fragment from the agarose gel, GeneCleanII Kit (Bio 101) was used.

After digesting pSE380ScFv or pET24ScFv vector prepared in Reference Example 2 with restriction enzymes NaeI (TaKaRa Shuzo) and NotI (TaKaRa Shuzo), the products were elctrophoresed in 1% agarose gel and the gel containing each DNA fragment was cut out and extracted. This was ligated with the H chain and L chain DNA fragments digested with the restriction enzymes as above. The ligation was performed using Ligation Ver. 2 Kit (TaKaRa Shuzo). For the extraction of DNA fragment from the agarose gel, GeneCleanII Kit (Bio 101) was used. The plasmids obtained by the above operations were introduced into Escherichia coli DH5 strain. The transformed Escherichia coli cells derived from pSE380ScFv vector were inoculated on an ampicillin-containing LB plate while those derived from pET24ScFv vector were inoculated on a kanamycin-containing LB plate, which were cultivated overnight. Several of the colonies which emerged were selected and plasmid DNAs were extracted and purified by a conventional method. The digestion patterns of the plasmids were examined using suitable restriction enzymes and those having the patterns coincided with the expected patterns were selected. The plasmids obtained by the above operations expressing 4H5ScFv (N-terminal VH-linker-VL, C-terminal type: hereinafter HL type) antibodies having the sequence of anti-CD4 antibody were named pSE380ScFv4H5HL or pET24ScFv4H5HL. Sequence ID No. 10 in the Sequence Listing shows the amino acid sequence of single chain antibody (ScFv4H5HL) together with an example of a nucleic acid nucleotide sequence encoding it.

[Example 9]

The Escherichia coli DH5 strain transformed with pSE380ScFv 4R5LH plasmid in Example 7 was cultivated on 100 µg/ml ampicillin and 1% glycerol-added 2×YT medium (1.6% bactotryptone, 1% bactoyeast extract, 0.5% NaCl). The day next, a portion of it was added in a 10 fold amount of the above medium. After 1 hour's cultivation, the supernatant was discarded and the medium was replaced by the equivalent amount of 1 mM IPTG, 100 µg/ml ampicillin and 1% glycerol-added 2×YT medium. After cultivating for additional 3 hours, the cells after removal of the supernatant were suspended in 1/10 fold the volume of the culture of ice-cooled TES solution (200 mM Tris-HCl (pH=8.0), 0.5 mM EDTA, 0.5 M sucrose). The suspension was allowed to stand at ice temperature for 10 minutes, and centrifuged at 14,000 rpm at 4°C for 20 minutes. The supernatant was discarded and the residue was suspended in 1/10 fold the culture volume of ice-cooled TE solution (10 mM Tris-HCl (pH=8.0), 0.5 mM EDTA) . The suspension was allowed to stand at ice temperature for 30 minutes, and centrifuged at 14,000 rpm at 4°C for 20 minutes for separation to recover antibodies in periplasm. The obtained crude antibody fraction was purified by anti-FLAG-M2 gel (Eastman Chemical) column chromatography and further purified by TALON metal affinity gel (Clontech). The column chromatography was performed according to the protocol attached to each gel. This was a purified preparation of anti-human CD4 single chain antibody (ScFv4H5LH).

A PBS(-) solution (5.2 µg/ml) of human soluble CD4 prepared in Example 1 was dispensed in the wells of polystyrene 96-well plate in an amount of 50 µl/well and left to stand at 4°C overnight to immobilize the antigen. The supernatant was discarded and the residue precipitates were washed with 250 µl of 0.05% Tween 20-added PBS(-) (hereinafter, referred to as PBS-T). 100µl of Block Ace (Dainippon Seiyaku) was dispensed in each well and left to stand at room temperature for 1 hour to effect blocking. After discarding the supernatant from each well, the precipitates were washed twice with 250 µl of PBS-T. The supernatant, 50 µl of the above prepared PBS(-) solution of 10 µg/ml anti-human CD4 single chain antibody (ScFv4H5LH) was dispensed and left to stand at room temperature for 2 hours for reaction. The supernatant was discarded and the residue was washed with 250 µl of PBS-T three times. After the supernatant was discarded, 50 µl of horseradish peroxidase-labeled anti-myc antibody (Invitrogen) 1,000 fold-diluted with PBS-T was dispensed in each well and left to stand at room temperature for 2 hours. The supernatant was discarded and the residue was washed with 250 µl of PBS-T three times. The supernatant was discarded and 50 µl of a coloring reagent solution prepared by adding 0.01% $H_2O_2$ to a PBS(-) solution of orthophenylenediamine (SIGMA) was added and allowed to react at room temperature for 7 minutes. Then 50 µl of 2N sulfuric acid solution was added to stop the enzyme reaction. The absorbance of the plate at 490 nm was measured. The absorbance measured was 0.574. The same experiments were repeated except that only the immobilization operation of human soluble CD4 was omitted resulted in absorbance of 0.109. These results indicated that anti-human CD4 single chain antibody (ScFv4H5LH) recognized human CD4 and had affinity therefor.

[Example 10]

2-Iodoacetamide (Tokyo Kasei) (50 g) was suspended in 100 ml of distilled water and 20 ml of 37% formaldehyde (Wako Pure Chemicals Industries, Ltd.) was added thereto. The mixture was dissolved at 50°C with stirring. 25.2 g of potassium carbonate was added slowly and the mixture was stirred for 10 minutes by a stirrer and then left to stand at room temperature for 2 hours. Further, after the mixture was left to stand at 4°C overnight, the supernatant was discarded by decantation. After 40 ml of distilled water was added thereto, the precipitates were dissolved at 50°C and the solution was left to stand at room temperature for 2 hours and further at 4°C overnight. This procedure was repeated once again, the resulting precipitates were recovered by passing them through G-3 glass filter (filter diameter 30 mm: Nippon Rikagaku Kikai Co., Ltd.). The precipitates were dried by suction using an aspirator for 1 hour and then freeze-dried for 24 hours to prepare N-hydroxymethyl iodoacetamide, which is an activating group capable of being introduced to insoluble carriers for use in immobilizing antibodies.

In a glass flask were charged 5.7 ml of sulfuric acid, 7.2 ml of nitrobenzene, and 0.0363 g of paraformaldehyde, which were dissolved with stirring. Thereafter, 0.58 g of N-hydroxymethyliodoacetamide was added and the mixture was stirred. To this 0.3 g of polypropylene nonwoven fabric (average fiber diameter 3.3 μm) was added and allowed to react at room temperature overnight. After the reaction, the nonwoven fabric was taken out, washed with pure water, and vacuum dried to obtain nonwoven fabric to which an activated group for immobilizing antibodies was introduced. The nonwoven fabric was cut to disks of 0.7 cm in diameter. Four disks each were dipped in a solution of anti-human CD4 antibody (4H5 antibody) dissolved in PBS(-) (17.7 μg/400 μl) or a solution of the anti-human CD4 single chain antibody (ScFv4H5LH) (17.7 μg/400 μl) at room temperature for 2.5 hours to immobilize the antibodies. After 2.5 hours, they were washed with PBS(-). In a 1ml container having inlet and outlet were filled the four disks to make a column. As a comparative control, a column was made to which bovine serum albumin (BSA; Pierce Albumin standard) was immobilized in the same manner instead of the antibody.

In the same manner as in Example 1, cells of mononuclear cell layer were recovered by density gradient centrifugation using Ficoll-Paque (Pharmacia) from healthy human blood. The recovered cells were washed with PBS(-) and adjusted to $1.2 \times 10^6$ cells/ml. 4 ml of the mononuclear cell suspension was delivered at a flow rate of 1 ml/min using a syringe pump, and was flown from the inlet of the column fabricated as above. The liquid after the treatment was recovered from the outlet of the column. The ratio of CD4-positive cells was measured by a known flow cytometry method (FACS). The staining of the cells upon FACS analysis was made using FITC-labeled anti-CD4 antibody (Fermingen) and measured using FACSCalibur (Becton Dickinson).

Table 1 shows the ratios of CD4-positive cells and other cells (CD4-negative cells) before and after the passage through the column. In this Example, the elimination ratio of CD4-positive cells after the passage through the 4H5 antibody-immobilized column was 100% while the elimination ratio after the passage through the ScFv4H5LH-immobilized column was 99.9%. These results indicated that upon comparison with 4H5 antibody, the anti-human CD4 single chain antibody (ScFv4H5LH) had substantially the same functions as those of 4H5 antibody in terms of binding affinity for and specificity to CD4-positive cells.

Table 1

|  | Ratio of CD4-positive Cells | Ratio of other cells |
|---|---|---|
| Before passage through column | 39.3% | 60.7% |
| After passage through 4H5 antibody-immobilized column | 0.3 | 99.7 |
| After passage through ScFv4H5LH-Immobilized column | 2.6 | 97.4 |
| After passage through bovine Serum albumin-immobilized column | 45.0 | 55.0 |

[Example 11]

The 4H5 hybridoma was procured as follows.

Blood was overlayed on Ficoll-High-Paque and after centrifugation, mononuclear leukocyte layer was recovered. Further, CD8-positive cells were eliminated by an immunoprecipitation method with anti-CD8 antibody to obtain a fraction in which CD4 lymphocyte was concentrated. The cell fraction was stimulated with PHA lectin (E. Y. Laboratory) to prepare cells for immunization. After the cells were washed with DMEM medium (Gibco), they were mixed with Titer Max (CytRx), adjuvant, and the mixture was intraperitoneally administered to Balb/c mouse (Japan Charles River) in a dose of about $2 \times 10^7$ cells per mouse and immunized. Every 2 weeks starting from the initial immunization, booster immunization was performed. Thereafter, 3 months after the initial immunization, cells prepared in the same manner were administered for booster immunization by intravenous injection.

NS1 cell line (ATCC TIB-18), which was a BALB/c mouse-derived bone marrow cell line, was subcultured in 20% FCS-added DMEM medium (Gibco).

The spleen procured from the above immunized animal, mouse, was macerated, filtered through mesh while washing with DMEM medium, and centrifuged to separate spleen cells. The spleen cells and the above propagated NS1 cell line were mixed and then centrifuged. To the mixed cells, polyethylene glycol (Boehringer-Mannheim) was added to a final concentration of 30% to form a suspension.

The cells were separated by centrifugation and slowly dispersed in DMEM tissue culture medium (GIBCO) containing 20% fetal bovine serum using mouse spleen cells as a feeder. Then, in the wells of a flat bottomed 96-well microtiter plate (Nunc) were inoculated the cells in a population of $10^6$ cells/100 $\mu$l and cultivated at 37°C in 5% carbon dioxide.

One day after the cell fusion, 100 $\mu$l of HAT medium (the above growth medium was supplemented so as to contain 13.61 $\mu$g/ml hypoxanthine, 3.88 $\mu$g/ml thymidine, and 0.18 M aminopterin) was added. Thereafter, for 3 days, about half the HAT medium was replaced by fresh HAT medium every day. Thereafter, the medium was replaced in the same manner every 2 or 3 days. The clones of hybridoma (fused cell) were cultivated in HT medium (HAT medium containing no aminopterin) and preserved.

Several cells of the above hybridoma were subcloned by a limiting dilution method. The cell number of the hybridoma was counted by a Trypan Blue dye exclusion method and using a hemocytometer. The hybridoma was suspended in HT medium in a growing cell ratio of 0.5 cell/100 $\mu$l of HT medium and the suspension was dispensed in the wells of flat bottomed 96-well microplate in an amount of 100 $\mu$l/well. The medium was replaced with fresh one every 2 or 3 days to propagate the hybridoma.

The culture supernatant of the hybridoma obtained was allowed to react with human leukocyte to detect an antibody which recognizes CD4-positive cells of human leukocyte. The detection was performed using FACScan (Becton Dickinson). By this operation, 4H5 cells excellent in specificity and bindability were successfully procured from the obtained antibody group.

[Example 12]

Production and purification of 4H5 antibody were performed. Anti-human CD4 antibody producing hybridoma 4H5 (Accession number FERM BP-6729) was cultivated in 10% fetal bovine serum (FBS) (ICN)-added DMEM medium (GIBCO). The hybridoma was subjected in advance to a limiting dilution method to separate clones and the culture supernatant was treated by a human soluble CD4 (Repligen)-immobilized ELISA method to select clones having high bindability to human soluble CD4. The 4H5 hybridoma clone was transplanted to in the abdominal cavity of Balb/c mouse and propagated. The obtained ascites was purified by affinity chromatography using Protein A Sepharose (Pharmacia) to obtain a purified preparation of 4H5 antibody.

[Example 13]

A PBS(-) solution (5.2 $\mu$g/ml) of human soluble CD4 prepared in Example 1 was dispensed in the wells of polystyrene 96-well plate (NUNC) in an amount of 50 $\mu$l/well and left to stand at 4°C overnight to immobilize the antigen. The supernatant was discarded and the precipitates were washed with 250 $\mu$l of 0.05% Tween 20-added PBS(-) (hereinafter, referred to as PBS-T). 100$\mu$l of Block Ace (Dainippon Seiyaku) was dispensed in each well and left to stand at room temperature for 1 hour to effect blocking. The supernatant was discarded from each well, the residue was washed with 250 $\mu$l of PBS-T twice. The supernatant was discarded and 50 $\mu$l each of PBS(-) solution of 4H5 antibody (prepared in Example 12, isotype was IgG1-$\kappa$) or MT310 antibody (DACCO, isotype was IgG1-$\kappa$) adjusted to various concentrations was dispensed and left to stand at room temperature for 2 hours for reaction. The supernatant was discarded, the residue was washed with 250 $\mu$l of PBS-T three times. After the supernatant was discarded, 50 $\mu$l of biotin-labeled anti-mouse IgG (H+L) antibody (VECTOR) diluted 200 folds with PBS-T was dispensed in each well and left to stand at room temperature for 1 hour. After the supernatant was discarded from each well, the residue was washed with 250 $\mu$l of PBS-T three times. After the supernatant was discarded, 50 $\mu$l of an equivolume mixture of A solution and B solution of vectastain Elite standard kit (VECTOR) diluted 100 folds with PBS-T was dispensed in each well and left to stand at room temperature for 1 hour. After the supernatant was discarded from each well, the residue was washed with 250 $\mu$l of PBS-T 6 times. After the supernatant was discarded , 50 $\mu$l of a coloring reagent solution prepared by adding 0.01% $H_2O_2$ to a PBS(-) aqueous solution of orthophenylenediamine (SIGMA) was added and allowed to react at room temperature for 3 minutes. Then 50 $\mu$l of 2N sulfuric acid solution was added to stop the reaction. The absorbance of the plate at 490 nm was measured.

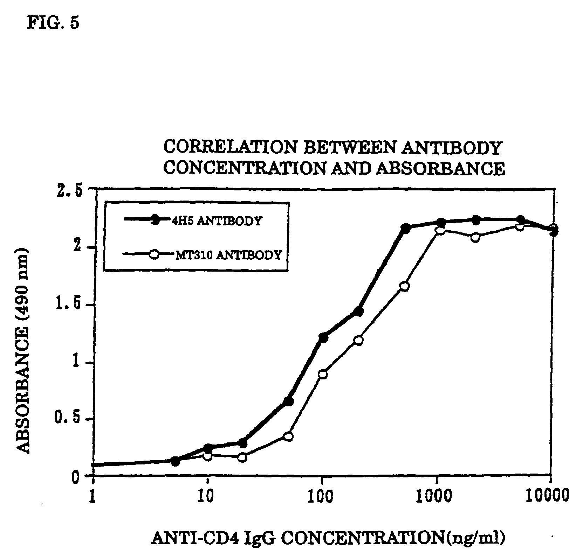

Fig. 5 illustrates the relationship between the concentration of antibody (4H5 antibody or MT310 antibody) used and absorbance. It revealed that 4H5 antibody could detect CD4 molecules at a lower concentration than MT310 antibody and had higher affinity for human CD4 molecules than MT310 antibody.

[Example 14]

4H5 Antibody (prepared in Example 12, isotype was IgG1-$\kappa$) and 13B8.2 antibody (IMUNOTECH, isotype was IgG1-$\kappa$) were subjected to buffer replacement to 10 mM acetate buffer pH=5.0 using Superdex 200 gel filtration column (Pharmacia) and SMARTsystem (Pharmacia). The antibody solutions (100 $\mu$g/ml, 70 $\mu$l) were supplied to different lanes of Sensor Chip CM5 (Pharmacia) at a rate of 5 µl/min so that it could be immobilized by an amine coupling method to obtain an anti-human CD4 antibody immobilized sensor chip. Next, the human soluble CD4 prepared in Example 1 was subjected to buffer replacement to HBSbuffer (Pharmacia) using Superdex 200 gel filtration column (Pharmacia) and SMARTsystem (Pharmacia). Using BIA-core 2000 (Pharmacia), the human soluble CD4 (15.2 µg/ml, 20 µl) was supplied as an analyte at a rate of 5 µl/min on the antibody immobilized sensor chip fabricated in advance to each lane of the sensor chip to obtain a sensor-grain upon association. Thereafter, HBSbuffer only was supplied to each lane of the sensor chip at a rate of 5 µl/min to obtain a sensor-gram upon dissociation. The analyses of the sensor-grams gave dissociation constant of human soluble CD4 and anti-human CD4 antibody (KD: the lower this value is, the higher the binding affinity).

As a result, the dissociation constant of 4H5 antibody was such that $KD=1.71 \times 10^{-9}M$ ($Kon=1.35 \times 10^{5}M^{-1}S^{-1}$, $Koff=2.31 \times 10^{-4}S^{-1}$). The dissociation constant of 13B8.2 antibody was such that $KD=7.58 \times 10^{-9}$ M ($Kon=1.82 \times 10^{4}$ $M^{-1}S^{-1}$, $Koff=1.38 \times 10^{-4}S^{-1}$). Comparison of the dissociation constants revealed that 4H5 antibody had a binding affinity for human soluble CD4 by about 4.4 times higher than 13B8.2 antibody.

[Example 15]

A stable anti-CD4 antibody-containing preparation was produced. The 4H5 antibody obtained in Example 12 was aseptically replaced by 20 mM phosphate buffer, pH=7.4 (PBS) containing 100 mM sodium chloride by gel filtration. 1 mg of the obtained 4H5 antibody and 200 mg of human serum albumin were mixed with PBS to make 2ml. This was aseptically injected into a glass vial and sealed.

[Example 16]

A stable human-mouse chimera anti-CD4 antibody-containing preparation was produced. The chimera 4H5 antibody obtained in Example 4 was aseptically replaced by 20 mM phosphate buffer, pH=7.4 (PBS) containing 100 mM sodium chloride by gel filtration. 1 mg of the obtained chimera 4H5 antibody and 200 mg of human serum albumin were mixed with PBS to make 2 ml. This was aseptically injected into a glass vial and sealed.

[Example 17]

A stable single chain anti-CD4 antibody-containing preparation was produced. The anti-human CD4 single chain antibody (ScFv4H5LH) obtained in Example 9 was aseptically replaced by 20 mM phosphate buffer, pH=7.4 (PBS) containing 100mM sodium chloride by gel filtration. 500 µg of the obtained single chain antibody and 200 mg of human serum albumin were mixed with PBS to make 2 ml. This was aseptically injected into a glass vial and sealed.

[Example 18]

To isolate antibody genes, hybridoma Anti-My-10 (ATCC HB-8483) was cultivated in 10% fetal bovine serum (Flow)-added RPMI-1640 medium (Gibco). The hybridoma was subjected in advance to a limiting dilution method to separate clones and the culture supernatant was assayed to select clones having high bindability to human acute bone marrow leukemia cell KG-1a. Total RNA was separated from the obtained $6 \times 10^{7}$ cells by the AGPC method (Chomczynski, P. and Sacchi, N. (1987) Single-step method of RNA isolation by acid guanidinium thiocyanate-PhOH-chloroform extraction, Anal. Biochem., 162, 156-159). Furthermore, from the separated RNA, mRNA was isolated using QuickPrep mRNA Purification Kit (Pharmacia). Using the obtained mRNA as a template, 1st strand cDNA was synthesized. This was performed using cDNA Synthesis Kit (Pharmacia) according to the protocol attached thereto.

Thereafter, the PCR method was practiced to amplify the target gene. As for the primer, a plurality of candidate sequences that mouse antibody gene cDNA can be synthesize were synthesized with reference to the gene sequence of mouse antibody variable region described in Sequences of Proteins of Immunological Interest, 5th edition, 1991 (published by USA NIH), and PCR method was conducted combining these primers. Genes were amplified from the primers having the nucleic acid sequences (5'GTCCCAGGATCCTCTGAAGCAGTCAGGCCC3') and (5'ACAGTGGGCCCGTCGTTTTGGCTGAGGAGA3') concerning the procurement of H chain and from the primers having the nucleic acid sequences (5'TGTGCCCTCGAGGTGACTCAAACTCCACTCTC3') and (5'ATGGATACTAGTGGTGCAGCATCAGCCC3') concerning the procurement of L chain. The amplified gene fragments were cloned using TA cloning kit (Invitrogen). The obtained gene was determined on the nucleotide sequences of the H chain and L chain of the variable region using DNA Sequencer ver. 1.2.0, Model 373 (Applied Biosystems) according to the protocol attached thereto. The labeling reaction was performed using a nucleic acid sequence (5'CTCTTGGAGGAGGGTGCCAG3') for H chain and a nucleic acid sequence of (5'CCAGATTTCAACTGCTCATCAGA3') for κ chain as primers and using PRISM Ready Reaction DyeDeoxy Terminator Cycle Sequencing Kit (Applied Biosystems). The method was in accordance with the protocol attached to the kit. The staining was performed using a labeling kit of ABI. As a result, the gene fragment obtained from the primer of H chain is shown by Sequence ID No. 39 and the gene fragment obtained from the primer of L chain is shown by Sequence ID No. 40.

[Example 19]

The gene fragments containing the nucleotide sequences of H chain and L chain variable regions of the anti-CD34 antibody obtained in Example 18 were incorporated into pG1 plasmid to prepare a plasmid capable of expressing an anti-CD34 antibody. After the L chain of the plasmid DNA of the clone was digested with restriction enzymes XhoI (TaKaRa Shuzo) and SpeI (TaKaRa Shuzo), the product was spread on 0.7% agarose gel by electrophoresis and the gel containing DNA fragment including L chain variable region nucleotide sequence was cut out and extracted. The extraction of the DNA fragment from the electrophoresed agarose gel was operated using GeneCleanII Kit (Funakoshi) according to the protocol attached thereto. Next, vector pG1 was digested with restriction enzymes XhoI and SpeI and then ligated to the one similarly cut out from 0.7% agarose gel electrophoresis and extracted. Then, the DNA fragment containing H chain variable region nucleotide sequence of plasmid DNA of the clone was cleaved with restriction enzymes ApaI (TaKaRa Shuzo) and BamHI (TaKaRa Shuzo) and extracted. The extraction of the DNA fragment from the electrophoresed agarose gel was operated using GeneCleanII Kit (Funakoshi) according to the protocol attached thereto. Next, after the vector pG1 to which the DNA fragment containing H chain variable region nucleotide sequence had been inserted as described above was digested with restriction enzymes ApaI and BamHI, the digestion product was ligated to the one cleaved and extracted from 0.7% agarose gel electrophoresis and the ligation product was introduced into Escherichia coli JM109 strain. The ligation reaction was performed using Ligation Kit ver. 2 (TaKaRa Shuzo). The transformed Escherichia coli cells were inoculated on an ampicillin-containing LB plate, which was cultivated overnight. Several of the colonies which emerged were selected and plasmid DNAs were extracted and purified by a conventional method. These were digested with the restriction enzymes used upon the incorporation, i.e., restriction enzymes XhoI and ApaI, and one having inserted therein the fragment containing the H chain and L chain variable region nucleotide sequences was selected. The secretion type antibody expression plasmid obtained by the above method was named pG1My10.

[Example 20]

The secretion type antibody expression plasmid pG1My10 obtained in Example 19 was introduced into COS7 cells by the DEAE dextran method (Beb-bington, C. R. (1991); METHODS: A Compa-nion to Methods in Enzymology, 2(2), 136-45). Four (4) days before the incorporation of the gene, COS7 cells were re-inoculated in 10% fetal bovine serum (FBS)-added Dulbecco's Modified Eagle's Medium (DMEM) to about $6.1 \times 10^5$ cells/10 ml per 100mm dish and cultured. After 4 days, first the supernatant was removed, the cells were gently washed with phosphate buffered saline (not containing calcium and magnesium) (PBS(-)), and 4 ml of 10% FCS-added Dulbecco's Modified Eagle's Medium (DMEM) was added thereto. Then, DEAE dextran/secretion type antibody expression plasmid mixture was sprayed uniformly on the cells and the cells were incubated at 37°C for 4 hours. The mixture was prepared by mixing an aqueous 20 mg/ml DEAE dextran (Pharmacia Code No. 1703 50-01, Lot. PF97323) solution with a solution of 0.17 μg/μl of secretion type antibody plasmid in TBS(-) (20 mM Tris-HCl (pH 7.4), 0.15 M NaCl) in a ratio of 2:1 (v/v). This was added in art amount of 180 μl/dish. After the incubation, the supernatant was discarded and 5 ml of 10% dimethyl sulfoxide (DMSO)-added PBS(-) was added. The mixture was left to stand for 1 minute. Then, the supernatant was discarded and after washing the residue with PBS(-), 7 ml of 2% FBS-added DMEM containing 100 μM Chloroquine (Sigma, No. C6628) was added, and the mixture was incubated at 37°C for 3 hours. Thereafter, the supernatant was discarded, the residue was washed with PBS(-), 10ml of 10% FCS-added DMEM was added and the cells were cultivated. The day next, the FCS-added DMEM was removed sufficiently with PBS(-) and DMEM and 10ml of serum-free DMEM was added to thereby start the production.

Three days after the initiation of the production, the culture supernatant was recovered and thereafter, the production was continued for about 2 weeks. The obtained culture supernatant was collected and purified by protein G Sepharose column chromatography to obtain a purified preparation of human-mouse chimera anti-CD34 antibody.

The binding of the purified preparation to CD34 antigen was confirmed by the following method. In a 1.5-ml tube culture cell KG1a was prepared to $1 \times 10^6$ cells and the tube was washed with cell suspension (2% FCS-added PBS(-)) twice. To the washed cells was added 50 μl of the cell suspension to suspend the cells. To this was added 1 μg of purified preparation and the mixture was left to stand in ice for 30 minutes. Thereafter, the cells were further washed with the cell suspension 3 times. And then 50 μl of the cell suspension was added to suspend the cells. To this was added 1 μg of FITC-labeled goat anti-human IgG (Fc) antibody (1/20 dilution, Immunotec) and the mixture was left to stand on ice for 30 minutes. Thereafter, the cells were further washed with the cell suspension 3 times. Thereafter, 300

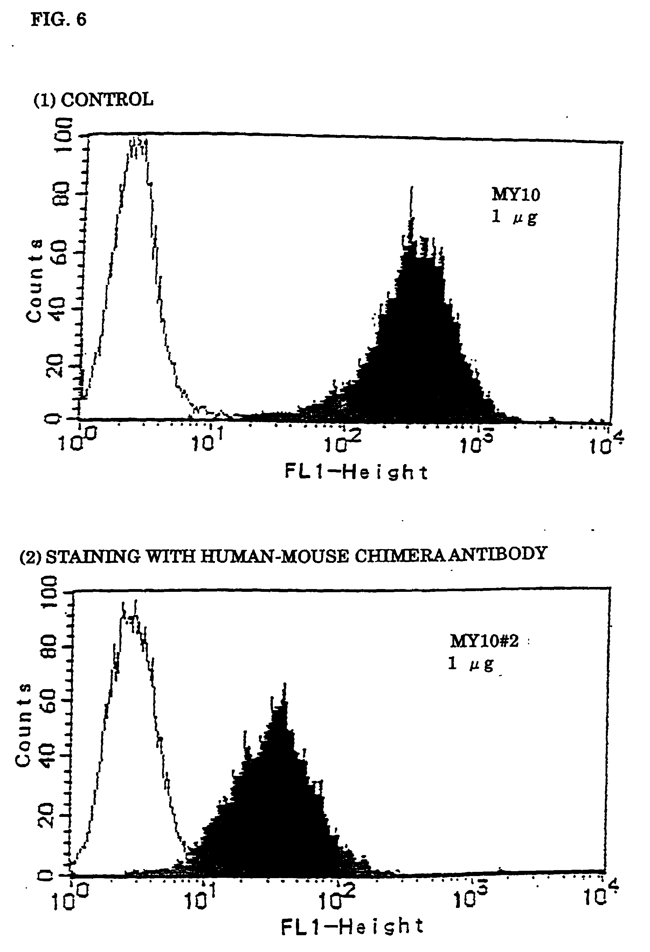

μl of the cell suspension was added to suspend the cells. The thus treated cells were irradiated laser beam by a flow cytometer (Becton Dickinson) and the antibody titer bound on the cell was measured by fluorescence. As a control, 1 μg of myeloma-derived human IgG antibody was added instead of the purified preparation and the cells were treated by the same operation as above. Fig. 6 illustrates binding of the purified preparation to CD34 antigen. It was confirmed that the anti-CD34 antibody pG1MY10-derived purified preparation retained binding activity to CD34 antigen and the present human-mouse chimera anti-CD34 antibody was demonstrated to have a binding activity to CD34 antigen. Also, it was confirmed that CD34-positive cells could be detected by the present purified preparation.

[Example 21]

To incorporate antibody genes into ScFv antibody producing vector, the genes obtained in Example 19 were amplified by a PCR method using primers containing restriction enzyme sequences. The primers used for H chain had nucleic acid sequences (5'GCGGCCCAGCCGGCCATGGCCCAGGTGCAGCTGAAGCAGTCAG3') and (5'AGACGGTGACCGTGGTGCCTTGGCCCC3') and those for L chain had nucleic acid sequences of (5'TCGAGCTCACTCAGTCTCCACTCTCCCTGCCT3') and (5'CACCTGCGGCCGCCCGTTTCAGCTC3'). The PCR conditions were such that using GeneAmp PCR Reagent Kit with AmpliTaq DNA Polymerase (TaKaRa Shuzo), a cycle of 94°C for 1 minute, 55°C for 1 minute, and 72°C for 2 minutes was repeated 30 times. The PCR apparatus used was Model name: DNA Thermal Cycler 480 (Perkin Elmer). The H chains of the amplified genes were digested with restriction enzymes SfiI (Toyobo) and BstPI (TaKaRa Shuzo) and the L chains of the amplified genes were digested with restriction enzymes SacI (TaKaRa Shuzo) and NotI (TaKaRa Shuzo) and then electrophoresed in 1.5% agarose gel. The gel portions containing each DNA fragment was cut out and extracted. For the extraction of DNA fragment from the agarose gel, Gene-CleanII Kit (Funakoshi) was used.

As for the vector producing ScFv antibody, a plasmid producing any desired antibody gene was prepared in advance before the anti-CD34 antibody gene could be introduced. Using a Recombinant Phage Antibody System kit (Pharmacia), any desired gene of hybridoma which produces antibody was incorporated. The method for preparing the vector was in accordance with the protocol attached to the kit.

After the plasmid containing any desired antibody gene was digested with restriction enzymes SacI (TaKaRa Shuzo) and NotI (TaKaRa Shuzo), the digesting product was electrophoresed in 0.8% agarose gel and a vector DNA fragment in which L chain region was deleted was extracted and purified. The DNA fragment and the previously prepared L chain gene of anti-CD34 antibody were ligated to incorporate the L chain gene. Then, after the L chain-replaced plasmid was digested with restriction enzymes SfiI (Toyobo) and BstPI (TaKaRa Shuzo), the digestion product was electrophoresed in 0.8% agarose gel and a vector DNA fragment in which H chain region was deleted was extracted and purified. This DNA fragment and the previously prepared H chain gene of anti-CD34 antibody were ligated to incorporate the H chain gene. For each ligation, Ligation ver. 2 Kit (TaKaRa Shuzo) was used. The extraction of the DNA fragment from the agarose gel was operated using GeneCleanII Kit (Funakoshi). The plasmid obtained by the above operations was introduced in Escherichia coli HB2151 (Pharmacia). The transformed Escherichia coli cells were inoculated on an ampicillin-containing 2% Glucose-added 2xYT plate and cultivated overnight. Several of the colonies which emerged were selected and plasmid DNAs were extracted and purified by a conventional method. The cleaving patterns of the plasmids were examined using suitable restriction enzymes and those having the patterns coincided with the expected patterns were selected. The plasmid obtained by the above operations expressing ScFv antibody having the sequence of anti-CD34 antibody was named pCANMY10.

The Escherichia coli cells transformed with the above plasmid was cultivated on 100 μg/ml ampicillin-added SB medium to which 2% glucose was added (3.5% bactotryptone, 2% bactoyeast extract, 0.5% NaCl). The day next, a portion of it was added in a 10 fold amount of the above medium. After 1 hour's cultivation, the supernatant was discarded and the medium was replaced by the equivalent amount of 1 mM IPTG- and 100 μg/ml ampicillin-added SB medium. After cultivating for additional 3 hours, the cells were treated according to the protocol of RPAS Purification Module (Pharmacia) to recover antibody in periplasm. The obtained crude antibody fraction was purified using an Etag antibody column (Pharmacia). The purification using the column was performed using the attached reagent according to the protocol attached thereto. By the above operations, a purified preparation of ScFv antibody was obtained.

[Example 22]

Using the purified preparation of anti-CD34 ScFv antibody obtained in Example 21, detection of human CD34 antigen was performed. $1\times10^7$ KG-1a cells were treated with 1 ml of PBS(-) containing 0.05% TritonX100 (Nakarai Tesc) on ice for 10 minutes to perform extraction of protein from the cell membrane. The KG-1a cells used had been cultivated in RPMI-1640 medium containing 10% fetal bovine serum. The extract solution (10 μl) was boiled for 3 minutes in the presence of mercaptoethanol. The solution was spread using SDS polyacrylamide electrophoresis gel (ACI). The electrophoresis was carried out under the conditions described in manual attached to the gel. After the electrophoresis, the proteins in the gel were transferred onto PVDF membrane (BioRad). The transfer was performed using 25 mM Tris 192 mM glycine buffer and Electrophoresis power Supply-EPS 600 apparatus (Pharmacia) at 100 V for 1 hour. The filter was blocked with 10% skimmed milk-added PBS(-) at room temperature for 1 hour. The filter was washed with 0.05% Tween-20 and then reacted with 15 µg/ml single chain antibody for 1 hour.

Next, the filter was washed and allowed to react with 5 µg/ml anti-E-tag antibody (Pharmacia) as a secondary antibody. Further, the filter was washed and allowed to react with 5 µg/ml peroxidase-labeled anti-mouse Fc antibody as a tertiary antibody for 1 hour. After washing the filter, the band of electrophoresis to which the primary antibody was bound was treated with ECL solution (Amersham) to emit light and detected using a high sensitivity film (Hyperfilm-ECL: Amersham). As a result, like positive control, the band of CD34 molecule was detected at the position of about 120 kD. As the positive control, a filter was prepared by using anti-HPCA-1 antibody (Becton Dickinson) as a primary antibody and peroxidase-labeled anti-mouse Fc antibody as a secondary antibody and allowed to develop color in the same manner. As a negative control, only the single chain antibody, primary antibody, was eliminated and treated in the same manner. No band of 120 kD was detected. Therefore, it was confirmed that the anti-CD34 single chain antibody could detect CD34 molecules.

[Example 23]

Cord blood (29 ml) was made 48 ml with Hanks' balanced physiological saline (Gibco) and 12 ml aliquot was dispensed in each of four centrifuge tubes to each of which 3 ml of Ficoll-Paque (Pharmacia) had been dispensed and separation was performed under the conditions of 800 rpm for 20 minutes to recover cells of mononuclear cell layer. The recovered cells were washed with the Hanks' solution. To $2.0 \times 10^7$ such cells was added 5 µg of human-mouse chimera antibody produced in Example 20 to make 1 µl/ml and allowed to react on ice for 30 minutes. After the cells were washed with the Hanks' solution, $9 \times 10^6$ Dyna bead (Dynal) anti-human Ig beads were added and the mixture was allowed to react on ice for 1 hour. The separation of Dyna beads was operated according to the attached manual. The obtained cells were cultivated in 10% fetal bovine serum-added Dulbecco MEM medium in a 5% carbon dioxide atmosphere at 37°C for 14 hours. After the cultivation, the cells and beads were completely separated by pipetting. The beads were removed by a magnet according to the manual attached to the Dyna beads. A portion of the recovered cells was used for the measurement of cell number to obtain number of recovered cells. The remaining cells were allowed to react with anti-HPCA-2 antibody (Becton) on ice for 30 minutes, washed with cold physiological saline containing 2% fetal bovine serum, and recovered by centrifugation. The obtained cells were measured of CD34-positive cell ratio using a flow cytometer (Coulter). CD34-positivity ratio was assumed as purity of CD34 cell and the cell number obtained by multiplying CD34-positivity ratio by number of recovered cell was assumed as number of recovered CD34-positive cells. The recovery ratio of CD34-positive cells was calculated from the number of positive-cells before separation and the number of recovered positive cells. As a result, the CD34-positive cell had a purity of 80.4% and a recovery ratio of 45.3%. This revealed that the human-mouse chimera antibody produced in Example 20 separated CD34-positive cells.

[Example 24]

An antibody column to which the human-mouse chimera antibody produced in Example 20 was bound was prepared utilizing CNBr activated Sepharose4B (Pharmacia). The antibody was bound to Sepharose4B beads by the method according to the manual attached thereto. The human-mouse chimera antibody liquid was allowed to react with 4.0 ml of the gel to prepare an affinity gel with a coupling efficiency of 99.5%. A glass column ($2 \text{ cm}^2 \times 3$ cm) having a small amount of glass wool immobilized at a lower part inside the column was siliconized and 1 ml of the previously prepared gel was packed in the column to make a gel column for separating cells.

To 32 ml of cord blood was added 3 ml of a silica solution (Immune Biology Research Institute Co., Ltd.) and the mixture was incubated at 37°C for 1 hour. The cell suspension was made 48 ml with Hanks' balanced physiological saline (Gibco) and 12 ml aliquot was dispensed in each of four centrifuge tubes to each of which 3 ml of Ficoll-Paque (Pharmacia) had been dispensed and separation was performed under the conditions of 800 rpm for 20 minutes to recover cells of mononuclear cell layer. The recovered cells were washed with the Hanks' solution. A 1 ml cell suspension containing $2.1 \times 10^7$ such cells was charged in the above antibody column and allowed to react at 4°C for 1 hour while gently stirring it. To the column was flown Hanks' balanced physiological saline containing 10% fetal bovine serum, 100 U/ml penicillin, and 100 µg/ml streptomycin to wash the cells. The column was cultured in a carbon dioxide incubator at 37°C for 13 hours after addition of 2 ml of MEM medium (Gibco) containing 10% fetal bovine serum, 100 U/ml penicillin, and 100 µg/ml streptomycin. By moving the column up and down, the gel was stirred slightly strong, and the medium was recovered from the column. Further, through the column was flown 5 ml of Hanks' balanced physiological saline containing 10% fetal bovine serum, 100 U/ml penicillin, and 100 µg/ml streptomycin and this solution was also recovered. The cells in these recovered solutions were recovered by centrifugation. The number of a portion of cells was counted. The remaining cells were allowed to react with anti-HPCA-2 antibody (Becton) on ice for 30 minutes, washed with cold physiological saline containing 2% fetal bovine serum, and recovered by centrifugation. The obtained cells were measured of CD34-positive cell ratio using a flow cytometer (Coulter). CD34-positivity ratio was assumed as purity of CD34 cell and the cell number obtained by multiplying CD34-positivity ratio by number of recovered cell was assumed as number of recovered CD34-positive cells. As a result, a purity of 45.2% and a recovery ratio of 62.6% were obtained. This revealed that the device for separating cells for hematopoietic undifferentiated cells comprising a column device packed with a gel is effective.

[Example 25]

Cord blood (29 ml) was made 48 ml with Hanks' balanced physiological saline (Gibco) and 12 ml aliquot was dispensed in each of four centrifuge tubes to each of which 3 ml of Ficoll-Paque (Pharmacia) had been dispensed and separation was performed under the conditions of 800 rpm for 20 minutes to recover cells of mononuclear cell layer. The recovered cells were washed with the Hanks' solution. To $2.0 \times 10^7$ such cells was added 25μg of the single chain antibody produced in Example 21 to make 5 μl/ml and allowed to react on ice for 1 hour. After washing with the Hanks' solution, the cells were allowed to react with anti-Etag (Pharmacia) antibody for 30 minutes. After the cells were washed, $9 \times 10^6$ Dyna bead (Dynal) anti-mouse IgG beads were added and the mixture was allowed to react on ice for 1 hour. According to the manual attached to Dyna bead, the beads together with the cells adsorbed thereon were separated. The obtained cells were cultivated in 10% fetal bovine serum-added Dulbecco MEM medium in a 5% carbon dioxide atmosphere at 37°C for 12 hours. After the cultivation, the cells and beads were completely separated by pipetting. The beads were removed by a magnet according to the manual attached to the Dyna beads. A portion of the recovered cells was used for the measurement of cell number to obtain number of recovered cells. The remaining cells were allowed to react with anti-HPCA-2 antibody (Becton) on ice for 30 minutes, washed with cold physiological saline containing 2% fetal bovine serum, and recovered by centrifugation. The obtained cells were measured of CD34-positive cell ratio using a flow cytometer (Coulter). CD34-positivity ratio was assumed as purity of CD34 cell and the cell number obtained by multiplying CD34-positivity ratio by number of recovered cell was assumed as number of recovered CD34-positive cells. As a result, a purity of 36.1% and a recovery ratio of 35.3% were obtained. This revealed that the single chain antibody produced in Example 21 separated CD34-positive cells.

[Example 26]

An antibody column to which the single chain antibody produced in Example 21 was bound was prepared utilizing CNBr activated Sepharose4B (Pharmacia). The antibody was bound to Sepharose4B beads by the method according to the manual attached thereto. The single chain antibody liquid was allowed to react with 4.0 ml of the gel to prepare an affinity gel with a coupling efficiency of 99.0%. A glass column ($2 \text{ cm}^2 \times 4$ cm)having a small amount of glass wool immobilized at a lower part inside the column was siliconized and 1 ml of the previously prepared gel was packed in the column to make a gel column device for separating cells.
To 28 ml of cord blood was added 3 ml of a silica solution (Immune Biology Research Institute Co., Ltd.) and the mixture was incubated at 37°C for 1 hour. The cell suspension was made 48 ml with Hanks' balanced physiological saline (Gibco) and 12 ml aliquot was dispensed in each of four centrifuge tubes to each of which 3 ml of Ficoll-Paque (Pharmacia) had been dispensed and separation was performed under the conditions of 800 rpm for 20 minutes to recover cells of mononuclear cell layer. The recovered cells were washed with the Hanks' solution. A 1 ml cell suspension containing $1.8 \times 10^7$ such cells was charged in the above antibody column and allowed to react at 4°C for 1 hour while weekly stirring it. To the column was flown Hanks' balanced physiological saline containing 10% fetal bovine serum, 100 U/ml penicillin, and 100 μg/ml streptomycin to wash the cells. The column was cultured in a carbon dioxide incubator at 37°C for 13 hours after addition of 2 ml of MEM medium (Gibco) containing 10% fetal bovine serum, 100 U/ml penicillin, and 100 μg/ml streptomycin. By moving the column up and down, the gel was stirred slightly strong, and the medium was recovered from the column. Further, through the column was flown 5 ml of Hanks' balanced physiological saline containing 10% fetal bovine serum, 100 U/ml penicillin, and 100 μg/ml streptomycin and this solution was also recovered. The cells in these recovered solutions were recovered by centrifugation. The number of a portion of cells was counted. The remaining cells were allowed to react with anti-HPCA-2 antibody (Becton) on ice for 30 minutes, washed with cold physiological saline containing 2% fetal bovine serum, and recovered by centrifugation. The obtained cells were measured of CD34-positive cell ratio using a flow cytometer (Coulter). CD34-positivity ratio was assumed as purity of CD34 cell and the cell number obtained by multiplying CD34-positivity ratio by number of recovered cell was assumed as number of recovered CD34-positive cells. As a result, a purity of 52.8% and a recovery ratio of 41.0% were obtained. This revealed that the device for separating cells for hematopoietic undifferentiated cells comprising a column device packed with a gel is effective.

[Example 27]

The pCANMY10 vector prepared in Example 21 was improved to prepare an antibody which can increase bindability to the water-insoluble carrier. Subsequent to the E-tag sequence at the carboxyl terminal of the single chain antibody were introduced 3 amino acid lysine residues. Utilizing the restriction enzyme sites of pCANMY10 vector, the sequence containing the lysine residues was introduced by a PCR method. The sequence is shown under Sequence ID No. 4 in the Sequence Listing.

This vector was introduced in Escherichia coli HB2151 strain cells and a single chain antibody was prepared in the same manner as in Example 21.

An antibody column to which the obtained single chain antibody was bound was prepared utilizing CNBr activated Sepharose4B (Pharmacia). The antibody was bound to Sepharose4B beads by the method according to the manual attached thereto. The single chain antibody liquid was allowed to react with 4.0 ml of the gel to prepare an affinity gel. A glass column (2 cm$^2$ × 3 cm) having a small amount of glass wool immobilized at a lower part inside the column was siliconized and 1 ml of the previously prepared gel was packed in the column to make a gel column device for separating cells.

To 32 ml of cord blood was added 3 ml of a silica solution (Immune Biology Research Institute Co., Ltd.) and the mixture was incubated at 37°C for 1 hour. The cell suspension was made 48 ml with Hanks' balanced physiological saline (Gibco) and 12 ml aliquot was dispensed in each of four centrifuge tubes to each of which 3 ml of Ficoll-Paque (Pharmacia) had been dispensed and separation was performed under the conditions of 800 rpm for 20 minutes to recover cells of mononuclear cell layer. The recovered cells were washed with the Hanks' solution. A 1 ml cell suspension containing $2.1 \times 10^7$ such cells was charged in the above antibody column and allowed to react at 4°C for 1 hour while weekly stirring it. To the column was flown Hanks' balanced physiological saline containing 10% fetal bovine serum, 100 U/ml penicillin, and 100 μg/ml streptomycin to wash the cells. The column was cultured in a carbon dioxide incubator at 37°C for 14 hours after addition of 2 ml of MEM medium (Gibco) containing 10% fetal bovine serum, 100 U/ml penicillin, and 100 μg/ml streptomycin. By moving the column up and down, the gel was stirred slightly strong, and the medium was recovered from the column. Further, through the column was flown 5 ml of Hanks' balanced physiological saline containing 10% fetal bovine serum, 100 U/ml penicillin, and 100 μg/ml streptomycin and this solution was also recovered. The cells in these recovered solutions were recovered by centrifugation. The number of a portion of cells was counted. The remaining cells were allowed to react with anti-HPCA-2 antibody (Becton) on ice for 30 minutes, washed with cold physiological saline containing 2% fetal bovine serum, and recovered by centrifugation. The obtained cells were measured of CD34-positive cell ratio using a flow cytometer (Coulter). CD34-positivity ratio was assumed as purity of CD34 cell and the cell number obtained by multiplying CD34-positivity ratio by number of recovered cell was assumed as number of recovered CD34-positive cells. As a result, a purity of 71.0% and a recovery ratio of 52.0% were obtained. This confirmed that the device for separating cells for hematopoietic undifferentiated cells comprising a column device packed with a gel is effective and indicated that the recombinant antibody in which lysine residues were introduced at the carboxyl terminal thereof could separate the cells more efficiently.

INDUSTRIAL APPLICABILITY

According to the present invention, separation of CD4-positive cells and CD34-positive cells can be performed specifically and efficiently. The present invention is effective in the fields of collection of T lymphocytes having auto-reactive antigen receptor, elimination of lymphocytes from cells for use in bone marrow transplantation, and the like with view to treating autoimmune deficiency, etc. The present invention is also effective in the fields of collection of hematopoietic undifferentiated cells, elimination of lymphocytes from cells for use in bone marrow transplantation and the like with view to treating leukemia, etc.

REFERENCE TO DEPOSITED MICROBES

A. Name and address of depository authority to which the microorganisms are deposited

Depository authority:

National Institute of Bioscience and Human-Technology, Agency of industrial Science and Technology, Ministry of International Trade and Industry, Japan

Address:

1-3, Higashi 1-chome, Tsukuba-shi, Ibaraki-ken, Japan

B. Date of deposition to depository authority

May 14, 1998

C. Accession number given by the depository authority

FERM BP-6729

Note that this deposition was transferred to the deposition under Budapest treaty on May 21, 1999 based on the original deposition (Accession No.: FERM P-16807) made on May 14, 1998.

Fig. 1    H CHAIN        L CHAIN        VARIABLE REGION
CONSTANT REGION

Fig. 2    ABOUT 7 kbp

Fig. 3    tag SEQUNCE

Fig. 4    tag SEQUNCE

Fig. 5    CORRELATION BETWEEN ANTIBODY CONCENTRATION AND ABSORBANCE
ABSORBANCE
4H5 ANTIBODY        MT310 ANTIBODY
ANTI-CD4 IgG CONCENTRATION

Fig. 6    CONTROL
STAINING WITH HUMAN-MOUSE CHIMERA ANTIBODY

**Claims**

1. A device for separating cells using an antibody selected from a chimera antibody, a single chain antibody or combinations thereof.

2. A device for separating CD4-positive cells using an antibody selected from a chimera antibody, a single chain antibody which binds to CD4 molecules or combinations thereof.

3. A device for separating CD4-positive cells using a chimera antibody, wherein the antibody comprises an H chain variable region of which CDR-1 is an amino acid sequence represented by Sequence ID No. 1 in the Sequence Listing, CDR-2 is an amino acid sequence represented by Sequence ID No. 2 in the Sequence Listing, and CDR-3 is an amino acid sequence represented by Sequence ID No. 3 in the Sequence Listing, an L chain variable region of which CDR-1 is an amino acid sequence represented by Sequence ID No. 4 in the Sequence Listing, CDR-2 is an amino acid sequence represented by Sequence ID No. 5 in the Sequence Listing, and CDR-3 is an amino acid sequence represented by Sequence ID No. 6 in the Sequence Listing, and an Fc region of a human type.

4. A device for separating CD4-positive cells using a single chain antibody, wherein the antibody comprises an H chain variable region of which CDR-1 is an amino acid sequence represented by Sequence ID No. 1 in the Sequence Listing, CDR-2 is an amino acid sequence represented by Sequence ID No. 2 in the Sequence Listing, and CDR-3 is an amino acid sequence represented by Sequence ID No. 3 in the Sequence Listing, and an L chain variable region of which CDR-1 is an amino acid sequence represented by Sequence ID No. 4 in the Sequence Listing, CDR-2 is an amino acid sequence represented by Sequence ID No. 5 in the Sequence Listing, and CDR-3 is an amino acid sequence represented by Sequence ID No.6 in the Sequence Listing.

5. A device for separating CD34-positive cells using an antibody selected from a chimera antibody, a single chain antibody which binds to CD34 molecules or combinations thereof.

6. A device for separating human CD34-positive cells using antibody, wherein the antibody comprises an H chain variable region of which CDR-1 is an amino acid sequence represented by Sequence ID No. 43 in the Sequence Listing, CDR-2 is an amino acid sequence represented by Sequence ID No. 44 in the Sequence Listing, and CDR-3 is an amino acid sequence represented by Sequence ID No. 45 in the Sequence Listing, an L chain variable region of which CDR-1 is an amino acid sequence represented by Sequence ID No. 46 in the Sequence Listing, CDR-2 is an amino acid sequence represented by Sequence ID No. 47 in the Sequence Listing, and CDR-3 is an amino acid sequence represented by Sequence ID No. 48 in the Sequence Listing, and an Fc region of a human type.

7. A device for separating human CD34-positive cells using a single chain antibody, wherein the antibody comprises an H chain variable region of which CDR-1 is an amino acid sequence represented by Sequence ID No. 43 in the Sequence Listing, CDR-2 is an amino acid sequence represented by Sequence ID No. 44 in the Sequence Listing, and CDR-3 is an amino acid sequence represented by Sequence ID No. 45 in the Sequence Listing, and an L chain variable region of which CDR-1 is an amino acid sequence represented by Sequence ID No. 46 in the Sequence Listing, CDR-2 is an amino acid sequence represented by Sequence ID No. 47 in the Sequence Listing, and CDR-3 is an amino acid sequence represented by Sequence ID No. 48 in the Sequence Listing.

8. The device for separating cells according to any one of claims 1 to 7, wherein the antibody comprises an amino acid sequence containing a basic amino acid or acidic amino acid added to the C-terminal or N-terminal or both of the amino acid sequence of the antibody.

9. The device for separating cells according to any one of claims 1 to 8, wherein the antibody selected from a chimera antibody, a single chain antibody or combinations thereof is bound to an active group of a polypropylene nonwoven fabric reacted with a haloacetaminomethylating agent.

10. A method for separating or detecting cells, comprising using an antibody selected from a chimera antibody, a single chain antibody or combinations thereof.

11. A method for separating or detecting human CD4-positive cells, comprising using an antibody selected from a chimera antibody, a single chain antibody which bind to CD4 molecules or combinations thereof.

12. A method for separating or detecting human CD4-positive cells, comprising using a chimera antibody, wherein the antibody comprises an H chain variable region of which CDR-1 is an amino acid sequence represented by Sequence ID No.1 in the Sequence Listing, CDR-2 is an amino acid sequence represented by Sequence ID No. 2 in the Sequence Listing, and CDR-3 is an amino acid sequence represented by Sequence ID No. 3 in the Sequence Listing, an L chain variable region of which CDR-1 is an amino acid sequence represented by Sequence ID No. 4 in the Sequence Listing, CDR-2 is an amino acid sequence represented by Sequence ID No. 5 in the Sequence Listing, and CDR-3 is an amino acid sequence represented by Sequence ID No. 6 in the Sequence Listing, and an Fc region of a human type.

13. A method for separating or detecting human CD4-positive cells, comprising using a single chain antibody, wherein the antibody comprises an H chain variable region of which CDR-1 is an amino acid sequence represented by Sequence ID No. 1 in the Sequence Listing, CDR-2 is an amino acid sequence represented by Sequence ID No. 2 in the Sequence Listing, and CDR-3 is an amino acid sequence represented by Sequence ID No. 3 in the Sequence Listing, and an L chain variable region of which CDR-1 is an amino acid sequence represented by Sequence ID No. 4 in the Sequence Listing, CDR-2 is an amino acid sequence represented by Sequence ID No. 5 in the Sequence Listing, and CDR-3 is an amino acid sequence represented by Sequence ID No. 6 in the Sequence Listing.

14. A method for separating or detecting human CD34-positive cells, comprising using an antibody selected from a chimera antibody, a single chain antibody which bind to CD34 molecules or combinations thereof.

15. A method for separating or detecting human CD34-positive cells, comprising using antibody, wherein the antibody comprises an H chain variable region of which CDR-1 is an amino acid sequence represented by Sequence ID No. 43 in the Sequence Listing, CDR-2 is an amino acid sequence represented by Sequence ID No. 44 in the Sequence Listing, and CDR-3 is an amino acid sequence represented by Sequence ID No. 45 in the Sequence Listing, an L chain variable region of which CDR-1 is an amino acid sequence represented by Sequence ID No. 46 in the Sequence Listing, CDR-2 is an amino acid sequence represented by Sequence ID No. 47 in the Sequence Listing, and CDR-3 is an amino acid sequence represented by Sequence ID No. 48 in the Sequence Listing, and an Fc region of a human type.

16. A method for separating or detecting human CD34-positive cells, comprising using a chain strand antibody, wherein the antibody comprises an H chain variable region of which CDR-1 is an amino acid sequence represented by Sequence ID No. 43 in the Sequence Listing, CDR-2 is an amino acid sequence represented by Sequence ID No. 44 in the Sequence Listing, and CDR-3 is an amino acid sequence represented by Sequence ID No. 45 in the Sequence Listing, and an L chain variable region of which CDR-1 is an amino acid sequence represented by Sequence ID No. 46 in the Sequence Listing, CDR-2 is an amino acid sequence represented by Sequence ID No. 47 in the Sequence Listing, and CDR-3 is an amino acid sequence represented by Sequence ID No. 48 in the Sequence Listing.

17. The method for separating or detecting cells according to any one of claims 10 to 16, wherein the antibody comprises an amino acid sequence containing a basic amino acid or acidic amino acid added to the C-terminal or N-terminal or both of the amino acid sequence of the antibody.

18. An antibody comprising an H chain variable region of which CDR-1, CDR-2, and CDR-3 are amino acid sequences described in Sequence ID Nos. 1, 2, and 3, respectively, in the Sequence Listing and having affinity for CD4 antigen.

19. An antibody comprising an L chain variable region of which CDR-1, CDR-2, and CDR-3 are amino acid sequences described in Sequence ID Nos. 4, 5, and 6, respectively, in the Sequence Listing and having affinity for CD4 antigen.

20. A monoclonal antibody to CD4 antigen, produced by hybridoma 4H5 having a depository accession number FERM BP-6729.

21. A nucleic acid encoding the antibody according to any one of claims 18 to 20.

22. The nucleic acid according to claim 21, containing the nucleotide sequences described in Sequence ID Nos. 7 and 8.

**23.** A method for producing antibodies using the nucleic acid according to claim 21 or 22.

**24.** A recombinant antibody which can be obtained by the method according to claim 23 and which has affinity for CD4 antigen.

**25.** The recombinant antibody according to claim 24, wherein the antibody has an Fc region of a human type.

**26.** The recombinant antibody according to claim 24, wherein the antibody is a single chain antibody.

**27.** A medicinal composition comprising the antibody according to any one of claims 18 to 20 and a pharmaceutically acceptable carrier.

**28.** A medicinal composition comprising the recombinant antibody according to any one of claims 24 to 26 and a pharmaceutically acceptable carrier.

FIG. 1

FIG. 2

FIG. 3

RBS (SD)

Trc promotor

△Nco I, △BspHI

Naa I

Not I
Hind III

lacI

PelB signal

tag SEQUENCE

rrnB term.

pSE380ScFv

4.3 kbp

ColEI ori.

Amp. r

FIG. 4

FIG. 5

CORRELATION BETWEEN ANTIBODY
CONCENTRATION AND ABSORBANCE

FIG. 6

(1) CONTROL

MY10
1 μg

(2) STAINING WITH HUMAN-MOUSE CHIMERA ANTIBODY

MY10#2
1 μg

# INTERNATIONAL SEARCH REPORT

International application No.

PCT/JP99/02711

| A. CLASSIFICATION OF SUBJECT MATTER |
|---|
| Int.Cl⁶ C12N15/62, C12N15/13, C07K16/28, C07K16/46, C12P21/08, C12N5/12, C12M1/00, G01N33/53, A61K39/395 |
| According to International Patent Classification (IPC) or to both national classification and IPC |

| B. FIELDS SEARCHED |
|---|
| Minimum documentation searched (classification system followed by classification symbols) |
| Int.Cl⁶ C12N15/62, C12N15/13, C07K16/28, C07K16/46, C12P21/08, C12N5/12, C12M1/00, G01N33/53, A61K39/395 |

| Documentation searched other than minimum documentation to the extent that such documents are included in the fields searched |
|---|
|  |

| Electronic data base consulted during the international search (name of data base and, where practicable, search terms used) |
|---|
| SwissProt/PIR/GeneSeq, CA (STN), REGISTRY (STN), WPI (DIALOG), BIOSIS (DIALOG) |

## C. DOCUMENTS CONSIDERED TO BE RELEVANT

| Category* | Citation of document, with indication, where appropriate, of the relevant passages | Relevant to claim No. |
|---|---|---|
| P, X | JP, 10-155489, A (Asahi Chemical Industry Co., Ltd.), 16 June, 1998 (16. 06. 98) (Family: none) | 1, 5-10, 14-17 |
| Y | JP, 10-70986, A (BML, Inc.), 17 March, 1998 (17. 03. 98) (Family: none) | 1-4, 8-13, 17-28 |
| Y | WEISSENHORN, W et al., "Combinatorial functions of two chimeric antibodies directed to human CD4 and one directed to the α-chain of the human interleukin-2 receptor", Gene (1992) Vol. 121, No. 2 p.271-278 | 1-28 |
| Y | DONAHUE, R.E et al., "Helper virus induced T cell lymphoma in nonhuman primates after retroviral mediated gene transfer", J. of Experimental Medicine (1992) Vol. 176, No. 4 p.1125-1135 | 5-7, 14-16 |
| Y | JP, 2-238883, A (Becton,Dickinson and Co.), 21 September, 1990 (21. 09. 90) & EP, 365209, A | 1-4, 8-13, 17-28 |

| ☒ Further documents are listed in the continuation of Box C. | ☐ See patent family annex. |
|---|---|

| * | Special categories of cited documents: | "T" | later document published after the international filing date or priority |
|---|---|---|---|
| "A" | document defining the general state of the art which is not considered to be of particular relevance | | date and not in conflict with the application but cited to understand the principle or theory underlying the invention |
| "E" | earlier document but published on or after the international filing date | "X" | document of particular relevance; the claimed invention cannot be |
| "L" | document which may throw doubts on priority claim(s) or which is cited to establish the publication date of another citation or other special reason (as specified) | | considered novel or cannot be considered to involve an inventive step when the document is taken alone |
| "O" | document referring to an oral disclosure, use, exhibition or other means | "Y" | document of particular relevance; the claimed invention cannot be considered to involve an inventive step when the document is combined with one or more other such documents, such combination |
| "P" | document published prior to the international filing date but later than the priority date claimed | | being obvious to a person skilled in the art |
| | | "&" | document member of the same patent family |

| Date of the actual completion of the international search | Date of mailing of the international search report |
|---|---|
| 9 August, 1999 (09. 08. 99) | 31 August, 1999 (31. 08. 99) |

| Name and mailing address of the ISA/ | Authorized officer |
|---|---|
| Japanese Patent Office | |
| Facsimile No. | Telephone No. |

Form PCT/ISA/210 (second sheet) (July 1992)

EP 1 083 226 A1

# INTERNATIONAL SEARCH REPORT

| International application No. |
|---|
| PCT/JP99/02711 |

C (Continuation). DOCUMENTS CONSIDERED TO BE RELEVANT

| Category* | Citation of document, with indication, where appropriate, of the relevant passages | Relevant to claim No. |
|---|---|---|
| A | JP, 6-125783, A (Juridical Foundation The Chemo-Sero-Therapeutic Research Institute), 10 May, 1994 (10. 05. 94) (Family: none) | 1-4, 8-13, 17-28 |

Form PCT/ISA/210 (continuation of second sheet) (July 1992)

74

# SEQUENCE LISTING

<110>  ASAHIKASEI KOGYO KABUSHIKI KAISHA

ASAHI MEDICAL CO., LTD.

<120>  Separating apparatus of cells and separating method

<130>  ASAHI-1

<150>  JP 10/159957

JP 10/163023

<151>  1998-5-25

1998-5-26

<160>  48

<210>  1

<211>  5

<212>  PRT

<213>  mouse

<400>  1

Asp Tyr Val Ile Asn

<210> 2

<211> 17

<212> PRT

<213> mouse


<400> 2

Glu Ile Tyr Pro Gly Ser Gly Ser Ala Tyr Tyr Asn Glu Met Phe Lys
1               5                       10                      15

Gly


<210> 3

<211> 9

<212> PRT

<213> mouse


<400> 3

Arg Gly Thr Gly Thr Gly Phe Ala Tyr
1                       5


<210> 4

<211> 15

<212> PRT

<213> mouse


<400> 4

Lys Ala Ser Gln Ser Val Asp Tyr Asp Gly Asp Ser Tyr Met Asn

1          5          10          15

<210> 5

<211> 7

<212> PRT

<213> mouse


<400> 5

Ala Ala Ser Asn Leu Glu Ser

1          5


<210> 6

<211> 9

<212> PRT

<213> mouse


<400> 6

Gln Gln Ser Ser Glu Asp Pro Pro Thr

1          5


<210> 7

<211> 330

<212> DNA

<213> mouse


<400> 7

```
CCT GAG CTG GTG AAG CCT GGG GCT TCA GTC AAG ATG TCC TGC AAG GCT      48
Pro Glu Leu Val Lys Pro Gly Ala Ser Val Lys Met Ser Cys Lys Ala
  1               5                  10                  15

TCT GGA TAC ACA TTC ACT GAC TAT GTT ATA AAC TGG TTG AAC CAG AGA      96
Ser Gly Tyr Thr Phe Thr Asp Tyr Val Ile Asn Trp Leu Asn Gln Arg
                 20                  25                  30

ACT GGA CAG GGC CTT GAG TGG ATT GGA GAG ATT TAT CCT GGA AGT GGT     144
Thr Gly Gln Gly Leu Glu Trp Ile Gly Glu Ile Tyr Pro Gly Ser Gly
                 35                  40                  45

AGT GCT TAC TAC AAT GAG ATG TTC AAG GGC AAG GCC ACA CTG ACT GCA     192
Ser Ala Tyr Tyr Asn Glu Met Phe Lys Gly Lys Ala Thr Leu Thr Ala
             50                  55                  60

GAC AAA TCC TCC AAC ACA GCC TAC ATG CAG CTC AGC AGC CTG ACA TCT     240
Asp Lys Ser Ser Asn Thr Ala Tyr Met Gln Leu Ser Ser Leu Thr Ser
 65                  70                  75                  80

GAG GAC TCT GCG GTC TAT TTC TGT GCA AGA CGC GGA ACT GGG ACG GGG     288
Glu Asp Ser Ala Val Tyr Phe Cys Ala Arg Arg Gly Thr Gly Thr Gly
                 85                  90                  95

TTT GCT TAC TGG GGC CGA GGG ACT CTG GTC ACT GTC TCT GCA            330
Phe Ala Tyr Trp Gly Arg Gly Thr Leu Val Thr Val Ser Ala
                 100                 105                 110
```

<210> 8

<211> 309

<212> DNA

<213> mouse

34

<400> 8

GCT TCT TTG GCT GTG TCT CTA GGG CAG AGG GCC ACC ATC TCC TGC AAG   48
Ala Ser Leu Ala Val Ser Leu Gly Gln Arg Ala Thr Ile Ser Cys Lys
1             5             10             15

GCC AGC CAA AGT GTT GAT TAT GAT GGT GAT AGT TAT ATG AAC TGG TAC   96
Ala Ser Gln Ser Val Asp Tyr Asp Gly Asp Ser Tyr Met Asn Trp Tyr
           20             25             30

CAA CAG AAA CCA GGA CAG CCA CCC AAA CTC CTC ATC TAT GCT GCA TCC   144
Gln Gln Lys Pro Gly Gln Pro Pro Lys Leu Leu Ile Tyr Ala Ala Ser
           35             40             45

AAT CTA GAA TCT GGG ATC CCA GCC AGG TTT AGT GGC AGT GGG TCT GGG   192
Asn Leu Glu Ser Gly Ile Pro Ala Arg Phe Ser Gly Ser Gly Ser Gly
           50         ·55             60

ACA GAC TTC ACC CTC AAC ATC CAT CCT GTG GAG GAG GAG GAT GCT GCA   240
Thr Asp Phe Thr Leu Asn Ile His Pro Val Glu Glu Glu Asp Ala Ala
65             70             75             80

ACC TAT TAC TGT CAG CAA AGT AGT GAG GAT CCT CCG ACG TTC GGT GGA   288
Thr Tyr Tyr Cys Gln Gln Ser Ser Glu Asp Pro Pro Thr Phe Gly Gly
           85             90             95

GGC ACC AAG CTG GAA ATC AAA   309
Gly Thr Lys Leu Glu Ile Lys
           100

<210> 9
<211> 925

<212>   DNA

<213>   mouse

<400>   9

ATG AAA TAC CTG CTG CCG ACC GCT GCT GCT GGT CTG CTG CTC CTC GCG    48
Met Lys Tyr Leu Leu Pro Thr Ala Ala Ala Gly Leu Leu Leu Leu Ala
 1               5                   10                  15

GCC CAG CCG GCC ATG GCC GAC ATT GTG CTG ACC CAA TCT CCA GCT TCT    96
Ala Gln Pro Ala Met Ala Asp Ile Val Leu Thr Gln Ser Pro Ala Ser
                20                  25                  30

TTG GCT GTG TCT CTA GGG CAG AGG GCC ACC ATC TCC TGC AAG GCC AGC   144
Leu Ala Val Ser Leu Gly Gln Arg Ala Thr Ile Ser Cys Lys Ala Ser
                35                  40                  45

CAA AGT GTT GAT TAT GAT GGT GAT AGT TAT ATG AAC TGG TAC CAA CAG   192
Gln Ser Val Asp Tyr Asp Gly Asp Ser Tyr Met Asn Trp Tyr Gln Gln
            50                  55                  60

AAA CCA GGA CAG CCA CCC AAA CTC CTC ATC TAT GCT GCA TCC AAT CTA   240
Lys Pro Gly Gln Pro Pro Lys Leu Leu Ile Tyr Ala Ala Ser Asn Leu
65                  70                  75                  80

GAA TCT GGG ATC CCA GCC AGG TTT AGT GGC AGT GGG TCT GGG ACA GAC   288
Glu Ser Gly Ile Pro Ala Arg Phe Ser Gly Ser Gly Ser Gly Thr Asp
                85                  90                  95

TTC ACC CTC AAC ATC CAT CCT GTG GAG GAG GAG GAT GCT GCA ACC TAT   336
Phe Thr Leu Asn Ile His Pro Val Glu Glu Glu Asp Ala Ala Thr Tyr
                100                 105                 110

TAC TGT CAG CAA AGT AGT GAG GAT CCT CCG ACG TTC GGT GGA GGC ACC   384

```
Tyr Cys Gln Gln Ser Ser Glu Asp Pro Pro Thr Phe Gly Gly Gly Thr
        115             120             125

AAG CTG GAA ATC AAA GGT GGA GGC GGT TCA GGC GGA GGT GGC TCC GGA   432
Lys Leu Glu Ile Lys Gly Gly Gly Gly Ser Gly Gly Gly Gly Ser Gly
        130             135             140

GGT GGC GGA TCG CAG GTT CAG CTG CAG CAG TCT GGA CCT GAG CTG GTG   480
Gly Gly Gly Ser Gln Val Gln Leu Gln Gln Ser Gly Pro Glu Leu Val
145             150             155             160

AAG CCT GGG GCT TCA GTG AAG ATG TCC TGC AAG GCT TCT GGA TAC ACA   528
Lys Pro Gly Ala Ser Val Lys Met Ser Cys Lys Ala Ser Gly Tyr Thr
        165             170             175

TTC ACT GAC TAT GTT ATA AAC TGG TTG AAC CAG AGA ACT GGA CAG GGC   576
Phe Thr Asp Tyr Val Ile Asn Trp Leu Asn Gln Arg Thr Gly Gln Gly
        180             185             190

CTT GAG TGG ATT GGA GAG ATT TAT CCT GGA AGT GGT AGT GCT TAC TAC   624
Leu Glu Trp Ile Gly Glu Ile Tyr Pro Gly Ser Gly Ser Ala Tyr Tyr
        195             200             205

AAT GAG ATG TTC AAG GGC AAG GCC ACA CTG ACT GCA GAC AAA TCC TCC   672
Asn Glu Met Phe Lys Gly Lys Ala Thr Leu Thr Ala Asp Lys Ser Ser
        210             215             220

AAC ACA GCC TAC ATG CAG CTC AGC AGC CTG ACA TCT GAG GAC TCT GCG   720
Asn Thr Ala Tyr Met Gln Leu Ser Ser Leu Thr Ser Glu Asp Ser Ala
225             230             235             240

GTC TAT TTC TGT GCA AGA CGC GGA ACT GGG ACG GGG TTT GCT TAC TGG   768
Val Tyr Phe Cys Ala Arg Arg Gly Thr Gly Thr Gly Phe Ala Tyr Trp
        245             250             255
```

```
GGC CGA GGG ACT CTG GTC ACT GTC TCT GCA GCG GCC GCA GAC TAC AAG        816
Gly Arg Gly Thr Leu Val Thr Val Ser Ala Ala Ala Ala Asp Tyr Lys
            260                 265                 270
GAT GAC GAT GAC AAA GGC TCG AGC GAG CAG AAG CTG ATC AGC GAA GAG        864
Asp Asp Asp Asp Lys Gly Ser Ser Glu Gln Lys Leu Ile Ser Glu Glu
            275                 280                 285
GAT CTG GGC TCG AGG TCG ACC CAC CAT CAT CAT CAC CAC GGG TCG ACC        912
Asp Leu Gly Ser Arg Ser Thr His His His His His His Gly Ser Thr
            290                 295                 300
AAA TGA TAA GCT T                                                      925
Lys
305


<210>    10
<211>    925
<212>    DNA
<213>    mouse


<400>    10
ATG AAA TAC CTG CTG CCG ACC GCT GCT GCT GGT CTG CTG CTC CTC GCG        48
Met Lys Tyr Leu Leu Pro Thr Ala Ala Ala Gly Leu Leu Leu Leu Ala
1                 5                   10                  15
GCC CAG CCG GCC ATG GCC CAG GTT CAG CTG CAG CAG TCT GGA CCT GAG        96
Ala Gln Pro Ala Met Ala Gln Val Gln Leu Gln Gln Ser Gly Pro Glu
                20                  25                  30
CTG GTG AAG CCT GGG GCT TCA GTG AAG ATG TCC TGC AAG GCT TCT GGA        144
```

Leu Val Lys Pro Gly Ala Ser Val Lys Met Ser Cys Lys Ala Ser Gly
         35            40           45

TAC ACA TTC ACT GAC TAT GTT ATA AAC TGG TTG AAC CAG AGA ACT GGA   192
Tyr Thr Phe Thr Asp Tyr Val Ile Asn Trp Leu Asn Gln Arg Thr Gly
         50            55          60

CAG GGC CTT GAG TGG ATT GGA GAG ATT TAT CCT GGA AGT GGT AGT GCT   240
Gln Gly Leu Glu Trp Ile Gly Glu Ile Tyr Pro Gly Ser Gly Ser Ala
         65            70          75          80

TAC TAC AAT GAG ATG TTC AAG GGC AAG GCC ACA CTG ACT GCA GAC AAA   288
Tyr Tyr Asn Glu Met Phe Lys Gly Lys Ala Thr Leu Thr Ala Asp Lys
            85           90          95

TCC TCC AAC ACA GCC TAC ATG CAG CTC AGC AGC CTG ACA TCT GAG GAC   336
Ser Ser Asn Thr Ala Tyr Met Gln Leu Ser Ser Leu Thr Ser Glu Asp
           100        105       110

TCT GCG GTC TAT TTC TGT GCA AGA CGC GGA ACT GGG ACG GGG TTT GCT   384
Ser Ala Val Tyr Phe Cys Ala Arg Arg Gly Thr Gly Thr Gly Phe Ala
           115        120       125

TAC TGG GGC CGA GGG ACT CTG GTC ACT GTC TCT GCA GGT GGA GGC GGT   432
Tyr Trp Gly Arg Gly Thr Leu Val Thr Val Ser Ala Gly Gly Gly Gly
          130        135       140

TCA GGC GGA GGT GGC TCC GGA GGT GGC GGA TCG GAC ATT GTG CTG ACC   480
Ser Gly Gly Gly Gly Ser Gly Gly Gly Ser Asp Ile Val Leu Thr
145          150        155       160

CAA TCT CCA GCT TCT TTG GCT GTG TCT CTA GGG CAG AGG GCC ACC ATC   528
Gln Ser Pro Ala Ser Leu Ala Val Ser Leu Gly Gln Arg Ala Thr Ile
          165        170       175

```
TCC TGC AAG GCC AGC CAA AGT GTT GAT TAT GAT GGT GAT AGT TAT ATG    576
Ser Cys Lys Ala Ser Gln Ser Val Asp Tyr Asp Gly Asp Ser Tyr Met
            180             185             190

AAC TGG TAC CAA CAG AAA CCA GGA CAG CCA CCC AAA CTC CTC ATC TAT    624
Asn Trp Tyr Gln Gln Lys Pro Gly Gln Pro Pro Lys Leu Leu Ile Tyr
        195             200             205

GCT GCA TCC AAT CTA GAA TCT GGG ATC CCA GCC AGG TTT AGT GGC AGT    672
Ala Ala Ser Asn Leu Glu Ser Gly Ile Pro Ala Arg Phe Ser Gly Ser
        210             215             220

GGG TCT GGG ACA GAC TTC ACC CTC AAC ATC CAT CCT GTG GAG GAG GAG    720
Gly Ser Gly Thr Asp Phe Thr Leu Asn Ile His Pro Val Glu Glu Glu
225             230             235             240

GAT GCT GCA ACC TAT TAC TGT CAG CAA AGT AGT GAG GAT CCT CCG ACG    768
Asp Ala Ala Thr Tyr Tyr Cys Gln Gln Ser Ser Glu Asp Pro Pro Thr
            245             250             255

TTC GGT GGA GGC ACC AAG CTG GAA ATC AAA GCG GCC GCA GAC TAC AAG    816
Phe Gly Gly Gly Thr Lys Leu Glu Ile Lys Ala Ala Ala Asp Tyr Lys
            260             265             270

GAT GAC GAT GAC AAA GGC TCG AGC GAG CAG AAG CTG ATC AGC GAA GAG    864
Asp Asp Asp Asp Lys Gly Ser Ser Glu Gln Lys Leu Ile Ser Glu Glu
            275             280             285

GAT CTG GGC TCG AGG TCG ACC CAC CAT CAT CAT CAC CAC GGG TCG ACC    912
Asp Leu Gly Ser Arg Ser Thr His His His His His His Gly Ser Thr
        290             295             300

AAA TGA TAA GCT T                                                  925
Lys
```

305

<210> 11

<211> 28

<212> DNA

<213> Artificial Sequence

<220>

<223> Single strand DNA primer for PCR


<400> 11

AAGCTTATGA ACCGGGGAGT CCCTTTTA                                                28


<210> 12

<211> 56

<212> DNA

<213> Artificial Sequence

<220>

<223> Single strand DNA primer for PCR


<400> 12

GCGGCCGCTC ACTTGTCATC GTCGTCCTTG TAGTCTGGCT GCACCGGGGT GGACCA        56


<210> 13

<211> 34

<212> DNA

<213> Artificial Sequence

<220>

<223>    Single strand DNA primer for PCR


<400>    13

GGGAATTCAT GRAATGSASC TGGGTYWTYC TCTT                                    34


<210>    14

<211>    35

<212>    DNA

<213>    Artificial Sequence

<220>

<223>    Single strand DNA primer for PCR


<400>    14

CCCAAGCTTC CAGGGRCCAR KGGATARACN GRTGG                                   35


<210>    15

<211>    29

<212>    DNA

<213>    Artificial Sequence

<220>

<223>    Single strand DNA primer for PCR


<400>    15

TGTGCCCTCG AGCTNACNCA RAGYCCNGC                                          29

<210> 16

<211> 28

<212> DNA

<213> Artificial Sequence

<220>

<223> Single strand DNA primer for PCR


<400> 16

ATGGATACTA GTGGTGCAGC ATCAGCCC                                    28


<210> 17

<211> 33

<212> DNA

<213> Artificial Sequence

<220>

<223> Single strand DNA primer for PCR


<400> 17

GGGAATTCAT GGAGACAGAC ACACTCCTGC TAT                              33


<210> 18

<211> 21

<212> DNA

<213> Artificial Sequence

<220>

<223> Single strand DNA primer for PCR

<400> 18

CGTCGGAGGA TCCTCACTAC T                                           21


<210> 19

<211> 27

<212> DNA

<213> Artificial Sequence

<220>

<223> Single strand DNA primer for PCR


<400> 19

CAGGATCCGC TGCAGCAGTC TGGACCT                                     27


<210> 20

<211> 27

<212> DNA

<213> Artificial Sequence

<220>

<223> Single strand DNA primer for PCR


<400> 20

TGGGCCCGTC GTTTTGGCTG CAGAGAC                                     27


<210> 21

<211> 56

<210> DNA

<213> Artificial Sequence

<220>

<223> Single strand DNA primer for PCR

<400> 21

TCATGAAATA CCTGCTGCCG ACCGCTGCTG CTGGTCTGCT GCTCCTCGCG GCCCAG    56

<210> 22

<211> 56

<212> DNA

<213> Artificial Sequence

<220>

<223> Single strand DNA primer for PCR

<400> 22

TGCGGCCGCA GCCATGGTGT TTCCGGCCAT CGCCGGCTGG GCCGCGAGGA GCAGCA    56

<210> 23

<211> 56

<212> DNA

<213> Artificial Sequence

<220>

<223> Single strand DNA primer for PCR

<400> 23

TGCGGCCGCA GACTACAAGG ATGACGATGA CAAAGGCTCG AGCGAGCAGA AGCTGA          56


<210>    24

<211>    57

<212>    DNA

<213>    Artificial Sequence

<220>

<223>    Single strand DNA primer for PCR


<400>    24

GGTGGGTCGA CCTCGAGCCC AGATCCTCTT CGCTGATCAG CTTCTGCTCG CTCGAGC          57


<210>    25

<211>    23

<212>    DNA

<213>    Artificial Sequence

<220>

<223>    Single strand DNA primer for PCR


<400>    25

TGCGGCCGCA GACTACAAGG ATG          23


<210>    26

<211>    56

<212>    DNA

<213>    Artificial Sequence

<220>

<223>   Single strand DNA primer for PCR


<400>   26

TAAGCTTATC ATTTGGTCGA CCCGTGGTGA TGATGATGGT GGGTCGACCT CGAGCC          56


<210>   27

<211>   38

<212>   DNA

<213>   Artificial Sequence

<220>

<223>   Single strand DNA primer for PCR


<400>   27

AGCCGGCCAT GGCCGACATT GTGCTGACCC AATCTCCA                              38


<210>   28

<211>   58

<212>   DNA

<213>   Artificial Sequence

<220>

<223>   Single strand DNA primer for PCR


<400>   28

CTCCGGAGCC ACCTCCGCCT GAACCGCCTC CACCTTTGAT TTCCAGCTTG GTGCCTCC       58

<210> 29

<211> 40

<212> DNA

<213> Artificial Sequence

<220>

<223> Single strand DNA primer for PCR


<400> 29

CTCCGGAGGT GGCGGATCGC AGGTTCAGCT GCAGCAGTCT                    40


<210> 30

<211> 31

<212> DNA

<213> Artificial Sequence

<220>

<223> Single strand DNA primer for PCR


<400> 30

TGCGGCCGCT GCAGAGACAG TGACCAGAGT C                             31


<210> 31

<211> 35

<212> DNA

<213> Artificial Sequence

<220>

<223> Single strand DNA primer for PCR

<400>    31

AGCCGGCCAT GGCCCAGGTT CAGCTGCAGC AGTCT                                    35


<210>    32

<211>    56

<212>    DNA

<213>    Artificial Sequence

<220>

<223>    Single strand DNA primer for PCR


<400>    32

CTCCGGAGCC ACCTCCGCCT GAACCGCCTC CACCTGCAGA GACAGTGACC AGAGTC          56


<210>    33

<211>    43

<212>    DNA

<213>    Artificial Sequence

<220>

<223>    Single strand DNA primer for PCR


<400>    33

CTCCGGAGGT GGCGGATCGG ACATTGTGCT GACCCAATCT CCA                         43


<210>    34

<211>    33

<212>     DNA

<213>     Artificial Sequence

<220>

<223>     Single strand DNA primer for PCR


<400>     34

TGCGGCCGCT TTGATTTCCA GCTTGGTGCC TCC                                    33


<210>     35

<211>     118

<212>     PRT

<213>     mouse


<400>     35

Gln Val Gln Leu Gln Gln Ser Gly Pro Glu Leu Val Lys Pro Gly Ala
1                   5                   10                  15

Ser Val Lys Met Ser Cys Lys Ala Ser Gly Tyr Thr Phe Thr Asp Tyr
                20                  25                  30

Val Ile Asn Trp Leu Asn Gln Arg Thr Gly Gln Gly Leu Glu Trp Ile
                35                  40                  45

Gly Glu Ile Tyr Pro Gly Ser Gly Ser Ala Tyr Tyr Asn Glu Met Phe
            50                  55                  60

Lys Gly Lys Ala Thr Leu Thr Ala Asp Lys Ser Ser Asn Thr Ala Tyr
65                  70                  75                  80

Met Gln Leu Ser Ser Leu Thr Ser Glu Asp Ser Ala Val Tyr Phe Cys
                85                  90                  95

```
Ala Arg Arg Gly Thr Gly Thr Gly Phe Ala Tyr Trp Gly Arg Gly Thr
                100                 105                 110
Leu Val Thr Val Ser Ala
                115


<210>    36

<211>    111

<212>    PRT

<213>    mouse


<400>    36
Asp Ile Val Leu Thr Gln Ser Pro Ala Ser Leu Ala Val Ser Leu Gly
  1                   5                  10                  15
Gln Arg Ala Thr Ile Ser Cys Lys Ala Ser Gln Ser Val Asp Tyr Asp
                 20                  25                  30
Gly Asp Ser Tyr Met Asn Trp Tyr Gln Gln Lys Pro Gly Gln Pro Pro
                 35                  40                  45
Lys Leu Leu Ile Tyr Ala Ala Ser Asn Leu Glu Ser Gly Ile Pro Ala
                 50                  55                  60
Arg Phe Ser Gly Ser Gly Ser Gly Thr Asp Phe Thr Leu Asn Ile His
 65                   70                  75                  80
Pro Val Glu Glu Glu Asp Ala Ala Thr Tyr Tyr Cys Gln Gln Ser Ser
                 85                  90                  95
Glu Asp Pro Pro Thr Phe Gly Gly Gly Thr Lys Leu Glu Ile Lys
                100                 105                 110
```

<210> 37

<211> 354

<212> DNA

<213> mouse


<400> 37

```
CAG GTT CAG CTG CAG CAG TCT GGA CCT GAG CTG GTG AAG CCT GGG GCT      48
Gln Val Gln Leu Gln Gln Ser Gly Pro Glu Leu Val Lys Pro Gly Ala
 1               5                  10                  15
TCA GTG AAG ATG TCC TGC AAG GCT TCT GGA TAC ACA TTC ACT GAC TAT      96
Ser Val Lys Met Ser Cys Lys Ala Ser Gly Tyr Thr Phe Thr Asp Tyr
                20                  25                  30
GTT ATA AAC TGG TTG AAC CAG AGA ACT GGA CAG GGC CTT GAG TGG ATT     144
Val Ile Asn Trp Leu Asn Gln Arg Thr Gly Gln Gly Leu Glu Trp Ile
             35                  40                  45
GGA GAG ATT TAT CCT GGA AGT GGT AGT GCT TAC TAC AAT GAG ATG TTC     192
Gly Glu Ile Tyr Pro Gly Ser Gly Ser Ala Tyr Tyr Asn Glu Met Phe
         50                  55                  60
AAG GGC AAG GCC ACA CTG ACT GCA GAC AAA TCC TCC AAC ACA GCC TAC     240
Lys Gly Lys Ala Thr Leu Thr Ala Asp Lys Ser Ser Asn Thr Ala Tyr
65                  70                  75                  80
ATG CAG CTC AGC AGC CTG ACA TCT GAG GAC TCT GCG GTC TAT TTC TGT     288
Met Gln Leu Ser Ser Leu Thr Ser Glu Asp Ser Ala Val Tyr Phe Cys
                85                  90                  95
GCA AGA CGC GGA ACT GGG ACG GGG TTT GCT TAC TGG GGC CGA GGG ACT     336
Ala Arg Arg Gly Thr Gly Thr Gly Phe Ala Tyr Trp Gly Arg Gly Thr
```

```
              100                105                110
CTG GTC ACT GTC TCT GCA                                                 354
Leu Val Thr Val Ser Ala
              115
```

<210> 38

<211> 333

<212> DNA

<213> mouse


<400> 38

```
GAC ATT GTG CTG ACC CAA TCT CCA GCT TCT TTG GCT GTG TCT CTA GGG     48
Asp Ile Val Leu Thr Gln Ser Pro Ala Ser Leu Ala Val Ser Leu Gly
 1               5                  10                 15
CAG AGG GCC ACC ATC TCC TGC AAG GCC AGC CAA AGT GTT GAT TAT GAT     96
Gln Arg Ala Thr Ile Ser Cys Lys Ala Ser Gln Ser Val Asp Tyr Asp
             20                 25                 30
GGT GAT AGT TAT ATG AAC TGG TAC CAA CAG AAA CCA GGA CAG CCA CCC    144
Gly Asp Ser Tyr Met Asn Trp Tyr Gln Gln Lys Pro Gly Gln Pro Pro
             35                 40                 45
AAA CTC CTC ATC TAT GCT GCA TCC AAT CTA GAA TCT GGG ATC CCA GCC    192
Lys Leu Leu Ile Tyr Ala Ala Ser Asn Leu Glu Ser Gly Ile Pro Ala
         50                 55                 60
AGG TTT AGT GGC AGT GGG TCT GGG ACA GAC TTC ACC CTC AAC ATC CAT    240
Arg Phe Ser Gly Ser Gly Ser Gly Thr Asp Phe Thr Leu Asn Ile His
     65                 70                 75                 80
```

CCT GTG GAG GAG GAG GAT GCT GCA ACC TAT TAC TGT CAG CAA AGT AGT    288

Pro Val Glu Glu Glu Asp Ala Ala Thr Tyr Tyr Cys Gln Gln Ser Ser

               85                 90               95

GAG GAT CCT CCG ACG TTC GGT GGA GGC ACC AAG CTC GAA ATC AAA    333

Glu Asp Pro Pro Thr Phe Gly Gly Gly Thr Lys Leu Glu Ile Lys

             100             105             110

<210> 39

<211> 351

<212> DNA

<213> mouse


<400> 39

CAG GTG CAG CTG AAG CAG TCA GGA CCT GGC CTA GTG CAG CCC TCA CAG    48

Gln Val Gln Leu Lys Gln Ser Gly Pro Gly Leu Val Gln Pro Ser Gln

              5              10             15

AGC CTG TCC TTC ATC TGC ACA GTC TCT GGT TTC TCA TTA ACT AGT CAT    96

Ser Leu Ser Phe Ile Cys Thr Val Ser Gly Phe Ser Leu Thr Ser His

             20             25             30

GGT GTA CAC TGG GTT CGC CAG TCT CCA GGA AAG GGT CTG GAG TGG CTG    144

Gly Val His Trp Val Arg Gln Ser Pro Gly Lys Gly Leu Glu Trp Leu

             35             40             45

GGA GTG ATA TGG GGT GCT GGA AGG ACA GAC TAT AAT GCA GCT TTC ATA    192

Gly Val Ile Trp Gly Ala Gly Arg Thr Asp Tyr Asn Ala Ala Phe Ile

        50             55             60

TCC AGA CTG AGC ATC AGC AGG GAC ATT TCC AAG AGC CAA GTT TTC TTT    240

Ser Arg Leu Ser Ile Ser Arg Asp Ile Ser Lys Ser Gln Val Phe Phe

```
        65                    70                    75                    80
AAG ATG AAC AGT CTG CAA GTT GAT GAC ACA GCC ATA TAT TAC TGT GCC    288
Lys Met Asn Ser Leu Gln Val Asp Asp Thr Ala Ile Tyr Tyr Cys Ala
                            85                    90                    95
AGA AAT AGG TAC GAG AGC TAC TTT GAC TAC TGG GGC CAA GGC ACC ACT    336
Arg Asn Arg Tyr Glu Ser Tyr Phe Asp Tyr Trp Gly Gln Gly Thr Thr
                            100                   105                   110
TCC CTC ACA GTC TCC                                                 351
Leu Thr Val Ser Ser
                            115


<210>    40

<211>    339

<212>    DNA

<213>    mouse


<400>    40
GAT GTT GTG ATG ACC CAA ACT CCA CTC TCC CTG CCT GTC AGT CTT GGA    48
Asp Val Val Met Thr Gln Thr Pro Leu Ser Leu Pro Val Ser Leu Gly
                            5                     10                    15
GAT CAG GCC TCC ATC TCT TGC AGA TCT AGT CAG AAC CTT GTA CAC AGT    96
Asp Gln Ala Ser Ile Ser Cys Arg Ser Ser Gln Asn Leu Val His Ser
                            20                    25                    30
AAT GGA AAT ACC TAT TTA CAT TGG TAC CTG CAG AAG CCA GGC CAG TCT    144
Asn Gly Asn Thr Tyr Leu His Trp Tyr Leu Gln Lys Pro Gly Gln Ser
                            35                    40                    45
```

CCA AAT CTC CTG ATC TAC AAA GTT TCC AAC CGA TTT TCT GGG GTC CCA   192
Pro Asn Leu Leu Ile Tyr Lys Val Ser Asn Arg Phe Ser Gly Val Pro
     50             55           60

GAC AGG TTC AGT GGC AGT GGA TCA GGG ACA GAA TTC ACA CTC AAG ATC   240
Asp Arg Phe Ser Gly Ser Gly Ser Gly Thr Glu Phe Thr Leu Lys Ile
65           70           75           80

AGC AGA GTG GAG GCT GAG GAT CTG GGA GTT TAT TTC TGC TCT CAA AGT   288
Ser Arg Val Glu Ala Glu Asp Leu Gly Val Tyr Phe Cys Ser Gln Ser
         85           90           95

ACA CAT GTT CCG CTC ACG TTC GGT GCT GGG ACC AAG GTG GAG CTC AAA   336
Thr His Val Pro Leu Thr Phe Gly Ala Gly Thr Lys Val Glu Leu Lys
        100          105          110

CGG   339
Arg


<210> 41

<211> 879

<212> DNA

<213> mouse


<400> 41

ATG ACC ATG ATT ACG CCA AGC TTT GGA GCC TTT TTT TTG GAG ATT TTC   48
Met Thr Met Ile Thr Pro Ser Phe Gly Ala Phe Phe Leu Glu Ile Phe
        5              10           15

AAC GTG AAA AAA TTA TTA TTC GCA ATT CCT TTA GTT GTT CCT TTC TAT   96
Asn Val Lys Lys Leu Leu Phe Ala Ile Pro Leu Val Val Pro Phe Tyr

```
                    20                  25                  30
GCG GCC CAG CCG GCC ATG GCC CAG GTG AAG CTG CAG CAG TCT GGA CCT   144
Ala Ala Gln Pro Ala Met Ala Gln Val Lys Leu Gln Gln Ser Gly Pro

              35                  40                  45
GGC CTA GTG CAG CCC TCA CAG AGC CTG TCC TTC ATC TGC ACA GTC TCT   192
Gly Leu Val Gln Pro Ser Gln Ser Leu Ser Phe Ile Cys Thr Val Ser

        50                  55                  60
GGT TTC TCA TTA ACT AGT CAT GGT GTA CAC TGG GTT CGC CAG TCT CCA   240
Gly Phe Ser Leu Thr Ser His Gly Val His Trp Val Arg Gln Ser Pro

  65                  70                  75                  80
GGA AAG GGT CTG GAG TGG CTG GGA GTG ATA TGG GGT GCT GGA AGG ACA   288
Gly Lys Gly Leu Glu Trp Leu Gly Val Ile Trp Gly Ala Gly Arg Thr

                    85                  90                  95
GAC TAT AAT GCA GCT TTC ATA TCC AGA CTG AGC ATC AGC AGG GAC ATT   336
Asp Tyr Asn Ala Ala Phe Ile Ser Arg Leu Ser Ile Ser Arg Asp Ile

              100                 105                 110
TCC AAG AGC CAA GTT TTC TTT AAG ATG AAC AGT CTG CAA GTT GAT GAC   384
Ser Lys Ser Gln Val Phe Phe Lys Met Asn Ser Leu Gln Val Asp Asp

              115                 120                 125
ACA GCC ATA TAT TAC TGT GCC AGA AAT AGG TAC GAG AGC TAC TTT GAC   432
Thr Ala Ile Tyr Tyr Cys Ala Arg Asn Arg Tyr Glu Ser Tyr Phe Asp

              130                 135                 140
TAC TGG GGC CAA GGG ACC ACG GTC ACC GTC TCC TCA GGT GGA GGC GGT   480
Tyr Trp Gly Gln Gly Thr Thr Val Thr Val Ser Ser Gly Gly Gly Gly

145                 150                 155                 160
TCA GGC GGA GGT GGC TCT GGC GGT GGC GGA TCG GAC ATC GAG CTC ACT   528
```

Ser Gly Gly Gly Gly Ser Gly Gly Gly Gly Ser Asp Ile Glu Leu Thr
                165          170          175

CAG TCT CCA CTC TCC CTG CCT GTC AGT CTT GGA GAT CAG GCC TCC ATC   576
Gln Ser Pro Leu Ser Leu Pro Val Ser Leu Gly Asp Gln Ala Ser Ile
                180          185          190

TCT TGC AGA TCT AGT CAG AAC CTT GTA CAC AGT AAT GGA AAT ACC TAT   624
Ser Cys Arg Ser Ser Gln Asn Leu Val His Ser Asn Gly Asn Thr Tyr
                195          200          205

TTA CAT TGG TAC CTG CAG AAG CCA GGC CAG TCT CCA AAT CTC CTG ATC   672
Leu His Trp Tyr Leu Gln Lys Pro Gly Gln Ser Pro Asn Leu Leu Ile
                210          215          220

TAC AAA GTT TCC AAC CGA TTT TCT GGG GTC CCA GAC AGG TTC AGT GGC   720
Tyr Lys Val Ser Asn Arg Phe Ser Gly Val Pro Asp Arg Phe Ser Gly
225               230          235          240

AGT GGA TCA GGG ACA GAA TTC ACA CTC AAG ATC AGC AGA GTG GAG GCT   768
Ser Gly Ser Gly Thr Glu Phe Thr Leu Lys Ile Ser Arg Val Glu Ala
                245          250          255

GAG GAT CTG GGA GTT TAT TTC TGC TCT CAA AGT ACA CAT GTT CCG CTC   816
Glu Asp Leu Gly Val Tyr Phe Cys Ser Gln Ser Thr His Val Pro Leu
                260          265          270

ACG TTC GGT GCT GGG ACC AAG GTG GAG CTC AAA CGG GCG GCC GCA GGT   864
Thr Phe Gly Ala Gly Thr Lys Val Glu Leu Lys Arg Ala Ala Ala Gly
                275          280          285

GCG CCG GTG CCG TAT CCG GAT CCG CTG GAA CCG CGT GCC GCA TAG       909
Ala Pro Val Pro Tyr Pro Asp Pro Leu Glu Pro Arg Ala Ala
                290          295          300

```
<210>   42

<211>   918

<212>   DNA

<213>   mouse


<400>   42
ATG ACC ATG ATT ACG CCA AGC TTT GGA GCC TTT TTT TTG GAG ATT TTC     48
Met Thr Met Ile Thr Pro Ser Phe Gly Ala Phe Phe Leu Glu Ile Phe
                    5                   10          ·         15
AAC GTG AAA AAA TTA TTA TTC GCA ATT CCT TTA GTT GTT CCT TTC TAT     96
Asn Val Lys Lys Leu Leu Phe Ala Ile Pro Leu Val Val Pro Phe Tyr
                20                  25                  30
GCG GCC CAG CCG GCC ATG GCC CAG GTG AAG CTG CAG CAG TCT GGA CCT    144
Ala Ala Gln Pro Ala Met Ala Gln Val Lys Leu Gln Gln Ser Gly Pro
                35                  40                  45
GGC CTA GTG CAG CCC TCA CAG AGC CTG TCC TTC ATC TGC ACA GTC TCT    192
Gly Leu Val Gln Pro Ser Gln Ser Leu Ser Phe Ile Cys Thr Val Ser
        50                  55                  60
GGT TTC TCA TTA ACT AGT CAT GGT GTA CAC TGG GTT CGC CAG TCT CCA    240
Gly Phe Ser Leu Thr Ser His Gly Val His Trp Val Arg Gln Ser Pro
    65                  70                  75                  80
GGA AAG GGT CTG GAG TGG CTG GGA GTG ATA TGG GGT GCT GGA AGG ACA    288
Gly Lys Gly Leu Glu Trp Leu Gly Val Ile Trp Gly Ala Gly Arg Thr
                    85                  90                  95
GAC TAT AAT GCA GCT TTC ATA TCC AGA CTG AGC ATC AGC AGG GAC ATT    336
Asp Tyr Asn Ala Ala Phe Ile Ser Arg Leu Ser Ile Ser Arg Asp Ile
```

Asp Tyr Asn Ala Ala Phe Ile Ser Arg Leu Ser Ile Ser Arg Asp Ile
              100              105              110

TCC AAG AGC CAA GTT TTC TTT AAG ATG AAC AGT CTG CAA GTT GAT GAC    384
Ser Lys Ser Gln Val Phe Phe Lys Met Asn Ser Leu Gln Val Asp Asp
          115              120              125

ACA GCC ATA TAT TAC TGT GCC AGA AAT AGG TAC GAG AGC TAC TTT GAC    432
Thr Ala Ile Tyr Tyr Cys Ala Arg Asn Arg Tyr Glu Ser Tyr Phe Asp
          130              135              140

TAC TGG GGC CAA GGG ACC ACG GTC ACC GTC TCC TCA GGT GGA GGC GGT    480
Tyr Trp Gly Gln Gly Thr Thr Val Thr Val Ser Ser Gly Gly Gly Gly
145              150              155              160

TCA GGC GGA GGT GGC TCT GGC GGT GGC GGA TCG GAC ATC GAG CTC ACT    528
Ser Gly Gly Gly Gly Ser Gly Gly Gly Gly Ser Asp Ile Glu Leu Thr
              165              170              175

CAG TCT CCA CTC TCC CTG CCT GTC AGT CTT GGA GAT CAG GCC TCC ATC    576
Gln Ser Pro Leu Ser Leu Pro Val Ser Leu Gly Asp Gln Ala Ser Ile
              180              185              190

TCT TGC AGA TCT AGT CAG AAC CTT GTA CAC AGT AAT GGA AAT ACC TAT    624
Ser Cys Arg Ser Ser Gln Asn Leu Val His Ser Asn Gly Asn Thr Tyr
          195              200              205

TTA CAT TGG TAC CTG CAG AAG CCA GGC CAG TCT CCA AAT CTC CTG ATC    672
Leu His Trp Tyr Leu Gln Lys Pro Gly Gln Ser Pro Asn Leu Leu Ile
          210              215              220

TAC AAA GTT TCC AAC CGA TTT TCT GGG GTC CCA GAC AGG TTC AGT GGC    720
Tyr Lys Val Ser Asn Arg Phe Ser Gly Val Pro Asp Arg Phe Ser Gly
225              230              235              240

```
AGT GGA TCA GGG ACA GAA TTC ACA CTC AAG ATC AGC AGA GTG GAG GCT    768
Ser Gly Ser Gly Thr Glu Phe Thr Leu Lys Ile Ser Arg Val Glu Ala
                245                 250                 255
GAG GAT CTG GGA GTT TAT TTC TGC TCT CAA AGT ACA CAT GTT CCG CTC    816
Glu Asp Leu Gly Val Tyr Phe Cys Ser Gln Ser Thr His Val Pro Leu
                260                 265                 270
ACG TTC GGT GCT GGG ACC AAG GTG GAG CTG AAA CGG GCG GCC GCA GGT    864
Thr Phe Gly Ala Gly Thr Lys Val Glu Leu Lys Arg Ala Ala Ala Gly
                275                 280                 285
GCG CCG GTC CCG TAT CCG GAT CCG CTG GAA CCG CGT GCC GCA AAG        909
Ala Pro Val Pro Tyr Pro Asp Pro Leu Glu Pro Arg Ala Ala Lys
        290                 295                 300
AAG AAG TAG                                                         918
Lys Lys
        305
```

<210> 43

<211> 5

<212> PRT

<213> Artificial Sequence

<220>

<223> Amino acid sequence of heavy chain CDR-1

<400> 43

Ser His Gly Val His

<210> 44

<211> 16

<212> PRT

<213> Artificial Sequence

<220>

<223> Amino acid sequence of heavy chain CDR-2

<400> 44

Val Ile Trp Gly Ala Gly Arg Thr Asp Tyr Asn Ala Ala Phe Ile Ser
1               5                   10                  15

<210> 45

<211> 9

<212> PRT

<213> Artificial Sequence

<220>

<223> Amino acid sequence of heavy chain CDR-3

<400> 45

Asn Arg Tyr Glu Ser Tyr Phe Asp Tyr

<210> 46

<211> 16

<212> PRT

<213> Artificial Sequence

<220>

<223> Amino acid sequence of light chain CDR-1

<400> 46

Arg Ser Ser Gln Asn Leu Val His Ser Asn Gly Asn Thr Tyr Leu His
1               5                   10                  15

<210> 47

<211> 13

<212> PRT

<213> Artificial Sequence

<220>

<223> Amino acid sequence of light chain CDR-2

<400> 47

Lys Val Ser Asn Arg Phe Ser Gly Val Pro Asp Arg Phe

<210> 48

<211> 9

<212> PRT

<213> Artificial Sequence

<220>

<223> Amino acid sequence of light chain CDR-3

<400> 48

Ser Gln Ser Thr His Val Pro Leu Thr